# EUROPEAN PATENT APPLICATION

(11) **EP 1 559 785 A1**
(43) Date of publication of application: **03.08.2005**
(21) Application number: 05250464.4
(22) Date of filing: 28.01.2005
(51) Int. Cl.: C12N 15/11, G06F 17/50, G06F 17/40, G06F 17/30

(54) **Oligo- or polynucleotides for achieving RNA interference in mammalian cells**

(30) Priority: 28.01.2004 US 539332
(71) Applicant: Bio-Think Tank Co., Ltd., Tokyo 113-0033 (JP)
(72) Inventor: Saigo, Kaoru, Tokyo 168-0063 (JP); Ui-Tei, Kumiko, Tokyo 113-0023 (JP)
(74) Representative: Jones, Elizabeth Louise

(57) **Abstract**

The invention provides methods of designing oligo- or polynucleotide sequences, preferably shRNA, for achieving RNA interference. The invention further extends to such oligo- or polynucleotides for achieving RNA interference comprising a sense sequence, a trimming sequence and an antisense sequence.

In an example, the sense sequence is homologous to a part of a sequence of a target gene wherein the base of the terminal nucleotide at the 5' end is guanine, and the base of the terminal nucleotide at the 3' end is adenine, thymine, or uracil, the antisense sequence is complementary to the sense sequence and the 7-bp-long region at its 5' terminal is rich in at least one base selected from the group consisting of adenine, thymine, and uracil, and the trimming sequence comprises 5 to 52 nucleotides and is represented by the formula: (G or C)-X-Y-Z-(C or G) wherein the sense sequence, the trimming sequence, and the antisense sequence are consecutive, and this consecutive sequence does not comprise four or more consecutive nucleotides with thymine and/or uracil bases.

## Description

This invention relates to an oligo- or polynucleotide that is expressed in the presence of a RNA polymerase III transcriptional promoter and that can cause RNA interference, a recombinant DNA into which the oligo- or polynucleotide is inserted, a cell which has been transfected with the recombinant DNA, and a method for causing RNA interference in mammalian cells by using the recombinant DNA.

This invention also relates to a data processing device and a program for designing shRNA sequences, and a recording medium on which the program is recorded.

Further, this invention relates to a method for designing shRNA sequences.

RNA interference (RNAi) is a process of post-transcriptional gene silencing that relies on double-stranded RNA. RNA interference first appeared in the literature in 1998 in a publication by Fire, A. et al. which described the phenomenon that a target gene was not transcribed when cells of Caenorhabditis elegans were transfected with a double-stranded RNA consisting of a RNA that was homologous to the target gene and another RNA that was complementary to the above RNA (Fire, A. et al., Nature 391, p.p.806-811 (1998); WO99/32619). However, when a long, double-stranded RNA is introduced into mammalian cells, a cytotoxic interferon response often occurs. Thus, it had been difficult to study the RNA interference in mammalian cells.

In 1999, Tuschl T. et al. reported that when human cultured cells were transfected with double-stranded RNA, of which one of the strands of RNA consisted of 21 ribonucleotides having a sequence that was homologous to a part of a sequence of a target gene, silencing of expression of mRNA specific to the target gene was observed, namely, RNA interference occurred (Tuschl T. et al., Genes Dev. 13, p.p.3191-3197 (1999); WO01/75164). This short, double-stranded RNA was designated as short interference RNA (siRNA). siRNA rarely causes the interferon response even if it is introduced into mammalian cells. Therefore, after this paper was reported, studies about RNA interference in mammalian cells rapidly advanced.

In the beginning of the studies, chemically synthesized siRNAs were introduced directly into mammalian cells. Then, a method was proposed in which a plasmid vector was used which had an RNA polymerase promoter and into which the sense sequence and antisense sequence of the siRNA and a sequence (which was designated as, e.g., spacer, hairpin, or trimming) between the sense sequence and the antisense sequence were introduced (Zamore P., et al., Science 297, p.p.2056-2060 (2002); WO 03/006477). When mammalian cells are transfected with the plasmid vector, a double-stranded RNA (shRNA) comprising the sense sequence and the antisense sequence in which the sequence between the sense sequence and the antisense sequence is maintained, namely, an shRNA having a hairpin formation, is made. The shRNA is processed by Dicer (a base specific RNAse) to become siRNA. The siRNA transfers from the nucleus to the cytoplasm and causes RNA interference.

In mammalian cells it is becoming clear that only some siRNAs which have sequences that are homologous to parts of the sequence of the target gene can cause significantly effective RNA interference. In fact, software has been developed by which sequences that can effectively cause the RNA interference are selected. Many papers about methods for designing siRNAs have been reported.

Software has been developed which can select, from a nucleotide sequence of a target gene, partial sequences that can effectively cause RNA interference. However, the design of nucleotide sequences for shRNAs that can effectively cause RNA interference cannot be attained only by using the rules for designing sequences for siRNAs.

The present invention was attained as a result of studies for designing nucleotide sequences of shRNAs that can effectively cause RNA interference.

This invention includes the following aspects:
[1] An oligo- or polynucleotide for RNA interference comprising a sense sequence, a trimming sequence, and an antisense sequence in this order, wherein:
   the sense sequence consisting of (i) a sequence (B) which is homologous to a part of a sequence (b) of a target gene which is calculated to suffer from an RNA interference and (ii) at least one sequence selected from the group consisting of (ii-1) a sequence (C) which is added to the 3' end of the sequence (B) and comprises 0 to 5 nucleotides and (ii-2) a sequence (D) which is added to the 5' end of the sequence (B) and comprises 0 to 5 nucleotides, wherein a base in the nucleotide of the 5' end of the sense sequence is guanine, a base in the nucleotide of the 3' end of the sense sequence is adenine, thymine, or uracil, and the number of the nucleotides in the sense sequence is one by which number RNA interference can occur without cytotoxicity;
   the antisense sequence is complementary to the sense sequence, wherein the nucleotides of the 5' end and the 3' end of the antisense sequence are entirely complementary to the correspondent nucleotides of the sense sequence, respectively, the 7-bp-long region of the 5' terminal of the antisense sequence is rich in at least one base selected from the group consisting of adenine, thymine, and uracil, and the number of the nucleotides in the antisense sequence is one by which number RNA interference can occur without cytotoxicity; and
   the trimming sequence comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide which the Z region follows are not complementary to each other;
   wherein the sense sequence, the trimming sequence, and the antisense sequence are consecutive, and this consecutive sequence does not comprise four or more consecutive nucleotides of which the bases are thymine and/or uracil.
   [1-1] The oligo- or polynucleotide according to [1], wherein the sense sequence and the antisense sequence comprise no long stretches of nucleotides of which the bases are guanine and/or cytosine.
   [1-2] The oligo- or polynucleotide according to [1] or [1-1], wherein the Y region comprises 6 to 20 nucleotides which are not complementary to each other and becomes a loop portion when a sense strand and an antisense strand become a double-stranded portion.
   [1-3] The oligo- or polynucleotide according to [1], [1-1], or [1-2], wherein the sense sequence and the antisense sequence each comprise 13 to 28 nucleotides.
   [1-4] The oligo- or polynucleotide according to [1] or any of [1-1] to [1-3], which comprises an overhang portion which consists of 1 to 3 nucleotides and is added to the 3' end of the antisense sequence.
   [1-5] The oligo- or polynucleotide according to [1] or any of [1-1] to [1-4], wherein the sense sequence is identical to a part of the sequence (b) of the target gene, and the antisense sequence is entirely complementary to the sense sequence.
   [1-6] The oligo- or polynucleotide according to [1] or any of [1-1] to [1-4], wherein the sense sequence is identical to a part of the sequence (b) of the target gene except for the nucleotide of the 3' end, and the antisense sequence is entirely complementary to the sense sequence.
   [1-7] The oligo- or polynucleotide according to [1] or any of [1-1] to [1-6], wherein the lengths of the sense sequence and the anti-sense sequence are decided so that the cleavage sites by Dicer are between the second nucleotide and the third nucleotide from the 5' end of the trimming sequence, and between the trimming sequence and the antisense sequence.
   [ 1 -8] The oligo- or polynucleotide according to [1] or any of [1-1] to [1-7], which comprise 30 to 90 nucleotides.
[2] An oligo- or polynucleotide for RNA interference comprising an antisense sequence, a trimming sequence, and a sense sequence in this order, wherein:
   the sense sequence consisting of (i) a sequence (K) which is homologous to a part of a sequence (b) of a target gene which is calculated to suffer from an RNA interference and (ii) at least one sequence selected from the group consisting of (ii-1) a sequence (L) which is added to the 3' end of the sequence (K) and comprises 0 to 5 nucleotides and (ii-2) a sequence (M) which is added to the 5' end of the sequence (K) and comprises 0 to 5 nucleotides, wherein a base in the nucleotide of the 5' end of the sense sequence is guanine or cytosine, a base in the nucleotide of the 3' end of the sense sequence is cytosine, thymine, or uracil, the 7-bp-long region of the 3' terminal of the sense sequence is rich in at least one base selected from the group consisting of adenine, thymine, and uracil, and the number of the nucleotides in the sense sequence is one by which number the RNA interference can occur without cytotoxicity;
   the anti-sense sequence is complementary to the sense sequence, wherein the nucleotides of the 5' end and the 3' end of the antisense sequence are entirely complementary to the correspondent nucleotides of the sense sequence, respectively, and the number of the nucleotides in the antisense sequence is one by which number RNA interference can occur without cytotoxicity; and
   the trimming sequence comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
   wherein the antisense sequence, the trimming sequence, and the sense sequence are consecutive, and this consecutive sequence does not comprise four or more consecutive nucleotides of which the bases are thymine and/or uracil.
   [2-1] The oligo- or polynucleotide according to [2], wherein the sense sequence and the antisense sequence comprise no long stretches of nucleotides of which the bases are guanine and/or cytosine.
   [2-2] The oligo- or polynucleotide according to [2] or [2-1], wherein the Y region comprises 6 to 20 nucleotides which are not complementary to each other and becomes a loop portion when a sense strand and an antisense strand become a double-stranded portion.
   [2-3] The oligo- or polynucleotide according to [2], [2-1], or [2-2], wherein the sense sequence and the antisense sequence each comprise 13 to 28 nucleotides.
   [2-4] The oligo- or polynucleotide according to [2] or any of [2-1] to [2-3], which comprises an overhang portion which consists of 1 to 3 nucleotides and is added to the 3' end of the sense sequence.
   [2-5] The oligo- or polynucleotide according to [2] or any of [2-1 ] to [2-4], wherein the sense sequence is identical to a part of the sequence (b) of the target gene, and the antisense sequence is entirely complementary to the sense sequence.
   [2-6] The oligo- or polynucleotide according to [2] or any of [2-1] to [2-4], wherein the sense sequence is identical to a part of the sequence (b) of the target gene except for the nucleotide of the 5' end, and the antisense sequence is entirely complementary to the sense sequence.
   [2-7] The oligo- or polynucleotide according to [2] or any of [2-1] to [2-6], wherein the lengths of the sense sequence and the anti-sense sequence are decided so that the cleavage sites by Dicer are between the second nucleotide and the third nucleotide from the 5' end of the trimming sequence, and between the trimming sequence and the sense sequence.
   [2-8] The oligo- or polynucleotide according to [2] or any of [2-1] to [2-7], which comprises 30 to 90 nucleotides.
[3] The oligo- or polynucleotide according to any of [1], [1-1] to [1-8], [2], and [2-1 ] to [2-8], wherein the oligo- or polynucleotide is DNA.
[4] A double-stranded DNA comprising the DNA of [3] and another DNA which is complementary to the DNA of [3].
[5] A recombinant DNA comprising an RNA polymerase III-type transcriptional promoter and the double-stranded DNA of [4] which has been inserted downstream of the promoter.
   [5-1 ] The recombinant DNA according to [5], which is used for a gene therapy.
   [5-2] The recombinant DNA according to [5], which is used for prevention of a disease.
[6] The oligo- or polynucleotide according to any of [1], [1-1] to [1-8], [2], and [2-1 to [2-8], wherein the oligo - or polynucleotide is RNA.
[7] The oligo- or polynucleotide according to any of [1], [1-1] to [1-8], [2], and [2-1 to [2-8], wherein the oligo - or polynucleotide is shRNA.
[8] A double-stranded RNA comprising the RNA of [6] and another RNA which is complementary to the RNA of [6].
[9] A cell transfected with the recombinant DNA of [5].
[10] A method for causing RNA interference in a mammalian cell comprising transfecting the mammalian cell with the recombinant DNA of [5].
[11] A device (or apparatus) for processing information for designing the sequence of an oligo- or polynucleotide, preferably an shRNA sequence comprising:
   (1) a portion for acquiring (or receiving or retaining) information (or data) of a nucleotide sequence of a target gene for RNA interference and creating (or generating) information of partial sequences each having a predetermined number of consecutive nucleotides from the acquired information;
   (2) a portion for judging (or determining) a nucleotide of the 5' end wherein a sequence(s) containing a nucleotide of which the base is guanine at the 5' end is selected from the partial sequences in the created information of partial sequences;
   (3) a portion for judging a nucleotide of the 3' end wherein a sequence(s) containing a nucleotide of which the base is adenine, thymine, or uracil at the 3' end is selected from the partial sequences in the created information of partial sequences;
   (4) a portion for judging whether specific nucleotides are contained in a sequence wherein a sequence(s) which is rich in at least one base selected from the group consisting of adenine, thymine, and uracil in 7 nucleotides at the 3' terminal is selected from the partial sequences in the created information of partial sequences;
   (5) a portion for designing a sense sequence/antisense sequence wherein for each of the partial sequence(s) that were selected in all of the above portions (2), (3), and (4), a sense sequence that is identical to the selected sequence and an antisense sequence that is entirely complementary to the sense sequence are decided;
   (6) a portion for designing a trimming (or linking) sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other,
   (7) a portion for designing an RNA sequence wherein the sequences that were decided in the above portions (5) and (6) are consecutively arranged in the order of the sense sequence, the trimming sequence, and the antisense sequence; and
   (8) a portion for selecting at least one sequence which does not contain four or more consecutive nucleotides of which the bases are thymine and/or uracil from the RNA sequences designed in the above portion (7).
[12] A program for practicing a method for processing information for designing the sequence of an oligo- or polynucleotide, preferably an shRNA sequence in a computer comprising:
   (1) a step comprising acquiring information of a nucleotide sequence of a target gene for RNA interference and creating information of partial sequences each having a predetermined number of consecutive nucleotides from the acquired information;
   (2) a step of judging a nucleotide of the 5' end wherein a sequence(s) containing a nucleotide of which the base is guanine at the 5' end is selected from the partial sequences in the created information of partial sequences;
   (3) a step of judging a nucleotide of the 3' end wherein a sequence(s) containing a nucleotide of which the base is adenine, thymine, or uracil at the 3' end is selected from the partial sequences in the created information of partial sequences;
   (4) a step of judging whether specific nucleotides are contained in a sequence wherein a sequence(s) which is rich in at least one base selected from the group consisting of adenine, thymine, and uracil in 7 nucleotides at the 3' terminal is selected from partial sequences in the created information of partial sequences;
   (5) a step of designing a sense sequence/antisense sequence wherein for each of the partial sequence(s) that was selected in all of the above steps (2), (3), and (4), a sense sequence that is identical to the selected sequence and an antisense sequence that is entirely complementary to the sense sequence are decided;
   (6) a step of designing a trimming sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
   (7) a step of designing an RNA sequence wherein the sequences that were decided in the above steps (5) and (6) are consecutively arranged in order of the sense sequence, the trimming sequence, and the antisense sequence; and
   (8) a step of selecting at least one sequence which does not contain four or more consecutive nucleotides of which the bases are thymine and/or uracil from the RNA sequences designed in the above step (7).
[13] A computer-readable recording medium in which the program according to [12] is recorded.
[14] A device for processing information for designing the sequence of an oligo-or polynucleotide, preferably an shRNA sequence comprising:
   (1) a portion for acquiring information of a nucleotide sequence of a target gene for RNA interference and creating information of partial sequences each having a predetermined number of consecutive nucleotides from the acquired information;
   (2) a portion for judging a nucleotide of the 5' end wherein a sequence(s) containing a nucleotide of which the base is guanine or cytosine at the 5' end is selected from the partial sequences in the created information of partial sequences;
   (3) a portion for judging a nucleotide of the 3' end wherein a sequence(s) containing a nucleotide of which the base is cytosine, thymine, or uracil at the 3' end is selected from the partial sequences in the created information of partial sequences;
   (4) a portion for judging whether specific nucleotides are contained in a sequence wherein a sequence(s) which is rich in at least one base selected from the group consisting of adenine, thymine, and uracil in 7 nucleotides at the 3' terminal is selected from partial sequences in the created information of partial sequences;
   (5) a portion for designing a sense sequence/antisense sequence wherein for each of the partial sequence(s) that were selected in all of the above portions (2), (3), and (4), a sense sequence that is identical to the selected sequence and an antisense sequence that is entirely complementary to the sense sequence are decided;
   (6) a portion for designing a trimming sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
   (7) a portion for designing an RNA sequence wherein the sequences that were decided in the above portions (5) and (6) are consecutively arranged in the order of the sense sequence, the trimming sequence, and the antisense sequence; and
   (8) a portion for selecting at least one sequence which does not contain four or more consecutive nucleotides of which the bases are thymine and/or uracil from the RNA sequences designed in the above portion (7).
[15] A program for practicing a method for processing information for designing the sequence of an oligo- or polynucleotide, preferably an shRNA sequence in a computer comprising:
   (1) a step of acquiring information of a nucleotide sequence of a target gene for RNA interference and creating information of partial sequences each having a predetermined number of consecutive nucleotides from the acquired information;
   (2) a step of judging a nucleotide of the 5' end wherein a sequence(s) containing a nucleotide of which the base is guanine or cytosine at the 5' end is selected from the partial sequences in the created information of partial sequences;
   (3) a step of judging a nucleotide of the 3' end wherein a sequence(s) containing a nucleotide of which the base is cytosine, thymine, or uracil at the 3' end is selected from the partial sequences in the created information of partial sequences;
   (4) a step of judging whether specific nucleotides are contained in a sequence wherein a sequence(s) which is rich in at least one base selected from the group consisting of adenine, thymine, and uracil in 7 nucleotides at the 3' terminal is selected from partial sequences in the created information of partial sequences;
   (5) a step of designing a sense sequence/antisense sequence wherein for each of the partial sequence(s) that were selected in all of the above steps (2), (3), and (4), a sense sequence that is identical to the selected sequence and an antisense sequence that is entirely complementary to the sense sequence are decided;
   (6) a step of designing a trimming sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
   (7) a step of designing an RNA sequence wherein the sequences that were decided in the above steps (5) and (6) are consecutively arranged in order of the sense sequence, the trimming sequence, and the antisense sequence; and
   (8) a step of selecting at least one sequence which does not contain four or more consecutive nucleotides of which the bases are thymine and/or uracil from the RNA sequences designed in the above step (7).
[16] A computer-readable recording medium in which the program according to [15] is recorded.
[17] A method for designing the sequence of an oligo- or polynucleotide, preerably the sequence of an shRNA, which can cause RNA interference in mammalian cells, which is expressed by an RNA polymerase III-type promoter, and which comprises a sense sequence, a trimming sequence, and an anti-sense sequence in this order, comprising:
   selecting as the sense sequence a sequence (A) consisting of (i) a sequence (B) which is homologous to a part of a sequence (b) of a target gene which is calculated to suffer from an RNA interference and (ii) at least one sequence selected from the group consisting of (ii-1) a sequence (C) which is added to the 3' end o f the sequence (B) and comprises 0 to 5 nucleotides and (ii-2) a sequence (D) which is added to the 5' end of the sequence (B) and comprises 0 to 5 nucleotides, wherein a base in the nucleotide of the 5' end of the sense sequence is guanine, a base in the nucleotide of the 3' end of the sense sequence is adenine or uracil (or thymine in DNA), and the number of nucleotides in the sense sequence is one by which number RNA interference can occur without cytotoxicity;
   selecting as the antisense sequence a sequence (E) which is complementary to the sense sequence, wherein the nucleotides of the 5' end and 3' end of the antisense sequence are entirely complementary to the correspondent nucleotides of the sense sequence, respectively, the 7-bp-long region of the 5' terminal of the antisense sequence is rich in at least one base selected from the group consisting of adenine and uracil (or thymine), and the number of the nucleotides in the antisense sequence is one by which number RNA interference can occur without cytotoxicity; and
   selecting as the trimming sequence a sequence (F) which comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, uracil, (or thymine) guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
   wherein the sense sequence, the trimming sequence, and the antisense sequence are consecutive, and this consecutive sequence does not comprise four or more consecutive nucleotides of which the bases are uracils (or thymines).
[18] A method for designing the sequence of an oligo- or polynucleotide, preferably the sequence of an shRNA, which can cause an RNA interference, which is expressed by an RNA polymerase III-type promoter, and which comprises an antisense sequence, a trimming sequence, and a sense sequence in this order, comprising:
   selecting as the sense sequence a sequence (J) consisting of (i) a sequence (K) which is homologous to a part of a sequence (b) of a target gene which is calculated to suffer from an RNA interference and (ii) at least one sequence selected from the group consisting of (ii-1) a sequence (L) which is added to the 3' end o f the sequence (K) and comprises 0 to 5 nucleotides and (ii-2) a sequence (M) which is added to the 5' end of the sequence (K) and comprises 0 to 5 nucleotides, wherein the base in the nucleotide of the 5' end of the sense sequence is guanine or cytosine, a base in the nucleotide of the 3' end of the sense sequence is cytosine or uracil (or thymine), the 7-bp-long region of the 3' terminal of the sense sequence is rich in at least one base selected from the group consisting of adenine and uracil (or thymine), and the number of the nucleotides in the sense sequence is one by which number RNA interference can occur without cytotoxicity;
   selecting as the antisense sequence a sequence (N) which is complementary to the sense sequence, wherein the nucleotides of the 5' end and 3' end of the antisense sequence are entirely complementary to the correspondent nucleotides of the sense sequence, respectively, and the number of the nucleotides in the antisense sequence is one by which number RNA interference can occur without cytotoxicity; and
   selecting as the trimming sequence a sequence (F) which comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides of which the bases are selected from the group consisting of adenine, uracil (or thymine), guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
wherein the antisense sequence, the trimming sequence, and the sense sequence are consecutive, and this consecutive sequence does not comprise four or more consecutive nucleotides of which bases are uracils (or thymine).

Figure 1 is a flowchart to explain a program of this invention.

Figure 2 is a block diagram that shows an example of a system for processing information comprising a device for processing information of this invention.

Figure 3 is a diagram that shows an example of information stored in a file 106a of a target gene's nucleotide sequence.

Figure 4 is a diagram that shows an example of information stored in a file 106b of partial sequences.

Figure 5 is a diagram that shows an example of information stored in a file 106c of judged results (1).

Figure 6 is a diagram that shows an example of information stored in a file 106d of sense sequences/antisense sequences.

Figure 7 is a diagram that shows an example of information stored in a database 106e of reference sequences.

Figure 8 is a diagram that shows an example of information stored in a file 106f of degrees of similarities.

Figure 9 is a diagram that shows an example of information stored in a file 106g of evaluated results.

Figure 10 is a block diagram that shows an example of a structure of a portion 102a for creating partial sequences.

Figure 11 is a block diagram that shows an example of a structure of a portion 102h to evaluate whether a sequence targets an unrelated gene.

Figure 12 is a flowchart that shows an example of main processing by using a device for processing information of this invention.

Figure 13 is a flowchart that shows an example of processing to evaluate whether a sequence targets an unrelated gene.

Figure 14 shows locations of 16 cognate siRNAs for silencing of the firefly luc gene.

Figures 15A and15B show the relationship between luc siRNA sequences and induced luc-gene-silencing (RNAi) activities. For the activities, data obtained from 2-4 experiments were averaged and shown. Thin vertical lines indicate the average of three mammalian cells. For the sequences, the 7 bp-long terminal region with the 5' antisense strand end is boxed.

Figure 16A shows highly effective silencings of endogenous genes by class Ia siRNAs. The sequences of siRNAs examined are depicted in the right margin.

Figure 16B shows highly effective silencings of endogenous genes by class Ia siRNAs. The sequences of siRNAs examined are depicted in the right margin. Four pictures on the left are phase contrast photographs. Class Ia siRNA-specific degradation of Oct 4 mRNA visualized with RT-PCR is shown at the center. Gapd was used as a control.

Figure 16C shows RNAi caused by an uncognate siRNA. The sequences of siRNAs examined are depicted in the right margin.

Figure 16D shows class Ia-siRNA-dependent RNAi in chick embryos. The sequences of siRNAs examined are depicted in the right margin.

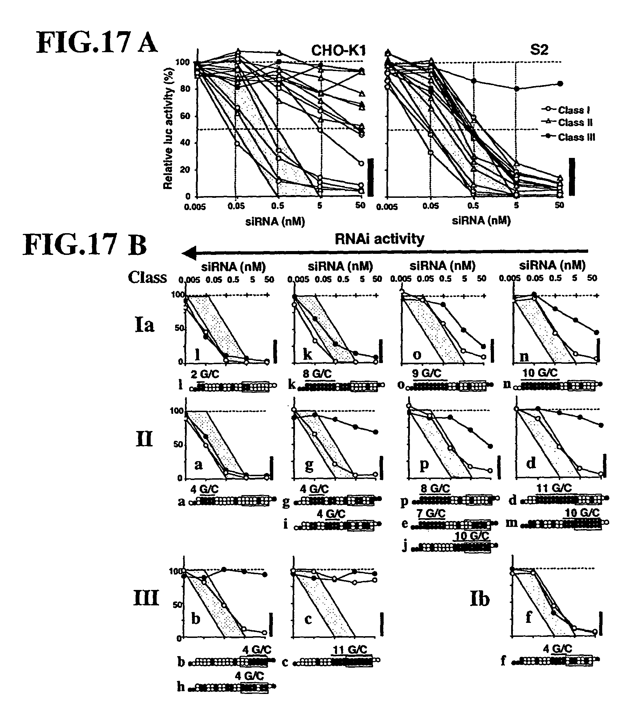

Figure 17A shows graphs of dose dependencies of RNAi effects in CHO-K1 and S2 cells. The shaded area is the region bounded by two lines, intersecting, respectively, the horizontal axis at 0.5 and 5 and the 50% line of luc activity at 0.05 and 0.5. The thick vertical bar at the right of each panel indicates the region with more than 77% reduction in luc activity. The graphs show changes in *luc*-gene silencing activities with siRNAs ranging from 0.005-50 nM in CHO-K1 (left) and S2 (right) cells. siRNAs a-p are grouped into three classes, I (open circles), II (open triangles), and III (closed circles).

Figure 17B shows graphs by which RNAi activity curves in S2 (open circles) and CHO-K1 cells (filled circles) can be directly compared. The sequences of corresponding or similar siRNAs are schematically shown in the lower margin. Filled circles, G/C. Open circles, A/U. The 7 bp-long duplex region containing the 5' AS end is boxed.

Figure 18 is a graph that shows GC content distributions of highly effective class Ia siRNAs. This graph presents distributions of the GC contents of 31 highly effective class Ia siRNAs shown in Figs. 15 A-B and 16A-D. Position 1 corresponds to the siRNA duplex end including the 5' antisense strand end. The average GC content of the regions 2 to 7 was 19% while that of the regions 8 to 18 was 52%.

Figure 19 is a graph that shows comparisons of siRNA-based RNAi and DNA-based RNAi in HeLa cells. The predicted sequences of hairpin-type transcripts are shown on the left, while induced RNAi activity (reduction in relative luciferase activity) is shown by open boxes on the right. Stippled boxes indicate relative-luciferase-activity reduction due to cognate siRNA in HeLa cells. On the left, predicted antisense strands are shaded. Data obtained from 2-4 experiments were averaged and shown.

Figure 20A is a graph that shows thermodynamic profiles of highly effective 32 siRNAs. The vertical bars show a standard deviation of 32 highly effective siRNAs.

Figure 20B is a graph that shows thermodynamic profiles of siRNAs that give rise to highly effective RNAi in mammalian cells. The thick vertical bar indicates a free energy change range at position 1 of highly effective siRNAs.

Figure 20C is a graph that shows thermodynamic profiles of siRNAs that give rise to intermediate RNAi in mammalian cells. The thick and open vertical bars, respectively, indicate free energy change ranges at position 1 of highly effective and ineffective siRNAs.

Figure 20D is a graph that shows thermodynamic profiles of siRNAs that give rise to ineffective RNAi in mammalian cells. The open vertical bar indicates a free energy change range at position 1 of ineffective siRNAs.

Figure 21A shows a possible model of siRNA-based RNAi in mammalian cells. Specifically, it shows the rules for siRNA sequence preference. A/U at the 5' AS and SS ends and their counterparts in the sense and antisense strands, respectively, are shown as hatched circles; G or C, closed circles. The terminal AU-rich and GC-rich regions are boxed. The open arrows indicate the direction of siRNA unwinding due to a hypothetical siRNA helicase.

Figure 21B shows a possible model of siRNA-based RNAi in mammalian cells. Specifically, it shows a site for binding of an unidentified protein possibly suppressing siRNA unwinding and another site for binding a putative unwinding stimulation factor other than helicase.

Figure 21 C shows a possible model of siRNA-based RNAi in mammalian cells. Specifically, it shows that a long GC stretch might prevent the elongation of siRNA-duplex denaturation from the AS end.

Hereafter, this invention is specifically explained. However, this invention is not restricted by the following explanations. The scope of the invention is specified only by the claims.

First, methods for designing the sequence of an oligo- or polynucleotide, preferably the sequence of an shRNA are explained. Methods of designing shRNA is described. The production of other oligo- or polynucleotides of the invention may be readily generated by using analogous methods. When concerned with DNA, reference to uracil should be considered to be reference to thymine. Such oligo- or polynucleotide molecules are suitable for achieving RNA interference in mammalian cells, e.g. may be formulated into recombinant DNA molecules for expression in cells, as described herein.

A first method is one for designing a sequence of an shRNA which can cause an RNA interference in mammalian cells, which is expressed by an RNA polymerase III-type promoter, and which comprises a sense sequence, a trimming sequence, and an anti-sense sequence in this order, comprising:
selecting as the sense sequence a sequence (A) consisting of (i) a sequence (B) which is homologous to a part of a sequence (b) of a target gene which is calculated to suffer from RNA interference and (ii) at least one sequence selected from the group consisting of (ii-1) a sequence (C) which is added to the 3' end o f the sequence (B) and comprises 0 to 5 nucleotides and (ii-2) a sequence (D) which is added to the 5' end of the sequence (B) and comprises 0 to 5 nucleotides, wherein a base in the nucleotide of the 5' end of the sense sequence is guanine, a base in the nucleotide of 3' end of the sense sequence is adenine or uracil, and the number of the nucleotides in the sense sequence is one by which number RNA interference can occur without cytotoxicity;
selecting as the antisense sequence a sequence (E) which is complementary to the sense sequence, wherein the nucleotides of the 5' end and 3' end of the antisense sequence are entirely complementary to the correspondent nucleotides of the sense sequence, respectively, the 7-bp-long region of the 5' terminal of the antisense sequence is rich in at least one base selected from the group consisting of adenine and uracil, and the number of the nucleotides in the antisense sequence is one by which number RNA interference can occur without cytotoxicity; and
selecting as the trimming sequence a sequence (F) which comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
wherein the sense sequence, the trimming sequence, and the antisense sequence are consecutive, and this consecutive sequence does not comprise four or more consecutive nucleotides of which bases are uracils.

When the sense sequence consists of the sequence (B), the first method can also be specified as follows:
A method for designing a sequence of an shRNA which can cause an RNA interference in mammalian cells and which is expressed by an RNA polymerase III-type promoter, comprising:
   retrieving, from a sequence of a target gene which is calculated to suffer from an RNA interference, a region which satisfies the following rules (1) to (4):
      (1) the base of the nucleotide at the 3' end o f the retrieved region is adenine, thymine, or uracil;
      (2) the base of the nucleotide at the 5' end of the retrieved region is guanine or cytosine;
      (3) the 7-bp-long region of the 3' terminal of the retrieved region is rich in at least one base selected from the group consisting of adenine, thymine and uracil; and
      (4) the number of the nucleotides in the retrieved region is such that RNA interference can occur without cytotoxicity;
   and arranging a sense sequence, a trimming sequence, and an antisense sequence in this order;
wherein the sense sequence is homologous to the sequence of the retrieved region wherein the base of the terminal nucleotide of the 3' end of the sense sequence is adenine or uracil, and the base of the terminal nucleotide of the 5' end o f the sense sequence is guanine;
the antisense sequence is complementary to the sense sequence wherein the nucleotides of the 5' end and the 3' end of the antisense sequence are entirely complementary to the correspondent nucleotides of the sense sequence, respectively;
the trimming sequence comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides of which the bases are selected from the group consisting of adenine, uracil, guanine, and cytosine and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other; and
the sense sequence, the trimming sequence, and the antisense sequence are consecutive, and this consecutive sequence does not comprise four or more consecutive nucleotides of which bases are uracils.

For expression of the shRNA, vectors such as plasmids are used. In this invention, vectors which comprise RNA polymerase III-type transcriptional promoters are used. Examples of the RNA polymerase III-type transcriptional promoters include U6 promoter, SP6 promoter, T3 promoter, and class III of T7 promoter. Promoters which can induce expression only under specific conditions, e.g., a Tet inducement promoter can be used.

The shRNA that are designed by the first method comprises the sense sequence, the trimming sequence, and the antisense sequence in this order, at least a part of the trimming sequence portion can form a loop or hairpin structure, and the sense sequence portion and the antisense sequence portion can together form a double-stranded portion.

The sense sequence is a sequence (A) consisting of (i) a sequence (B) which is homologous to a part of a sequence (b) of a target gene which is calculated to suffer from an RNA interference and (ii) at least one sequence selected from the group consisting of (ii-1) a sequence (C) which is added to the 3' end of the sequence (B) and comprises 0 to 5 nucleotides and (ii-2) a sequence (D) which is added to the 5' end of the sequence (B) and comprises 0 to 5 nucleotides. It is preferable that the sequences (D) and (C) each comprise 0 nucleotides, in other word, there is no sequence (D) or (C).

The "target gene" means a gene on which RNA interference is desired i.e. to which RNA intereference is to be directed. The "sequence (b) of the target gene" may be set as one coding region, a plurality of coding regions, or all the polynucleotides whose sequences have been revealed.

The "gene" means a medium which codes genetic information. The "gene" is made of a substance, such as DNA, RNA, or a complex of DNA and RNA, which codes genetic information.

Organisms having target genes, i.e., the targeting organisms, are not specifically restricted. For example, prokaryotes such as E. Coli, microorganisms such as yeast and fungus, animals (mammals), insects, and plants can be used.

The sense sequence is a sequence (B) that is homologous to a part of a sequence (b) of a target gene, a sequence in which 1 to 5 nucleotides (the sequence (C)) are added to the 3' end of the sequence (B), a sequence in which 1 to 5 nucleotides (the sequence (D)) are added to the 5' end of the sequence (B), or a sequence in which 1 to 5 nucleotides (the sequence (C)) and 1 to 5 nucleotides (the sequence (D)) are added to the 3' end and the 5' end of the sequence (B), respectively.

The phrase "homologous sequence" refers to the same sequence and to a sequence in which mutations, such as deletions, substitutions, and additions, have occurred to the selected sequence which is a part of the sequence (b) to an extent that the function of causing RNA interference has not been lost.

Although depending on the conditions, such as the type of the target gene and the sequence, the range of the allowable mutation, in terms of homology, is preferably 80% or more, more preferably 90% or more, and still more preferably 95% or more. When homology in the range of the allowable mutation is calculated, desirably, the numerical values calculated using the same search algorithm are compared. The search algorithm is not particularly limited. A search algorithm suitable for searching for local sequences is preferable. More specifically, BLAST, ssearch, and the like can preferably be used.

As explained above, in the sense sequence, a few nucleotides can be mutated from the partial sequence of the sequence (b) of the target gene. However, it is especially preferable that the number of the nucleotides of the sense sequence is the same as that of the nucleotides of the selected partial sequence of the sequence (b). In the case where the number of the nucleotides are the same, the allowance for change (mutation) may be, for example, as follows: the rate of nucleotides that may be mutated is preferably 20% or less, more preferably 10% or less, and particularly preferably 5% or less. For example, when a sequence having 19 nucleotides is designed, preferably 16 or more nucleotides and more preferably 18 or more nucleotides correspond to those of the selected sequence.

In the sense sequence, the base of the nucleotide of the 5' end is guanine and that of the 3' end is adenine or uracil. Thus, if the selected sequence that is a part of the sequence (b) satisfies these rules, the selected sequence can be used as it is. However, even if the selected sequence does not satisfy these rules, by changing the type of the nucleotide, or by adding 1 to 5 nucleotides to the 3' end and/or the 5' end of the selected sequence, a sequence satisfying the above rules can be generated.

The number of the nucleotides of the sense sequence is one by which number the RNA interference can occur without cytotoxicity. The number is usually 13 to 28, preferably 16 to 22, more preferably 18 to 22, and particularly preferably 19.

The antisense sequence is the sequence (E) that is complementary to the sense sequence. The term "complementary" commonly means that, for example, when a base of a nucleotide in a sense sequence is adenine, in an antisense sequence a base of a nucleotide which corresponds to the nucleotide in the sense sequence is uracil (in the case of RNA) or thymine (in the case of DNA). In this specification, the term refers to not only the case where all nucleotides in an antisense sequence correspond to all nucleotides in a sense sequence, but also to the case where a part of the nucleotides in the antisense sequence do not correspond to a part of the nucleotides in the sense sequence to an extent that the function of causing RNA interference is not lost. The phrase "entirely complementary" refers to the case where uracil or thymine correspond to adenine, cytosine corresponds to guanine, adenine corresponds to uracil or thymine, and guanine corresponds to cytosine.

The nucleotides of the 3' end and the 5' end of the antisense sequence are entirely complementary to the correspondent nucleotides in the sense sequence, respectively.

In the 7-bp-long region of the 5' terminal of the antisense sequence, at least one base selected from the group consisting of adenine and uracil is rich. The term "rich" means that the frequency of a specific base appearing is high. Specifically, it means that the 7-bp-long region of the 5' terminal of the antisense sequence contains at least one base selected from the group consisting of adenine and uracil in an amount of usually 40% or more and preferably 50% or more. In other words, in the 7-bp-long region of the 5' terminal of the antisense sequence, preferably at least 3 bases, more preferably at least 4 bases, and particularly preferably at least 5 bases, are selected from the group consisting of adenine and uracil.

The number of the nucleotides in the antisense sequence is one by which number RNA interference can occur without cytotoxicity. This number is usually 13 to 28, preferably 16 to 22, more preferably 18 to 20, and particularly preferably 19.

The trimming sequence is arranged between the sense sequence and the antisense sequence, and is cleaved with a base-specific RNase ("Dicer" in cells). Thus, this sequence should contain a sequence which is recognized by the base-specific RNase to be used. The trimming sequence contains 5 to 52 nucleotides, preferably 6 to 42 nucleotides, more preferably 7 to 32 nucleotides, and particularly preferably 8 to 22 nucleotides.

The trimming sequence is represented by the formula: (G or C)-X-Y-Z-(C or G). Namely, the base of one end of it which follows the sense sequence is guanine or cytosine, and the base of the other end of it, i.e., the base of the end which the antisense sequence follows, is entirely complementary to the base of one end.

The X region and the Z region each comprise 0 to 10 nucleotides, preferably 1 to 8 nucleotides, more preferably 2 to 6 nucleotides, and particularly preferably 2 nucleotides, of which the bases are selected from the group consisting of adenine, uracil, guanine, and cytosine. The X region and the Z region are complementary to each other when the trimming sequence is turned in the Y region.

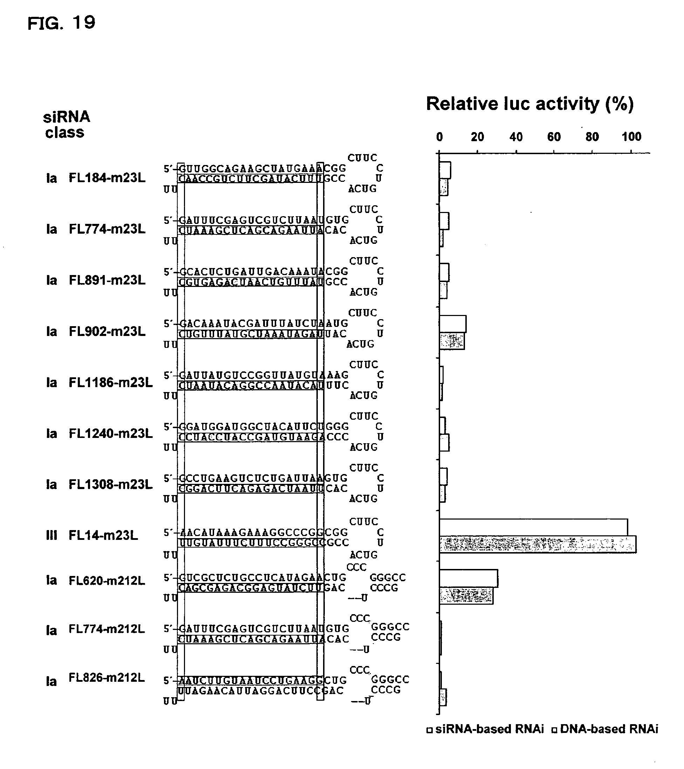

The Y region comprises 3 to 50 nucleotides, preferably 4 to 40 nucleotides, more preferably 5 to 30 nucleotides, particularly preferably 6 to 20 nucleotides, and most preferably 10 nucleotides, and at least the nucleotides of both ends of it are not complementary to each other. Because at least the nucleotides of both ends of the Y region are not complementary to each other, in a transcript, the Y region can be a loop or hairpin structure. The Y region may be a loop or hairpin structure as a whole. Or, a part of the Y region may be complementary. Namely, it can contain a part which constructs a complementary double strands in a transcript. Please see FL-620-m212L, FL774-m212L, and FL826-m212L in Figure19.

An siRNA that is a double-stranded RNA has overhang portions at the 3' ends of the sense sequence and the antisense sequence which are usually based on the sequence of the target gene. Namely, the overhang portions are ones which are parts of the sequence (b) and follow or border on the selected sequence in the sequence (b). Thus, in the trimming sequence the nucleotide (G or C) which follows the sense sequence, or the nucleotide (G or C) and at least one nucleotide at the 5' terminal of the X region which follows the nucleotide (G or C), preferably the nucleotide (G or C) and two nucleotides at the 5' terminal of the X region which follows the nucleotide (G or C), may correspond to nucleotides of one overhang portion of the siRNA.

As the trimming sequence, a sequence of a loop portion of human miRNA, e.g., miR-23 and miR-212, or another sequence which was obtained by mutating (or changing) at least one nucleotide in the loop portion of human miRNA can be used. It is preferable that the trimming sequence that is selected, which is contained in an shRNA, and the shRNA can be easily transferred from the nucleus to the cytoplasm, cleaved by RNase with difficulty, and can be stably maintained.

The sense sequence, the trimming sequence, and the antisense sequence are consecutive. This consecutive sequence should not contain four or more consecutive nucleotides of which the bases are uracils. This is because the RNA polymerase III-type transcriptional promoter finishes the transcription between the second uracil and the third uracil in four or more consecutive uracils.

Preferably, the sequence (A), i.e., the sense sequence, and the sequence (E), i.e., the antisense sequence, comprise no long stretches of nucleotides of which the bases are guanine and/or cytosine. If this rule is satisfied, the efficacy of the RNA interference is still enhanced.

The phrase "long stretches of nucleotides" means consecutive nucleotides of which the bases are guanine and/or cytosine and the number of the consecutive nucleotides is at least 30% of the nucleotides in the sense or antisense sequence. For example, if the number of nucleotides in the sense sequence is 19, it is preferable that the sense sequence does not contain at least 7 consecutive nucleotides of which the bases are guanine and/or cytosine.

To the 3' end of the antisense, an overhang portion comprising 1 to 3 (preferably 2) nucleotides may be added. This overhang portion is usually based on the sequence of the target gene. Namely, the overhang portion has an antisense sequence that is homologous to a partial sequence of the sequence (b) that borders on the 5' end of the selected sense sequence in the sequence (b). In the shRNA that is a transcript, the overhang portion comprises two uracils, which shows completion of transcription.

As the sequence (A), i.e., the sense sequence, a sequence which is identical to a part of the sequence (b) of the target gene, and as the sequence (E), i.e., the antisense sequence, a sequence which is entirely complementary to the sense sequence can be selected.

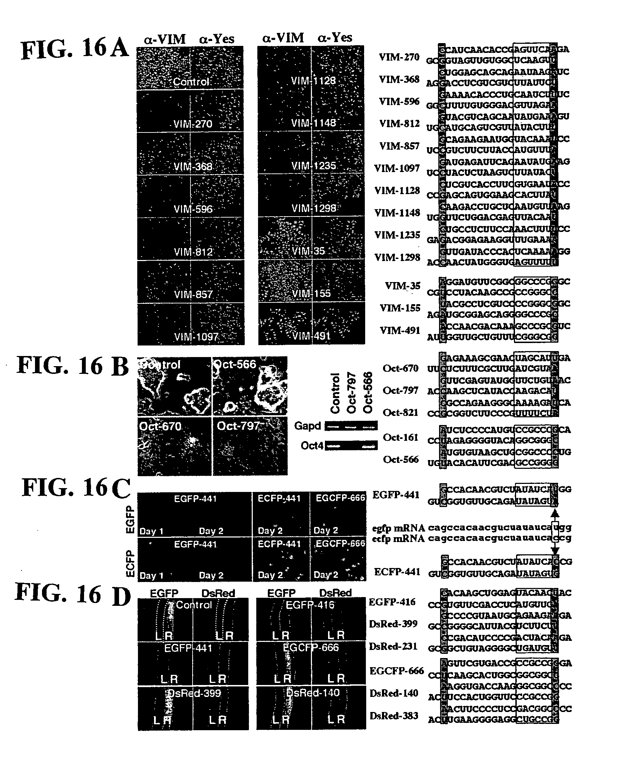

As the sequence (A), i.e., the sense sequence, a sequence which is identical to a part of the sequence (b) of the target gene except for the nucleotide of the 5' end, and as the sequence (E), i.e., the antisense sequence, a sequence which is entirely complementary to the sense sequence can be selected. An example is the sequence which is shown in Figure 16C.

An shRNA is cleaved by Dicer in cells to be an siRNA. It is preferable that the lengths of the sense sequence and the antisense sequence are selected so that the cleavage sites by Dicer are between the second nucleotide and the third nucleotide from the 5' end of the trimming sequence, and between the trimming sequence and the antisense sequence. To realize this aim, the number of the nucleotides in the sense or antisense sequence is usually 13 to 28, preferably 16 to 22, more preferably 18 to 20, and particularly preferably 19.

The base of the second nucleotide from the 5' end of the antisense sequence, and/or the base of the nucleotide of the 3' end of the trimming sequence (i.e., the nucleotide that borders on the antisense sequence) are preferably adenine or uracil. In this case, the cleavage site by Dicer in the antisense side may be between the second nucleotide from the 5' end of the antisense sequence and the nucleotide at the 5' end of the antisense sequence, between the antisense sequence and the trimming sequence, or between the nucleotide at the 3' end of the trimming sequence and the second nucleotide from the 3' end of the trimming sequence. This means that an siRNA having a specific sequence for causing RNA interference at a high efficiency can be obtained even if a site that was cleaved by Dicer was shifted from an intended site.

The total number of the nucleotides of the shRNA is usually 30 to 90, preferably 40 to 80, and more preferably 45 to 75.

In the case where the sense sequence comprises no sequence (C) or sequence (D) in the first method for designing a sequence of an shRNA, first, from a sequence of a target gene which is calculated to suffer from an RNA interference, a region which satisfies the following rules (1) to (4) is retrieved or selected:
(1) the base of the nucleotide of the 3' end of the retrieved region is adenine, thymine, or uracil;
(2) the base of the nucleotide of the 5' end of the retrieved region is guanine or cytosine;
(3) the 7-bp-long region of the 3' terminal of the retrieved region is rich in, at least one base selected from the group consisting of adenine, thymine, and uracil; and
(4) the number of the nucleotides in the retrieved region is one by which number RNA interference can occur without cytotoxicity.

The bases of the nucleotides in the retrieved region refer to those in the sense strand of the target gene.

Other rules have already been explained in the above explanation for the first method for designing a sequence of an shRNA.

A second method is one for designing a sequence of an shRNA which can cause RNA interference, which is expressed by an RNA polymerase III-type promoter, and which comprises an antisense sequence, a trimming sequence, and a sense sequence in this order, comprising:
selecting as the sense sequence a sequence (J) consisting of (i) a sequence (K) which is homologous to a part of a sequence (b) of a target gene which is calculated to suffer from RNA interference and (ii) at least one sequence selected from the group consisting of (ii-1) a sequence (L) which is added to the 3' end o f the sequence (K) and comprises 0 to 5 nucleotides and (ii-2) a sequence (M) which is added to the 5' end of the sequence (K) and comprises 0 to 5 nucleotides, wherein the base in the nucleotide of the 5' end of the sense sequence is guanine or cytosine, a base in the nucleotide of the 3' end of the sense sequence is cytosine or uracil, the 7-bp-long region of the 3' terminal of the sense sequence is rich in at least one base selected from the group consisting of adenine and uracil, and the number of the nucleotides in the sense sequence is one by which number RNA interference can occur without cytotoxicity;
selecting as the antisense sequence a sequence (N) which is complementary to the sense sequence, wherein the nucleotides of the 5' end and 3' end of the antisense sequence are entirely complementary to the correspondent nucleotides of the sense sequence, respectively, and the number of the nucleotides in the antisense sequence is one by which number RNA interference can occur without cytotoxicity; and
selecting as the trimming sequence a sequence (F) which comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides of which the bases are selected from the group consisting of adenine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
wherein the antisense sequence, the trimming sequence, and the sense sequence are consecutive, and this consecutive sequence does not comprise four or more consecutive nucleotides of which the bases are uracils.

When the sense sequence consists of the sequence (K), the first method can also be specified as follows:

A method for designing a sequence of an shRNA which can cause RNA interference in mammalian cells and which is expressed by an RNA polymerase III-type promoter, comprising:
retrieving, from a sequence of a target gene which is calculated to suffer from RNA interference, a region which satisfies the following rules (5) to (8):
   (5) the base of the nucleotide of the 3' end of the retrieved region is cytosine, thymine, or uracil;
   (6) the base of the nucleotide of the 5' end of the retrieved region is guanine or cytosine;
   (7) the 7-bp-long region of the 3' terminal of the retrieved region, is rich in at least one base selected from the group consisting of adenine, thymine, and uracil; and
   (8) the number of nucleotides in the retrieved region is one by which number RNA interference can occur without cytotoxicity;
and arranging an antisense sequence, a trimming sequence, and a sense sequence in this order;
wherein the antisense sequence is complementary to the sequence of the retrieved region wherein the base of the nucleotide of the 3' end of the antisense sequence is cytosine or guanine, and the base of the nucleotide of the 5' end of the antisense sequence is guanine or adenine;
the sense sequence is complementary to the antisense sequence wherein the nucleotides of the 5' end and the 3' end of the sense sequence are entirely complementary to the correspondent nucleotides of the antisense sequence, respectively;
the trimming sequence comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides of which the bases are selected from the group consisting of adenine, uracil, guanine, and cytosine and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other; and
the antisense sequence, the trimming sequence, and the sense sequence are consecutive, and this consecutive sequence does not comprise four or more consecutive nucleotides of which the bases are uracils.

The shRNA that are designed by the second method comprises the antisense sequence, the trimming sequence, and the sense sequence in this order, at least a part of the trimming sequence portion can form a loop or hairpin structure, and the antisense sequence portion and the sense sequence portion can form a double-strand.

The sense sequence is a sequence (K) that is homologous to a part of a sequence (b) of a target gene, a sequence in which 1 to 5 nucleotides (the sequence (L)) are added to the 3' end of the sequence (K), a sequence in which 1 to 5 nucleotides (the sequence (M)) are added to the 5' end of the sequence (K), or a sequence in which 1 to 5 nucleotides (the sequence (L)) and 1 to 5 nucleotides (the sequence (M)) are added to the 3' end and the 5' end of the sequence (K), respectively. It is preferable that the sequences (L) and (M) each comprise 0 nucleotide, in other word, there is no sequence (L) or (M).

The terms "homologous," "complementary," "mutation," and the like have already been explained in the explanations for the first method for designing a sequence of an shRNA.

In the sense sequence, the base of the nucleotide of the 5' end is guanine or cytosine and that of the 3' end is cytosine or uracil. Thus, if the selected sequence that is a part of the sequence (b) satisfies these rules, the selected sequence can be used as it is. However, even if the selected sequence does not satisfy these rules, by changing the type of the nucleotide, or by adding 1 to 5 nucleotides to the 3' end and/or the 5' end of the selected sequence, a sequence satisfying the above rules can be generated.

Also, the term "rich" and the phrase "one by which number the RNA interference can occur without cytotoxicity" have been already explained in the explanations for the first method for designing a sequence of an shRNA.

The means of "the nucleotides of the 5' end and the 3' end of the sense sequence are entirely complementary to the correspondent nucleotides of the antisense sequence, respectively" can be understood from the corresponding explanation for the antisense sequence for the first method for designing a sequence of an shRNA.

The antisense sequence is the sequence (N) that is complementary to the sense sequence.

The trimming sequence is the same as that in the first method for designing a sequence of an shRNA.

The antisense sequence, the trimming sequence, and the sense sequence are consecutive. This consecutive sequence should not contain four or more consecutive nucleotides of which the bases are uracils.

The base of the second nucleotide from the 5' end of the sense sequence, and/or the base of the nucleotide of the 3' end of the trimming sequence (i.e., the nucleotide that borders on the sense sequence) are preferably guanine or cytosine. In this case, the cleavage site by Dicer in the sense side may be between the second nucleotide from the 5' end of the sense sequence and the nucleotide at the 5' end of the sense sequence, between the sense sequence and the trimming sequence, or between the nucleotide at the 3' end of the trimming sequence and the second nucleotide from the 3' end of the trimming sequence. This means that an siRNA having a specific sequence for causing an RNA interference at a high efficiency can be obtained even if a site that was cleaved by Dicer was shifted from an intended site.

Preferably, the sequence (J), i.e., the sense sequence, and the sequence (N), i.e., the antisense sequence, comprise no long stretches of nucleotides of which the bases are guanine and/or cytosine. If this rule is satisfied, the efficacy of the RNA interference is still enhanced.

The means of "long stretches of nucleotides of which the bases are guanine and/or cytosine" have been already explained in the explanations for the first method for designing a sequence of an shRNA.

To the 3' end of the sense sequence, an overhang portion comprising 1 to 3 (preferably 2) nucleotides may be added. This overhang portion is usually based on the sequence of the target gene. Namely, the overhang portion has a sense sequence that is homologous to a partial sequence of the sequence (b) that follows or borders on the 3' end of the selected sense sequence in the sequence (b). In the shRNA that is a transcript, the overhang portion comprises two uracils, which shows completion of the transcription.

As the sequence (J), i.e., the sense sequence, a sequence which is identical to a part of the sequence (b) of the target gene, and as the sequence (N), i.e., the antisense sequence, a sequence which is entirely complementary to the sense sequence can be selected.

As the sequence (J), i.e., the sense sequence, a sequence which is identical to a part of the sequence (b) of the target gene except for the nucleotide of the 5' end, and as the sequence (N), i.e., the antisense sequence, a sequence which is entirely complementary to the sense sequence can be selected.

An shRNA is cleaved by Dicer in cells to be an siRNA. It is preferable that the lengths of the sense sequence and the antisense sequence are decided so that the cleavage sites by Dicer are between the second nucleotide and the third nucleotide from the 5' end of the trimming sequence, and between the trimming sequence and the sense sequence.

The total number of nucleotides of the shRNA is usually 30 to 90, preferably 40 to 80, and more preferably 45 to 75.

An example of the shRNA that was designed according to the second method is FL826-m212L in Figure 19.

In the case where the sense sequence comprises no sequence (L) or sequence (M) in the second method for designing a sequence of an shRNA, first, from a sequence of a target gene which is calculated to suffer from RNA interference, a region which satisfies the following rules (5) to (8) is retrieved or selected:
(5) the base of the nucleotide of 3' end of the retrieved region is cytosine, thymine, or uracil;
(6) the base of the nucleotide of 5' end of the retrieved region is guanine or cytosine;
(7) the 7-bp-long region of the 3' terminal of the retrieved region, is rich in at least one base selected from the group consisting of adenine, thymine, and uracil; and
(8) the number of nucleotides in the retrieved region is one by which number RNA interference can occur without cytotoxicity.

The bases of the nucleotides in the retrieved region refer to those in the sense strand of the target gene.

Other rules have already explained in the above explanation for the second method for designing a sequence of an shRNA.

The oligo- or polynucleotides of this invention are those comprising, in this order, the sense sequence, the trimming sequence, and the antisense sequence, which have been already explained in the explanations for the first method for designing a sequence of an shRNA, and those comprising, in this order, the antisense sequence, the trimming sequence, and the sense sequence, which have been already explained in the explanations for the second method for designing a sequence of an shRNA. The sense sequence and the antisense sequence may be combined i.e. bound, to each other by hydrogen bonding to become a double-stranded portion.

The oligo- or polynucleotides of this invention may be DNA, RNA, or comprise DNA and RNA (so-called chimera type). In embodiments of the invention, when the molecule of the invention or designed or used according to methods of the invention is or contains DNA thymine bases as opposed to uracil bases are present (or absent) and vice versa in the case of RNA molecules.

In the case where the oligo- or polynucleotide of this invention is DNA, it can be synthesized by, e.g., a known chemical synthetic method or a known enzymatic synthetic method.

In the case where the oligo- or polynucleotide of this invention is RNA, it can be synthesized by a chemical synthetic method or a method using common biotechnology. A suitable biotechnological method, for example, comprises first preparing a DNA strand having a predetermined sequence, and then synthesizing a single-stranded RNA by using a transcriptional enzyme and using the synthesized DNA as a template. A double-stranded RNA can be prepared by, e.g., a method for preparing the double-stranded RNA from two pieces of single-stranded RNAs.

With respect to the basic technique in molecular biology, there are many standard, experimental manuals, for example, BASIC METHODS IN MOLECULAR BIOLOGY (1986); Sambrook et al., MOLECULAR CLONING; A LABORATORY MANUAL, Second Edition, Cold Spring Harbor Laboratory Press, Cold Spring Harbor, N.Y. (1989); Saibo-Kogaku Handbook (Handbook for cell engineering), edited by Toshio Kuroki et al., Yodosha (1992); and Shin-Idenshi-Kogaku Handbook (New handbook for genetic engineering), edited by Muramatsu et al., Yodosha (1999).

This invention also provides a double-stranded DNA comprising the oligo-or polynucleotide of this invention as a single-stranded DNA and another DNA which is complementary to the single-stranded DNA, a double-stranded RNA comprising the oligo- or polynucleotide of this invention as a single-stranded RNA and another RNA which is complementary to the single-stranded RNA, and those comprising a double-stranded portion and single-stranded (loop) portion, like an shRNA. The method for synthesizing a double-stranded DNA or RNA from a single-stranded DNA or RNA are known. According to this invention, a double-stranded, chimera type DNA/RNA and a double-stranded, hybrid type DNA/RNA in which one strand is DNA and the other is RNA can also be obtained.

The recombinant DNA of this invention is, e.g., a plasmid having an RNA polymerase III-type transcriptional promoter, into which a double-stranded DNA of this invention has been inserted downstream of the promoter.

For example, U6 gene and H1 gene comprise RNA polymerase III-type transcriptional promoters. Specific examples of the promoter include U6 promoter, class III of T7 promoter, SP6 promoter, and T3 promoter. Promoters which can induce expression only under specific conditions, e.g., a Tet inducement promoter can be used.

Plasmids and vectors which can be used in this invention for preparing the recombinant DNA of this invention are not particularly restricted. Examples of suitable plasmids and vectors include pScilencer, psHpromoter, and pGE-1.

Plasmids and vectors are not restricted to those having inherently an RNA polymerase III-type transcriptional promoter. If a plasmid does not have the promoter, the promoter can be inserted into the plasmid at a suitable site.

The recombinant DNA of this invention can be prepared by inserting a double-stranded DNA of this invention into a suitable site, e.g., a multiple cloning site, of a plasmid. To insert the double-stranded DNA, sequences that match the sequences of restriction sites in a cloning site may be added to the 5' end and the 3' end of the double-stranded DNA.

The recombinant DNA of this invention can be used for gene therapy or prevention of a disease. In the administration of the recombinant DNA, for example, a drug delivery system using liposomes can be used for transfer to the target gene.

In the cell transfected with the recombinant DNA of this invention, a specific gene has been knocked out. The cell can be prepared by transfecting the cell in which RNA interference is desired with the recombinant DNA of this invention under a known, suitable condition, and culturing the transfected cell.

The method for causing RNA interference in a mammalian cell, in other words, a method for silencing an expression of a gene, comprises transfecting the mammalian cell with the recombinant DNA of this invention.

The mammalian cell may be a cultured cell, a cultured tissue, or a living body.

The cell in which RNA interference is desired, namely, the cell comprising the target gene whose expression is to be silenced, may be one derived from a biological species having the target gene which sequence may be used for designing a sequence of a shRNA or one derived from other biological species. However, the biological species of the cell that is used in the experiment of RNA interference is the same or close to the species having the target gene used in the above design process, as the target gene can be more specifically and effectively inhibited.

The transfection of the mammalian cell with the recombinant DNA of this invention can be carried out by a known method.

In the case where the sense sequence of the oligo- or polynucleotide of this invention consists of the sequence (B), i.e., there are no sequence (D) or sequence (C), and the antisense sequence is entirely complementary to the sense sequence, the method for processing information for designing an shRNA sequence of this invention can be carried out with a computer. The program, namely, the first program of this invention, comprises at least the following steps (1) to (8):
(1) a step comprising acquiring information of a nucleotide sequence of a target gene for RNA interference and creating information of partial sequences each having a predetermined number of consecutive nucleotides from the acquired information;
(2) a step of judging a nucleotide of the 5' end wherein a sequence(s) containing a nucleotide of which the base is guanine at the 5' end is selected from partial sequences in the created information of partial sequences;
(3) a step of judging a nucleotide of the 3' end wherein a sequence(s) containing a nucleotide of which the base is adenine, thymine, or uracil at the 3' end is selected from the partial sequences in the created information of partial sequences;
(4) a step of judging whether specific nucleotides are contained in a sequence wherein a sequence(s) rich in at least one base selected from the group consisting of adenine, thymine, and uracil in 7 nucleotides at the 3' terminal is selected from the partial sequences in the created information of partial sequences;
(5) a step of designing a sense sequence/antisense sequence wherein for each of the partial sequence(s) that were selected in all of the above steps (2), (3), and (4), a sense sequence that is identical to the selected sequence and an antisense sequence that is entirely complementary to the sense sequence are decided;
(6) a step of designing a trimming sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other,
(7) a step of designing an RNA sequence wherein the sequences that were decided in the above steps (5) and (6) are consecutively arranged in order of the sense sequence, the trimming sequence, and the antisense sequence; and
(8) a step of selecting at least one sequence which does not contain four or more consecutive nucleotides of which the bases are thymine and/or uracil from the RNA sequences designed in the above step (7).

Among above steps (1) to (8), step (1) is firstly carried out. Steps (2) to (5) are a series of steps. Among steps (2) to (5), the order of steps (2) to (4) is not restricted. Step (6) is carried out after step (1) or (5).

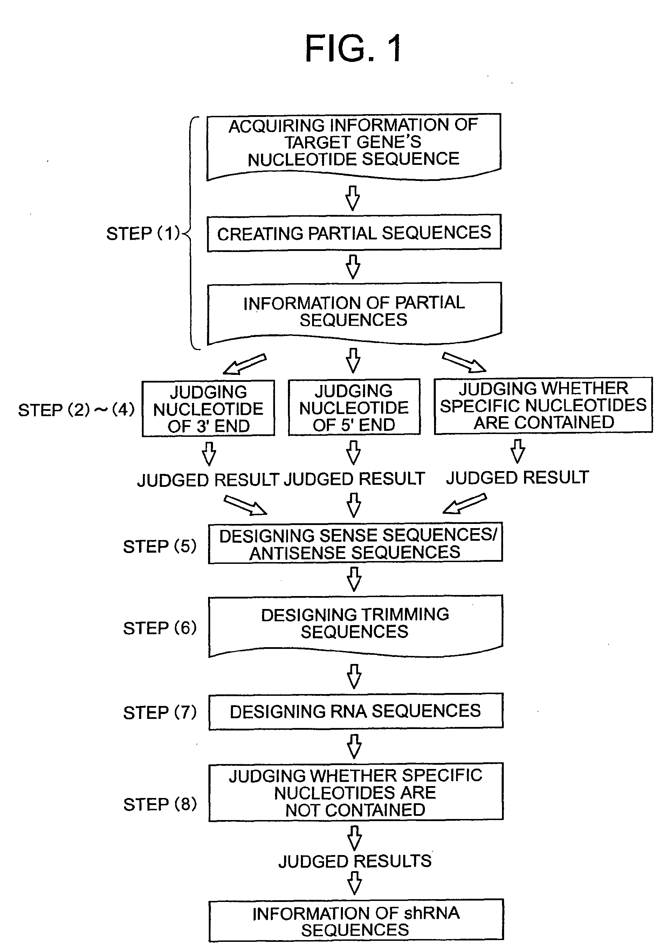

Figure 1 shows an example of the first program of this invention as a diagram.

In step (1), information of a nucleotide sequence of a target gene for RNA interference is acquired and information of the partial sequences each having a predetermined number of consecutive nucleotides is created from the acquired information.

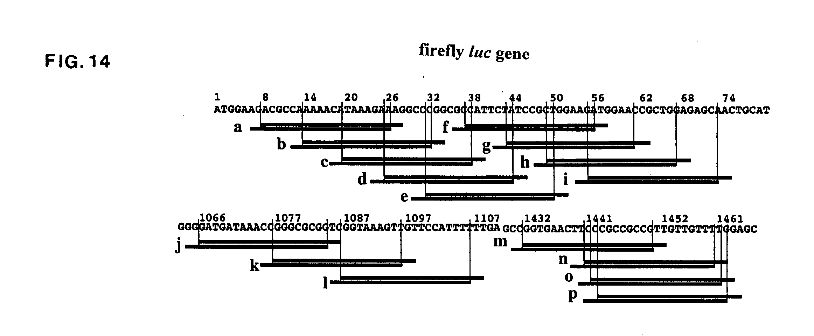

For example, as shown in Figure 14, from a nucleotide sequence of a target gene, partial sequences each having a predetermined number of consecutive nucleotides are specified in the way that the positions corresponding to the first, i.e., the 5' end, nucleotide of the partial sequences are shifted from the 5' end to the 3' end of the nucleotide sequence of the target gene by a predetermined number of nucleotides.

In this step, information of partial sequences each having a predetermined number of consecutive nucleotides can be created from a segment corresponding to a coding region or transcription region of the target gene.

Further, a portion which is common in a plurality of nucleotide sequence information derived from different organisms (e.g., human nucleotide sequence information and mouse nucleotide sequence information) may be used as the information of a nucleotide sequence of a target gene. Furthermore, a portion which is common in a plurality of analogous nucleotide sequence information in the same species may be used as the information of a nucleotide sequence of a target gene.

In step (1), information of the partial sequences each comprising an overhang portion may be created. Specifically, for example, information of partial sequences may be created in which for each sequence information on whether an overhang portion is present is added. The information of partial sequences and the information whether an overhang portion is present may be correlated with each other.

The predetermined number of consecutive nucleotides is usually 13 to 28, preferably 16 to 22, more preferably 18 to 20, and particularly preferably 19 if the overhang portion is not included. When the number is 19 in the case of a predetermined number of consecutive nucleotides, the number becomes 23 when the overhang portion of 2 nucleotides is included. With such a number of nucleotides, RNA interference can occur in mammalian cells without cytotoxicity.

Then, for each sequence in the information of partial sequences created in step (1), steps (2), (3), and (4) are carried out. The order for carrying out these steps is not restricted.
Step (2): for each partial sequence, a nucleotide of the 5' end is judged to establish whether its base is guanine, and partial sequences in which the base at the 5' end is guanine are selected.
Step (3): for each partial sequence, a nucleotide of the 3' end is judged to establish whether its base is adenine, thymine, or uracil, and partial sequences in which the base at the 3' end is adenine, thymine, or uracil are selected.
Step (4): for each partial sequence, 7 nucleotides at 3' terminal are judged to establish whether those nucelotides are rich in at least one base selected from the group consisting of adenine, thymine, and uracil, and partial sequences in which the bases are rich are selected. The meaning of "rich" has already been explained.

For step (2), for example, when the 5' end nucleotide contains guanine, "1" may be outputted as the determination result, and when it is not, "0" may be outputted. For steps (3) and (4) the same output can be carried out.

When partial sequences each having overhang portions are used, only the sequence segments excluding the overhang portions are used for the judgements in steps (2) to (4).

Steps (2) to (4) may be carried out in this order, namely, step (2) is carried out, and only for the sequences that have been selected in step (2), step (3) is carried out, and then only for the sequences that have been selected in step (3), step (4) is carried out. Or, for all sequences steps (2) to (4) are respectively carried out, and then sequences which have been selected in all of the steps (2) to (4) are selected.

The partial sequences which have been selected in all of steps (2) to (4) may be compared with a result which has been obtained by retrieving other nucleotide sequence information (e.g., sequence information published in a public database, such as RefSeq of NCBI) using a known homology retrieval method such as BLAST, FASTA, or ssearch. Then, among partial sequences that have been selected by steps (2) to (4), only those which are also included in the result of the known homology retrieval method may be selected.

For each of the sequences that have been selected in all of steps (2) to (4), a sense sequence/antisense sequence is designed (Step (5)).

In step (5), a sense sequence that is identical to the selected sequence and an antisense sequence that is entirely complementary to the sense sequence are decided.

In step (6), the trimming sequence is decided. The trimming sequence comprises 5 to 52 nucleotides and is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other. Thus, at least one sequence that satisfies the above restrictions may be designed in advance, and may be input to a computer.

When information of partial sequences each comprising overhang portions is used, the overhang portion at the 3 ' end of the sense sequence is a part of the trimming sequence (the 5 ' end of the trimming sequence).

In step (7), the sequences that were decided in the above steps (5) and (6) are consecutively arranged in the order of the sense sequence, the trimming sequence, and the antisense sequence. Thus, an RNA sequence is decided.

In step (8), each of the sequences which have been designed in the above step (7) is judged to establish whether the sequence comprises four or more consecutive nucleotides of which the bases are thymine and/or uracil, and sequences not comprising the four or more consecutive nucleotides are selected.

By undergoing these steps, an shRNA sequence(s) which can cause RNA interference specific to a target gene can be designed.

In the case where the sense sequence of the oligo- or polynucleotide of this invention consists of the sequence (K), i.e., there is no sequence (L) or sequence (M), and the antisense sequence is entirely complementary to the sense sequence, the method for processing information for designing an shRNA sequence of this invention can be carried out with a computer. The program, namely, the second program of this invention, comprises at least the following steps (1) to (8):
(1) a step of acquiring information of a nucleotide sequence of a target gene for RNA interference and creating information of partial sequences each having a predetermined number of consecutive nucleotides from the acquired information;
(2) a step of judging a nucleotide of the 5' end wherein a sequence(s) containing a nucleotide of which the base is guanine or cytosine at the 5' end is selected from partial sequences in the created information of partial sequences;
(3) a step of judging a nucleotide of the 3' end wherein a sequence(s) containing a nucleotide of which the base is cytosine, thymine, or uracil at the 3' end is selected from the partial sequences in the created information of partial sequences;
(4) a step of judging whether specific nucleotides are contained in a sequence wherein a sequence(s) rich in at least one base selected from the group consisting of adenine, thymine, and uracil in 7 nucleotides at the 3' terminal is selected from the partial sequences in the created information of partial sequences;
(5) a step of designing a sense sequence/antisense sequence wherein for each of the partial sequence(s) that were selected in all of the above steps (2), (3), and (4), a sense sequence that is identical to the selected sequence and an antisense sequence that is entirely complementary to the sense sequence are decided;
(6) a step of designing a trimming sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
(7) a step of designing an RNA sequence wherein the sequences that were decided in the above steps (5) and (6) are consecutively arranged in order of the sense sequence, the trimming sequence, and the antisense sequence; and
(8) a step of selecting at least one sequence which does not contain four or more consecutive nucleotides of which the bases are thymine and/or uracil from the RNA sequences designed in the above step (7).

Among the above steps (1) to (8), step (1) is firstly carried out. Steps (2) to (5) are a series of steps. Among steps (2) to (5), the order of steps (2) to (4) is not restricted. Step (6) is carried out after step (1) or (5).

Figure 1 shows also an example of the second program of this invention as a diagram.

Step (1) is the same as that in the first program of this invention.

Then, for each sequence in the information of partial sequences created in step (1), steps (2), (3), and (4) are carried out. The order for carrying out these steps is not restricted.
Step (2): for each partial sequence, a nucleotide of the 5' end is judged to establish whether its base is guanine or cytosine, and partial sequences in which the base at the 5' end is guanine or cytosine are selected.
Step (3): for each partial sequence, a nucleotide of the 3' end is judged to establish whether its base is cytosine, thymine, or uracil, and partial sequences in which the base at the 3' end is cytosine, thymine, or uracil are selected.
Step (4): for each partial sequence, 7 nucleotides at 3' terminal are judged to establish whether those nucleotides are rich in at least one base selected from the group consisting of adenine, thymine, and uracil, and partial sequences in which the bases are rich are selected. The meaning of "rich" has already been explained.

For step (2), for example, when the 5' end nucleotide contains guanine or cytosine, "1" may be outputted as the determination result, and when it is not, "0" may be outputted. For steps (3) and (4) the same output can be carried out.

When partial sequences each having overhang portions are used, only the sequence segments excluding the overhang portions are used for the judgements in steps (2) to (4).

Steps (5) and (6) are the same as those in the first program of this invention.

In step (7), the sequences that were decided in the above steps (5) and (6) are consecutively arranged in the order of the antisense sequence, the trimming sequence, and the sense sequence. Thus, an RNA sequence is decided.

The step (8) is the same as that in the first program of this invention.

By undergoing these steps, an shRNA sequence(s) which can cause RNA interference specific to a target gene can be designed.

The term "program" means a data processing method described in any language or by any description method, and the program may have any format (e.g., source code or binary code). The computer program is executed by being loaded into a RAM or the like, and is executed together with the CPU.

The program is not always limited to one having a single system configuration, and may have a distributed system configuration including a plurality of modules or libraries, or may achieve its function together with another program, such as OS (Operating System).

This invention also relates to a computer-readable recording medium on which the above program is recorded.

Here, examples of the "recording medium" include any "portable physical medium," such as a flexible disk, an optomagnetic disk, a ROM, an EPROM, an EEPROM, a CD-ROM, a MO, a DVD, and a flash disk; any " fixed physical medium,", such as a ROM, a RAM, and a HD which are incorporated into various types of computer systems; and a "communication medium" which holds the program for a short period of time, such as a communication line or carrier wave, in the case when the program is transmitted via a network, such as a LAN, a WAN, and the Internet.

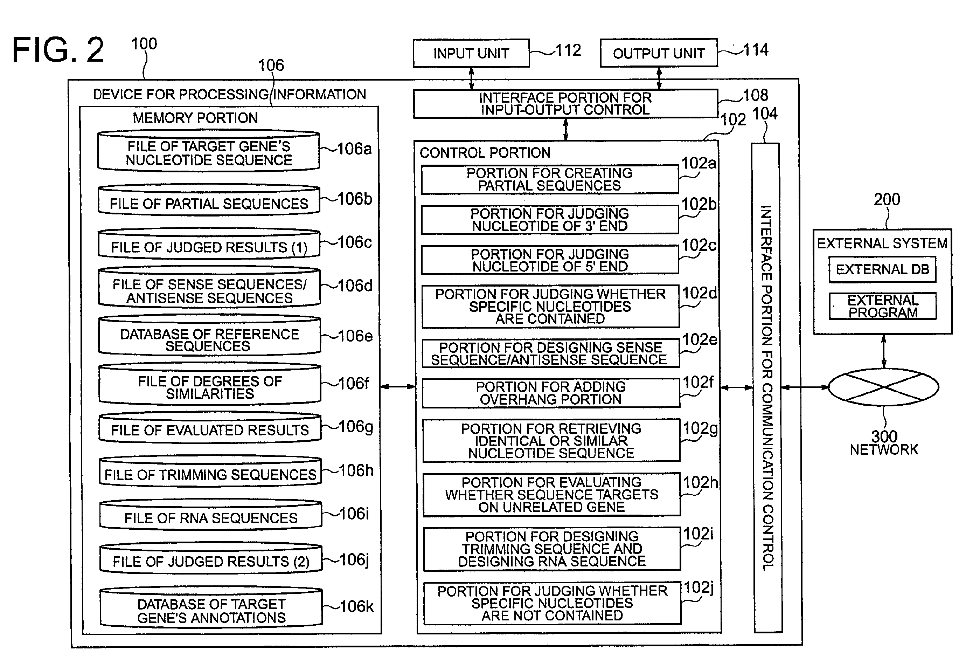

Next, the device (or data processing apparatus) for processing information of this invention is explained. Here, reference is made to Figure 2, which is a block diagram that shows an example of a system for processing information comprising the device of this invention.

In Figure 2, the device 100 for processing information schematically includes a control portion 102, such as a CPU, which controls the device 100 overall; an interface portion for communication 104 which is connected to a communication device (not shown in the drawing), such as a router, connected to a communication line or the like; an interface portion for input-output control 108 connected to an input unit 112 and an output unit 114; and a memory portion 106 which stores various databases and tables. These parts are connected via given communication channels in a communicable manner to each other. Furthermore, the device 100 is connected with the external system 200 via the network 300 in a communicable manner via a communication device, such as a router, and a wired or radio communication line. The external system 200 can provide databases such as sequence information of genes and structural information of proteins, and programs such as a program for homology retrieval.

Various databases (a file 106a of the target gene nucleotide sequence to a database 106k of target gene's annotations) which are stored in the memory portion 106 are storage means, such as fixed disk drives, for storing various programs, tables, files, databases, files for web pages, etc. These files may be stored to the memory portion 106 through a recording medium such as a flexible disk and a magneto-optical disk.

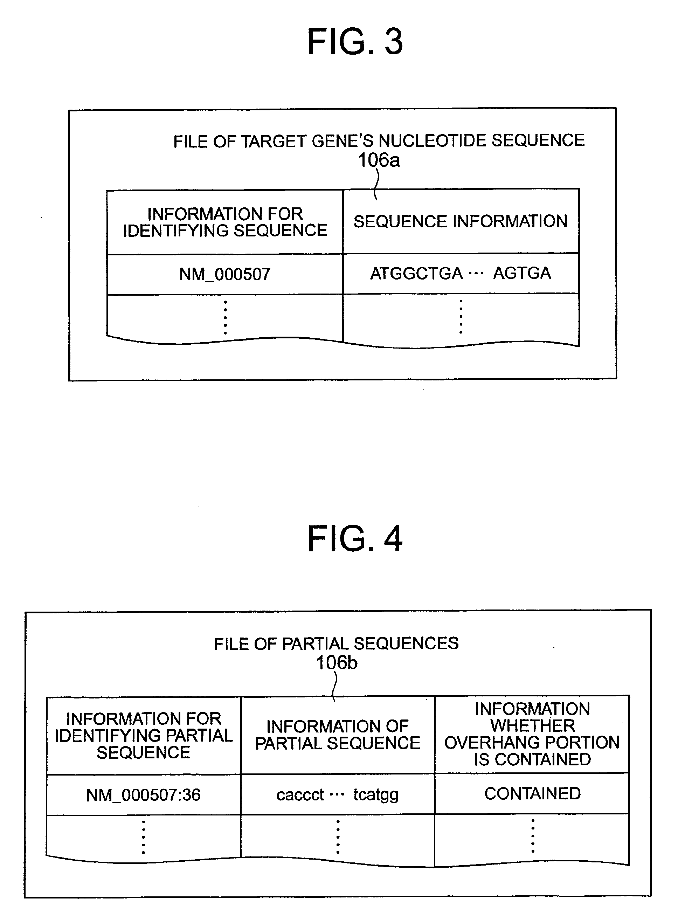

Among these components of the memory portion 106, the file 106a of the target gene's nucleotide sequence is a means for storing information of a nucleotide sequence(s) of a target gene for RNA interference. Figure 3 is a diagram which shows an example of information stored in the file 106a of a target gene's nucleotide sequence.

As shown in Figure 3, the information that is stored in the file 106a of the target gene's nucleotide sequence consists of information for identifying a sequence which uniquely identifies information of the nucleotide sequence of the target gene for RNA interference (e.g., "NM_000507" in Fig. 3) and sequence information, i.e., information of a target gene's nucleotide sequence (e.g., "ATGGCTGA ... AGTGA" in Fig. 3), and those pieces of information are associated with each other.

The file 106b of partial sequences is a means for storing information about partial sequences each having a predetermined number of nucleotides, which is derived from the information of the nucleotide sequence of the target gene for RNA interference. Figure 4 is a diagram which shows an example of information stored in the file 106b of partial sequences.

As shown in Figure 4, the information stored in the file 106b of partial sequences consists of information for identifying a partial sequence (e.g., "NM_000507:36" in Fig. 4), information of a partial sequence (e.g., "caccct ... tcatgg" in Fig. 4), and information about whether an overhang portion is present (e.g., "contained" in Fig. 4), and those pieces of information are associated with each other.

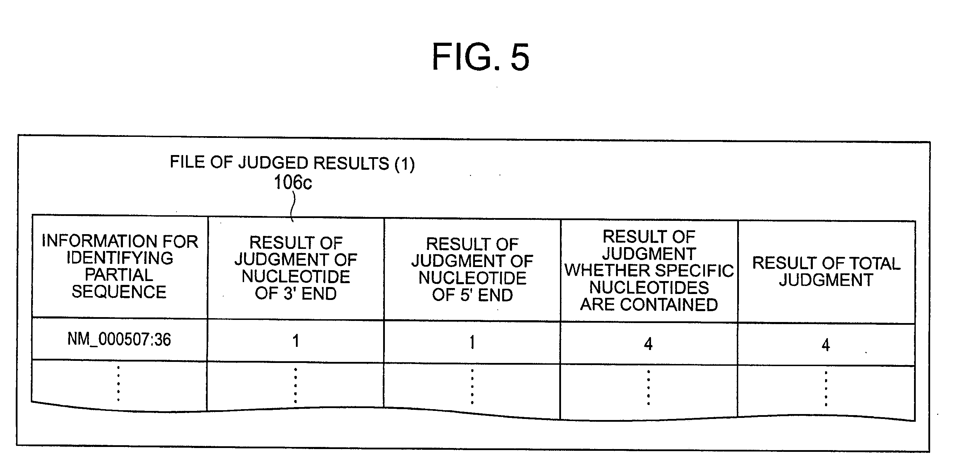

The file 106c of judged results (1) is a means for storing the results determined by the portion 102b for judging a nucleotide of the 3' end, the portion 102c for judging a nucleotide of the 5' end, and the portion 102d for judging whether specific nucleotides are present, which will be described below. Figure 5 is a diagram which shows an example of information stored in file 106c of judged results (1).

As shown in Figure 5, the information stored in file 106c of judged results (1) consists of information for identifying a partial sequence (e.g., "NM_000507:36" in Fig. 5), a result of judgement of the nucleotide of the 3' end corresponding to the result determined by the portion 102b for judging the nucleotide of the 3' end (e.g., "1" in Fig. 5), a result of judgement of the nucleotide of the 5' end corresponding to the result determined by the portion 102c for judging the nucleotide of the 5' end (e.g., "1" in Fig. 5), a result of judgement of whether specific nucleotides are present corresponding to the result determined by the portion 102d for judging whether specific nucleotides are present (e.g., "4" in Fig. 5), and a result of total judgement corresponding to the result obtained by putting together the results in the portions 102b, 102c, and 102d (e.g., "4" in Fig. 5), and those pieces of information are associated with each other.

Additionally, Figure 5 shows an example of the case in which, with respect to the result of judgement of the nucleotide of the 3' end and the result of judgement of the nucleotide of the 5' end, "1" is set when the result was "contained" (i.e. present) (for each of the portion 102b for judging the nucleotide of 3' end and the portion 102c for judging the nucleotide of the 5' end) and "0" is set when the result was "not contained." Further, Figure 5 shows an example of the case in which, as the result of judgement to establish whether specific nucleotides are present, the number of bases corresponding to at least one base selected from the group consisting of adenine, thymine, and uracil is set. Furthermore, Figure 5 shows an example of the case in which, as a result of the total judgement, the product of the above three results is set. Specifically, for example, when the product is 3 or less, "0" may be set.

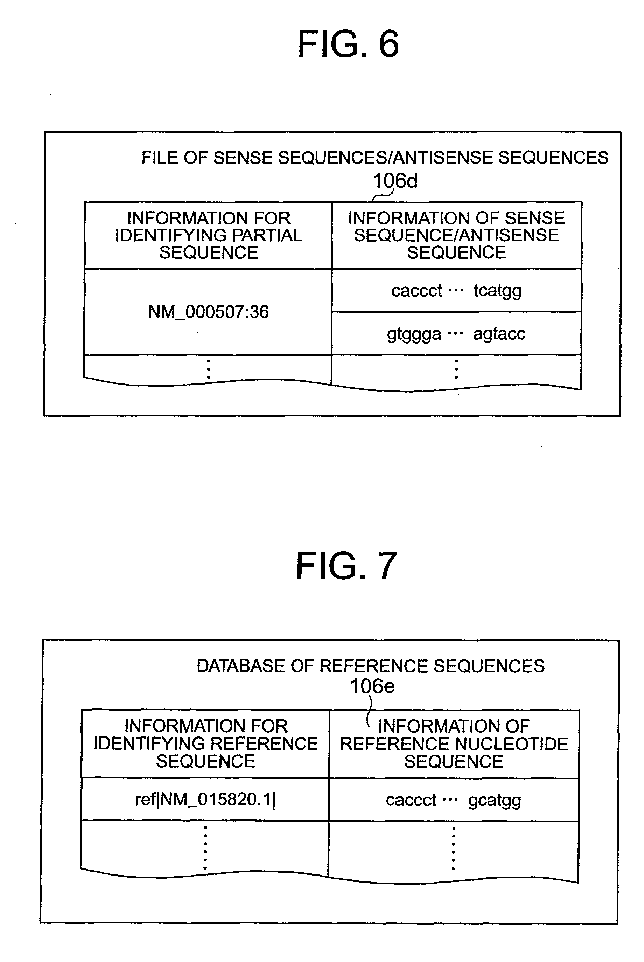

File 106d of sense sequences/antisense sequences is a means for storing partial nucleotide sequences which cause RNA interference specific to the target gene as sets of sense sequences and antisense sequences. Figure 6 is a diagram which shows an example of information stored in file 106d of sense sequences/antisense sequences.

As shown in Figure 6, the information stored in file 106d of sense sequences/antisense sequences consists of information for identifying a partial sequence (e.g., "NM_000507:36" in Fig. 6) and information of sense sequence/antisense sequence corresponding to information of partial nucleotide sequences which causes RNA interference specific to the target gene (e.g., "caccct ... tcatgg" in Fig. 6), and those pieces of information are associated with each other.

Furthermore, a database 106e of reference sequences is a database which stores information of a reference nucleotide sequence(s) which is used for retrieving a nucleotide sequence that is identical or similar to the sense/antisense sequence in the portion 102g for retrieving identical or similar nucleotide sequence, which will be described below. The database 106e of reference sequences may be a database of an external nucleotide sequence information accessed via Internet or may be an in-house database created by copying such a database, storing the original sequence information, or further adding unique annotation information to such a database. Figure 7 is a diagram which shows an example of information stored in database 106e of reference sequences.

As shown in Figure 7, the information stored in the database 106e of reference sequences consists of information for identifying a reference sequence (e.g., "ref|NM_015820.1|" in Fig. 7) and information about a reference nucleotide sequence (e.g., "caccct ... gcatgg" in Fig. 7), and those pieces of information are associated with each other.

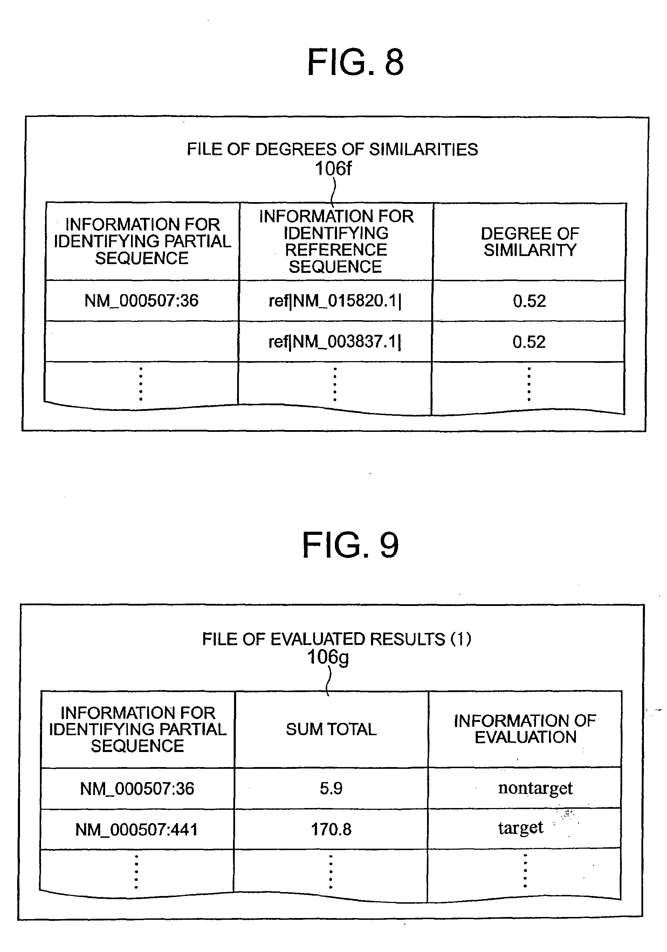

Furthermore, file 106f of degrees of similarities is a means for storing the degrees of similarities, which are values that are added to identical or similar nucleotide sequences that have been retrieved in portion 102g for retrieving an identical or similar nucleotide sequence, which will be described below. Figure 8 is a diagram which shows an example of information stored in file 106f of degrees of similarities.

As shown in Figure 8, the information stored in file 106f of degrees of similarities consists of information for identifying a partial sequence (e.g., "NM_000507:36" in Fig. 8), information for identifying a reference sequence (e.g., "ref|NM_015820.1|" and "ref|NM_003837.1|" in Fig. 8), and a degree of similarity (e.g., "0.52" in Fig. 8), and those pieces of information are associated with each other.

Furthermore, a file 106g of evaluated results is a means for storing the results of evaluations on whether a sequence targets an unrelated gene in the portion 102h for evaluating whether a sequence targets an unrelated gene, which will be described below. Figure 9 is a diagram which shows an example of information stored in the file 106g of evaluated results.

As shown in Fig. 9, the information stored in file 106g of evaluated results consists of information for identifying a partial sequence (e.g., "NM_000507:36" and "NM_000507:441" in Fig. 9), the sum total calculated in portion 102h1 for calculating the sum total of reciprocals of values of degrees of similarities, which will be described below, (e.g., "5.9" and "170.8" in Fig. 9), and information of evaluation (e.g., "nontarget" and "target" in Fig. 9), and those pieces of information are associated with each other. Additionally, in Figure 9, "non-target" means that the sense sequence/antisense sequence targets no gene unrelated to the target gene, and "target" means that the sense sequence/antisense sequence targets any gene(s) unrelated to the target gene.

The information that is stored in file 106h of trimming sequences is nucleotide sequences that satisfy the definition of the trimming sequence according to this invention. In portion 102i for designing a trimming sequence and designing an RNA sequence, a trimming sequence that is suitable to combine the selected sense sequence/antisense sequences is selected from the trimming sequences stored in this file in consideration of various conditions such as the sequence of an overhang portion. Then, the sense sequence, the designed trimming sequence, and the antisense sequence are arranged in this order.

The information that is stored in file 106i of RNA sequences is RNA sequences that are designed in the portion 102i for designing a trimming sequence and designing an RNA sequence. For each of the RNA sequences stored here, it is judged whether at least four consecutive uracils are present in the portion 102j for judging whether specific nucleotides are not present. RNA sequences that have been judged not to contain at least four consecutive uracils are stored in file 106j of judged results (2). In file 106j of judged results (2), the RNA sequences that have been judged not to contain at least four consecutive uracils are stored in relation to the information for identifying a partial sequence.

Database 106k of the target gene's annotations is a means for storing annotation information regarding the target gene. Database 106k of the target gene's annotations may be an external annotation database which stores annotation information regarding genes and which is accessed via Internet or may be an in-house database created by copying such a database, storing the original sequence information, or further adding unique annotation information to such a database.

The information stored in the database 106k of the target gene's annotations consists of information for identifying a target gene (e.g., the name of a gene to be targeted, and Accession number (such as "NM_000507" and "FBP1" described at the top in Fig. 3)) and simplified information on the target gene (e.g., " Homo sapiens fructose-1,6-bisphosphatase 1"), and those pieces of information are associated with each other.

In Figure 2, the interface portion 104 for communication control, controls communication between the device for processing information 100 and the network 300 (or a communication device, such as a router). Namely, the interface portion 104 for communication control practices data communication via other terminals and communication lines.

In Figure 2, the interface portion 108 for input-output control controls the input unit 112 and the output unit 114. Here, as the output unit 114, in addition to a monitor (including a home television), a speaker may be used (hereinafter, the output unit 114 may also be described as the monitor). As the input unit 112, a keyboard, a mouse, a microphone, or the like may be used. The monitor cooperates with a mouse to implement a pointing device function.

In Figure 2, the control portion 102 includes control programs, such as OS (Operating System), programs regulating various processing procedures, etc., and internal memories for storing required data, and practices information processing for implementing various processes using the programs, etc.

The control portion 102 functionally includes a portion 102a for creating partial sequences, a portion 102b for judging a nucleotide of the 3' end, a portion 102c for judging a nucleotide of the 5' end, a portion 102d for judging whether specific nucleotides are present, a portion 102e for designing a sense sequence/antisense sequence, a portion 102f for adding an overhang portion, a portion 102g for retrieving an identical or similar nucleotide sequence, a portion 102h for evaluating whether a sequence targets an unrelated gene, a portion 102i for designing a trimming sequence and designing an RNA sequence, and a portion 102j for judging whether specific nucleotides are not present.

Among them, portion 102a for creating partial sequences is a means for acquiring information of a nucleotide sequence of a target gene for RNA interference and creating information of partial sequences corresponding to a mass of partial nucleotide sequences each having a predetermined number of nucleotides.

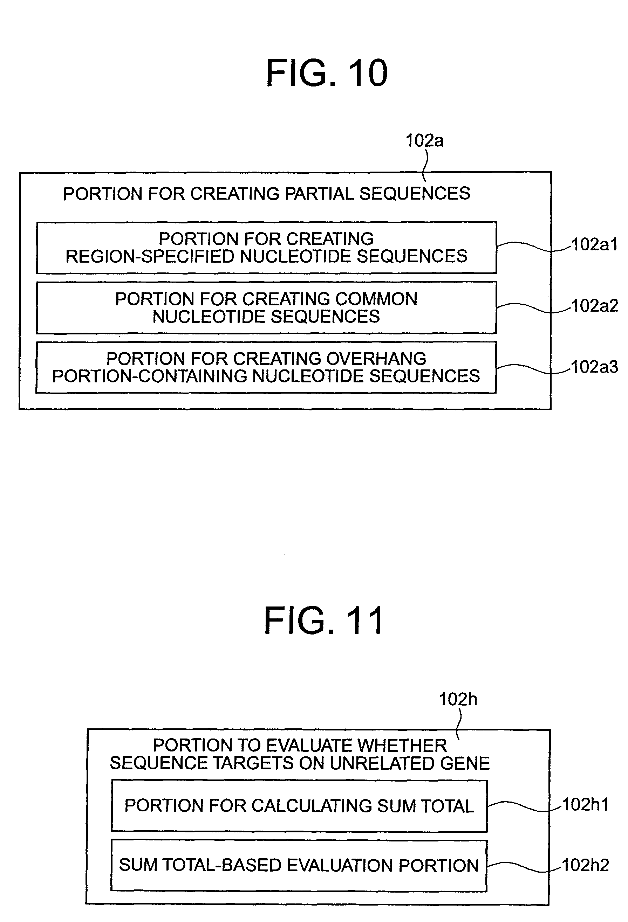

Figure 10 is a block diagram which shows an example of the structure of portion 102a for creating partial sequences of the system to which this invention is applied. Figure 10 shows only the portions related to this invention. As shown in Figure 10, portion 102a for creating partial sequences includes a portion 102a1 for creating region-specified nucleotide sequences, a portion 102a2 for creating common nucleotide sequences, and a portion 102a3 for creating overhang portion-containing nucleotide sequences.

In Figure 10, portion 102a1 for creating region-specified nucleotide sequences, information of partial sequences each consisting of a predetermined number of nucleotides is created from a segment corresponding to a coding region or transcription region of a target gene (contained in the information relating to nucleotide sequence(s)).

In the portion 102a2 for creating common nucleotide sequences, information of partial sequences each consisting of a predetermined number of nucleotides which is common in a plurality of nucleotide sequences derived from different organisms is created.

In the portion 102a3 for creating overhang portion- containing nucleotide sequences, information of partial nucleotide sequences each containing an overhang portion(s) is created.

Referring back to Figure 2, the portion 102b for judging a nucleotide of 3' end is a means for judging a nucleotide of the 3' end and whether the base contained in the nucleotide at the 3' end of each partial sequence in the information of partial sequences is adenine, thymine, or uracil.

The portion 102c for judging a nucleotide of the 5' end is a means for judging a nucleotide of the 5' end and whether the base contained in the nucleotide at the 5' end of each partial sequence in the information of partial sequences is guanine.

The portion 102d for judging whether specific nucleotides are present is a means for judging whether, the 7-bp-region of each partial sequence in the information of partial sequences, is rich in at least one base selected from the group consisting of adenine, thymine, and uracil.

The portion 102e for designing a sense sequence/antisense sequence is a means for selecting a sequence(s) which can cause RNA interference specific to the target gene from sequences contained in the information of partial sequences based on the results given in portion 102b for judging a nucleotide of the 3' end, portion 102c for judging a nucleotide of the 5' end, and portion 102d for judging whether specific nucleotides are present, and for identifying a sense sequence and a corresponding antisense sequence in the selected partial sequences as a set.

Portion 102f for adding an overhang portion(s) is a means for adding an overhang portion(s) to at least one end of the 5' end and the 3' end of the sense sequence and the 5' end and the 3' end of the antisense sequence in the information of sense sequences/antisense sequences.

Further, portion 102g for retrieving an identical or similar nucleotide sequence is a means for retrieving a nucleotide sequence which is identical or similar to the sequence in the information of sense sequences/antisense sequences from other nucleotide sequence information.

Furthermore, the portion 102h for evaluating whether a sequence targets an unrelated gene(s) is a means for evaluating whether a sequence in the information of sense sequences/antisense sequences targets a gene(s) unrelated to the target gene based on the information of identical or similar nucleotide sequences. As shown in Figure 11, the portion 102h for evaluating whether a sequence targets an unrelated gene(s) further includes a portion 102h1 for calculating the sum total and a sum total-based evaluation portion 102h2.

Figure 11 is a block diagram which shows an example of a structure of portion 102h for evaluating whether a sequence targets an unrelated gene(s) of the system to which this invention is applied. Figure 11 schematically shows only the portions related to this invention.

In Figure 11, portion 102h1 for calculating the sum total is a means for calculating the sum total of reciprocals of the values showing the degrees of similarity, based on the total amount of nucleotide sequences of the genes unrelated to the target gene in the information of identical or similar nucleotide sequences and based on the values showing the degrees of similarities attached to the nucleotide sequences of the genes unrelated to the target gene.

The sum total-based evaluation portion 102h2 is a means for evaluating whether a sequence in the information of sense sequences/antisense sequences targets any gene(s) unrelated to the target gene based on the sum total calculated in the portion 102h1 for calculating sum total.

The details of processing of each portion will be described later.

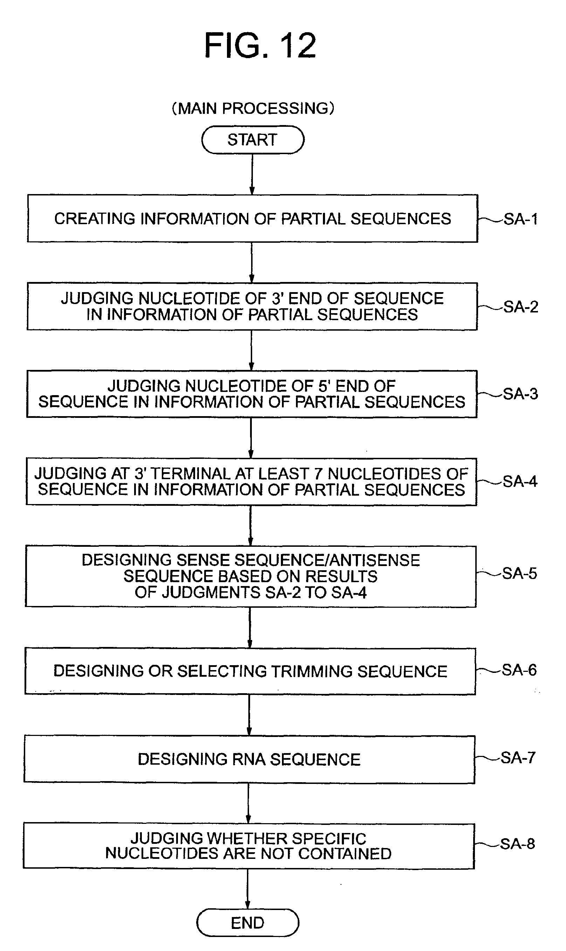

First, an example of a main processing of the system having the configuration described above will be explained with reference to Figure 12 and others. Figure 12 is a flowchart which shows an example of a main processing of the above system.

In the process of creating partial nucleotide sequences practiced in portion 102a for creating partial sequences, device 100 for processing information acquires information of a sequence of a target gene for RNA interference, stores the information in a predetermined memory region of file 106a of the target gene's nucleotide sequence, creates information of the partial nucleotide sequences which is a mass of partial nucleotide sequences each having a predetermined number of nucleotides from the information of the target gene's nucleotide sequence, and stores the created information in a predetermined memory region of file 106b of the partial sequences (step SA-1).

In step SA-1, portion 102a for creating partial sequences may create information of partial nucleotide sequences which is a mass of partial nucleotide sequences each having a predetermined number of nucleotides from a segment corresponding to a coding region or transcription region of the target gene in the information of the target gene's nucleotide sequence and may store the created information in a predetermined memory region of file 106b of the partial sequences by the processing of portion 102a1 for creating region-specified nucleotide sequences.

In step SA-1, the portion 102a for creating partial sequences may create information of partial nucleotide sequences which are common in a plurality of information of nucleotide sequences derived from different organisms (e.g., human nucleotide sequence information and mouse nucleotide sequence information), of which information consists of a mass of partial nucleotide sequences each having a predetermined number of nucleotides and may store the created information in a predetermined memory region of file 106b of partial sequences by the processing of portion 102a2 for creating common nucleotide sequences. Furthermore, information of partial nucleotide sequences which are common in a plurality of information of analogous nucleotide sequences in the same species may be created, which information consists of a mass of partial nucleotide sequences each having a predetermined number of nucleotides .

In step SA-1, portion 102a for creating partial sequences may create information of partial nucleotide sequences which is a mass of partial nucleotide sequences each having a predetermined number of nucleotides from segments corresponding to coding regions or transcription regions of the target gene which are common in a plurality of information of nucleotide sequences derived from different species, and may store the created information in a predetermined memory region of file 106b of partial sequences by the processing of portion 102a1 for creating region-specified nucleotide sequences and portion 102a2 for creating common nucleotide sequences. Further, portion 102a for creating partial sequences may create information of partial nucleotide sequences which is a mass of partial nucleotide sequences each having a predetermined number of nucleotides from the segment corresponding to a coding region or transcription region of the target gene which is common in a plurality of information of analogous nucleotide sequence in the same species.

Furthermore, in step SA-1, the portion 102a for creating partial sequences may create information of partial nucleotide sequences each containing an overhang portion(s) by the processing of portion 102a3 for creating overhang portion-containing nucleotide sequences. Specifically, for example, portion 102a may create information of partial nucleotide sequences, to which overhang portion-containing information which shows that the sequences contain the overhang portions, is added, and may store the created information of partial nucleotide sequences and the overhang portion-containing information so as to be associated with each other in a predetermined memory region of file 106b of partial sequences by the processing of portion 102a3 for creating overhang portion-containing nucleotide sequences.

Subsequently, device 100 judges whether the base of the nucleotide at the 3' end in a partial sequence in the information of partial sequences created in the step SA-1 is adenine, thymine, or uracil and stores the judged results in a predetermined memory region of the file 106c of judged results (1) by the processing of the portion 102b for judging nucleotide of the 3' end (step SA-2).

Specifically, for example, the device 100 may store " 1" when the base of the 3' end of a partial sequence in the information created in the step SA-1 is adenine, thymine, or uracil, and "0" when it is not, in a predetermined memory region of file 106c of judged results (1) by the processing of portion 102b for judging a nucleotide of the 3' end.

Subsequently, device 100 judges whether the base of the nucleotide at the 5' end in a partial sequence in the information of partial sequences created in step SA-1 is guanine and stores the judged results in a predetermined memory region of file 106c of judged results (1) by the processing of portion 102c for judging a nucleotide of the 5' end (step SA-3).

Specifically, for example, device 100 may store "1" when the base of the 5' end of a partial sequence in the information created in step SA-1 is guanine, and "0" when it is not, in a predetermined memory region of file 106c of judged results (1) by the processing of portion 102c for judging a nucleotide of the 5' end.

Subsequently, by the processing of portion 102d for judging whether specific nucleotides are present, device 100 judges whether, the 7-bp-region of the 3' terminal of a partial sequence in the information of partial sequences created in the step SA-1, is rich in at least one base selected from the group consisting of adenine, thymine, and uracil, and stores the judged results in a predetermined memory region of the file 106c of judged results (1) (step SA-4).

Specifically, for example, device 100, by the processing of portion 102d for judging whether specific nucleotides are present, may store the number of bases corresponding to at least one base selected from the group consisting of adenine, thymine, and uracil contained in the 7-bp-region of the 3' terminal of a partial sequence in the information of partial sequences created in step SA-1 in a predetermined memory region of the file 106c of judged results (1).

The term "rich" has been already explained above.

In steps SA-2 to SA-4, when a partial nucleotide sequence containing an overhang portion(s) is judged, a sequence segment excluding the overhang portion(s) is considered in the judgment.

Then, based on the results given in steps SA-2, SA-3, and SA-4, by the processing in portion 102e for designing a sense sequence/antisense sequence, the device 100 selects sequences which may cause RNA interference specific to the target gene from the information of partial nucleotide sequences created in step SA-1 and stores them as a set of the sense sequence and the antisense sequence in a predetermined memory region of the file 106d (step SA-5).

Specifically, for example, by the processing of portion 102e for designing a sense sequence/antisense sequence, the device 100 selects partial sequences which have been judged in step SA-2 to have as the base of the nucleotide at 3' end adenine, thymine, or uracil, which have been judged in the step SA-3 to have as the base of the nucleotide at the 5' end guanine, and which have been judged in the step SA-4 to be rich in at least one base selected from the group consisting of adenine, thymine, and uracil, and stores those sequences in a predetermined memory region of the file 106d as a set of the sense sequence and the antisense sequence.

For example, a product of the values outputted in steps SA-2, SA-3, and SA-4 may be calculated, and based on the product, sets of the sense sequence and the antisense sequence may be selected from the information of partial sequences created in step SA-1 by the processing of portion 102e for designing a sense sequence/antisense sequence.

The device 100 may add an overhang portion(s) to at least one end of the sense sequence/antisense sequence selected in step SA-5 and store the obtained sense sequence/antisense sequence containing the overhang portion(s) in a predetermined memory region of file 106d by the processing of portion 102f for adding an overhang portion.

Specifically, for example, by the processing of portion 102f for adding an overhang portion, the information of sense sequences/antisense sequences which has been recorded in file 106d may be changed to new information of sense sequences/antisense sequences in which each of the sense sequences/antisense sequences has an overhanging portion(s).

Furthermore, by the processing in portion 102g for retrieving an identical or similar nucleotide sequence, device 100 may retrieve any nucleotide sequence that is identical or similar to the sense sequences/antisense sequences selected in step SA-5 from other information of nucleotide sequences (e.g., information of nucleotide sequences published in a public database, such as RefSeq of NCBI) using a known homology search method, such as BLAST, PASTA, or ssearch, and may evaluate whether the selected sense sequences/antisense sequences targets any gene(s) unrelated to the target gene by processing to evaluate whether a sequence targets an unrelated gene which is practiced in portion 102h for evaluating whether a sequence targets an unrelated gene.

Specifically, for example, by the processing of portion 102g for retrieving an identical or similar nucleotide sequence, device 100 may retrieve any nucleotide sequence that is identical or similar to the sense sequences/antisense sequences selected in step SA-5 from other information of nucleotide sequences (e.g., information of nucleotide sequences published in a public database, such as RefSeq of NCBI) using a known homology search method, such as BLAST, FASTA, or ssearch. Portion 102h for evaluating whether a sequence targets an unrelated gene, by processing in portion 102h1 for calculating the sum total of reciprocals of values of degrees of similarity, may calculate the sum total of the reciprocals of the values showing the degree of similarity based on the total amount of nucleotide sequences that are unrelated to the target gene in the retrieved identical or similar nucleotide sequences and the values showing the degree of similarity which are given to nucleotide sequences that are unrelated to the target gene (e.g., "E value" in BLAST, FASTA, or ssearch), and by processing in the sum total-based evaluation portion 102h2, may evaluate whether the selected sense sequences/antisense sequences target any gene(s) unrelated to the target gene based on the calculated sum total.

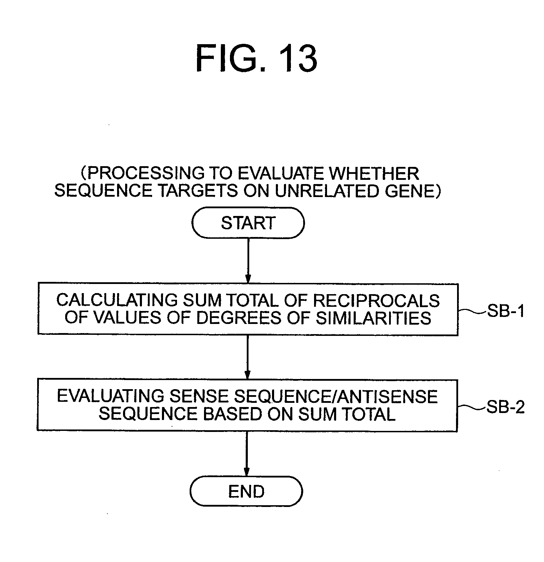

Here, the details of the process for evaluating whether a sequence targets an unrelated gene which is practiced in portion 102h for evaluating whether a sequence targets an unrelated gene will be described with reference to Figure 13.

Figure 13 is a flowchart which shows an example of the process for evaluating whether a sequence targets an unrelated gene in the above system.

First, by the processing of portion 102g for retrieving an identical or similar nucleotide sequence, device 100 retrieves any nucleotide sequence that is identical or similar to the sense sequences/antisense sequences selected in step SA-5 from other information of nucleotide sequences using a known homology search method, and stores information for identifying the sense sequence/antisense sequence (the information for identifying a partial sequence in Fig. 8), information for identifying the retrieved identical or similar sequence (the information for identifying a reference sequence in Fig. 8), and values showing degrees of similarity attached to the retrieved identical or similar nucleotide sequences (e.g., "E value" in BLAST, PASTA, or ssearch) ("degree of similarity" in Fig. 8) so as to be associated with one another in a predetermined memory region of file 106f of degrees of similarity.

Subsequently, portion 102h for evaluating whether a sequence targets an unrelated gene, by the processing of portion 102h1 for calculating the sum total of reciprocals of values of degrees of similarity, calculates the sum total of the reciprocals of the values showing the degrees of similarity based on the total amount of the nucleotide sequences that are unrelated to the target gene in the retrieved identical or similar nucleotide sequences and based on the values showing the degrees of similarities which are given to the nucleotide sequences that are unrelated to the target gene, and stores information for identifying a sense sequence/antisense sequence ("information for identifying a partial sequence" in Fig. 9) and the calculated sum total ("sum total" in Fig. 9) so as to be associated with each other in a predetermined memory region of file 106g of evaluated results (step SB-1).

Subsequently, by the processing of the sum total-based evaluation portion 102h2, portion 102h for evaluating whether a sequence targets an unrelated gene evaluates whether the selected sense sequences/antisense sequences target any gene(s) unrelated to the target gene based on the sum total calculated in step SB-1 (e.g., based on the size of the sum total calculated in step SB-1), and stores the evaluated results ("nontarget" and "target" in Fig. 9) in a predetermined memory region of the file 106g of evaluated results (step SB-2).

The oligo- or polynucleotide of this invention has a trimming sequence, in addition to a sense sequence and an antisense sequence. Thus, in portion 102i for designing a trimming sequence and designing an RNA sequence, a trimming sequence is selected from file 106h of trimming sequences or a trimming sequence that satisfies the conditions for that sequence (namely, the formula: (G or C) -X-Y-Z-(C or G)) is designed (step SA-6). Then, for each set of the sense sequences/antisense sequences which have been selected in step SA-5, an RNA sequence is designed by arranging a sense sequence, a trimming sequence, and an antisense sequence in this order (designing of an RNA sequence, step SA-7). For one set of a sense sequence and an antisense sequence, each of two or more trimming sequences may be combined and thus two or more RNA sequences may be designed. The RNA sequences thus designed are stored in a predetermined memory region of file 106i of RNA sequences.

When there is an overhang portion outside the 3' end of the sense sequence or outside the 5' end of the antisense sequence, the overhang portion forms a part of the trimming sequence.

Lastly, for each of the sequences that have been stored in file 106i of RNA sequences, a judgment is made on whether the sequence contains four or more consecutive uracils and the results are stored in a predetermined memory region of the file 106j of judged results (2) (step SA-8). In file 106j of judged results (2), only RNA sequences not containing four or more consecutive uracils may be stored. Alternatively, all of the RNA sequences which have been evaluated may be stored with the judged results (e.g., "has" and "does not have" four or more consecutive uracils).

The main process is thereby completed.

An embodiment of the device for processing information of this invention has been described above. However, it is to be understood that the invention can be carried out in various embodiments other than the embodiment described above within the scope of the technical idea described in the claims.

For example, although the case in which the device 100 practices processing in a stand-alone mode has been explained, construction may be such that processing is practiced in accordance with the request from a client terminal which is constructed separately from the device 100, and the processing results are sent back to the client terminal. Specifically, for example, the client terminal transmits a name of the target gene for RNA interference (e.g., gene name or accession number) or nucleotide sequence information regarding the target gene to device 100, and device 100 practices the processes as explained above in control portion 102 for the information of the nucleotide sequence corresponding to the name or the information of the nucleotide sequence transmitted from the client terminal to select RNA sequences which may cause RNA interference specific to the target gene, and transmits them to the client terminal. In this case, for example, by acquiring sequence information from a public database, the RNA sequences may be selected.

Further, device 100 may check the specificity of a sense sequence/antisense sequence with respect to genes unrelated to the target gene. Thereby, it is possible to select RNA sequences which can cause RNA interference specific to only the target gene.

Furthermore, in the system comprising a client terminal and the device 100, an interface function may be introduced in which, for example, the results of the RNA interference effect of an shRNA are fed back from the Web page users on the Web, and the experimental results fed back from the users are accumulated in device 100 so that the sequence requirements of the shRNA effective for RNA interference are improved.

Furthermore, device 100 may be realized by installing software by which the computer program of this invention is practiced on an application program server such as a personal computer and a workstation, and connecting to the sever some devices such as a printer, a monitor, and an image scanner.

Network 300 has the function of interconnecting between device 100 and external system 200, and for example, may include any one of Internet, intranets, LANs (including both wired and radio), VANs, personal computer communication networks, public telephone networks (including both analog and digital), dedicated line networks (including both analog and digital), CATV networks, portable line exchange networks/portable packet exchange networks of the IMT2000 system, GSM system, or PDC/PDC-P system, radio paging networks, local radio networks, such as the Bluetooth, PHS networks, and satellite communication networks, such as CS, BS, and ISDB.

The second device for processing information for designing an shRNA sequence of this invention, namely the device comprising the following portions (1) to (8), is now specifically explained:
(1) a portion for acquiring information of a nucleotide sequence of a target gene for RNA interference and creating information of partial sequences each having a predetermined number of consecutive nucleotides from the acquired information;
(2) a portion for judging a nucleotide of the 5' end wherein a sequence(s) containing a nucleotide of which the base is guanine or cytosine at the 5' end is selected from partial sequences in the created information of partial sequences;
(3) a portion for judging a nucleotide of the 3' end wherein a sequence(s) containing a nucleotide of which the base is cytosine, thymine, or uracil at the 3' end is selected from the partial sequences in the created information of partial sequences;
(4) a portion for judging whether specific nucleotides are present wherein a sequence(s) which is rich in at least one base selected from the group consisting of adenine, thymine, and uracil in 7 nucleotides at the 3' terminal is selected from the partial sequences in the created information of partial sequences;
(5) a portion for designing a sense sequence/antisense sequence wherein for each of the partial sequence(s) that were selected in all of the above portions (2), (3), and (4), a sense sequence that is identical to the selected sequence and an antisense sequence that is entirely complementary to the sense sequence are decided;
(6) a portion for designing a trimming sequence wherein a trimming sequence comprising 5 to 52 nucleotides is decided which is represented by the formula: (G or C)-X-Y-Z-(C or G), wherein the nucleotides of both ends are entirely complementary to each other, the X region and the Z region each comprise 0 to 10 nucleotides, of which the bases are selected from the group consisting of adenine, thymine, uracil, guanine, and cytosine, and are complementary to each other when the trimming sequence is turned in the Y region, the Y region comprises 3 to 50 nucleotides, and in the Y region at least one nucleotide which follows the X region and at least one nucleotide to which the Z region follows are not complementary to each other;
(7) a portion for designing an RNA sequence wherein the sequences that were decided in the above portions (5) and (6) are consecutively arranged in the order of the sense sequence, the trimming sequence, and the antisense sequence; and
(8) a portion for selecting at least one sequence which does not contain four or more consecutive nucleotides of which the bases are thymine and/or uracil from the RNA sequences designed in the above portion (7).

For the second device, the above explanations for the first device can also be applied, except for the kinds of the bases that are used in the judgements and except that the antisense sequence, the trimming sequence, and the sense sequence are arranged in this order.

By using the oligo- or polynucleotide of this invention, it becomes possible to produce shRNAs easily and efficiently. By eliminating a trimming sequence portion from an obtained shRNA, a double-stranded RNA can be obtained, without preparing a sense strand and an antisense strand and annealing them. The siRNA thus obtained exhibits highly effective RNA interference to a target gene, and there is little possibility that it targets any genes that are unrelated to the target gene.

Thus, the oligo- or polynucleotides of this invention can cause RNA interference which is specific to the target genes whose expressions it is desired to reduce or silence. Thus, they can be preferably used in experiments and treatments in which RNA interference is used. This invention is useful when the RNA interference is to be carried out in higher animals such as mammalians, especially homo sapiens.

The double-stranded DNAs of this invention can be effectively used to achieve RNA interference of the target genes.

The recombinant DNA of this invention can be effectively used to achieve RNA interference for gene therapy and for identifying functions of genes.

In the transfected cells of this invention, the target gene is knocked out. Thus, they are useful in the study of, e.g., the functions of the target gene.

By the method of causing RNA interference in a mammalian cell of this invention, target genes can be knocked out with high efficiency.

By using the device of this invention, sequences of shRNAs that can cause RNA interference specific to target genes can be designed.

By using the program or the computer-readable recording medium of this invention, sequences of shRNAs that can cause RNA interference specific to target genes can be designed.

The invention will now be specifically exemplified with reference to experimental examples.

### Example 1

### Materials and Methods

### 1. Cell culture

Drosophila S2 cells were cultured in Schneider's Drosophila Medium (Gibco BRL) at 25°C. Chinese hamster CHO-K1 (RIKEN Cell Bank) and human HeLa cells were cultured in Dulbecco's Modified Eagle's Medium (DMEM; Gibco BRL) at 37°C. Both media were supplemented with 10% heat-inactivated fetal bovine serum (FBS; Mitsubishi Kagaku) and antibiotics (10 units/ml of penicillin (Meiji) and 50 µg/ml of streptomycin (Meiji)). E14TG2a (mouse ES) cells were cultured in DMEM supplemented with 20% heat-inactivated FBS (Hyclone), 0.1 mM 2-mercaptoethanol (Wako), 8 µg/ml of adenosine, 8.5 µg/ml of guanosine, 7.3 µg/ml of cytidine, 7.3 µg/ml of uridine, 2.4 µg/ml of thymidine, 0.1 mM each nonessential amino acid and 1,000 units/ml of leukemia inhibitor factor (CHEMICON international).

### 2. Preparation of siRNA

RNA oligonucleotides were synthesized by Proligo. Double-stranded siRNA was prepared as described in Ui-Tei K., et al., FEBS Lett. 479, p.p.79 -82 (2000). The concentration of siRNA is shown based on that of the antisense strand. When necessary, siRNAs were numbered based on the nucleotide position within the coding region of the target mRNA, corresponding to the 3' siRNA-antisense-strand end.

### 3. Luc RNAi assay

One milliliter of 52 (1 x 10⁶ cells/ml), CHO-K1 (3x10⁵ cells/ml), HeLa (1 x 10⁵ cells/ml) or E14TG2a (2 x 10⁵ cells/ml) cell suspension was inoculated into a 1.5-cm well 24 hours prior to transfection. Cells were transfected with pGL3-Control DNA (1 µg, Promega) encoding the firefly luciferase gene and pRL -TK DNA (0.1-1 µg, Promega) or pRL-SV40 DNA (0.1-1 µg, Promega), both encoding the Renilla luciferase gene, with or without siRNA. The calcium phosphate precipitation method was used for transfection for S2, HeLa or CHO-K1 cells, while DMRIE C reagent (Invitrogen) was used for E14TG2a transfection. Cells were harvested 24 hours after transfection and luciferase activity was measured using the Dual-Luciferase Reporter Assay System (Promega).

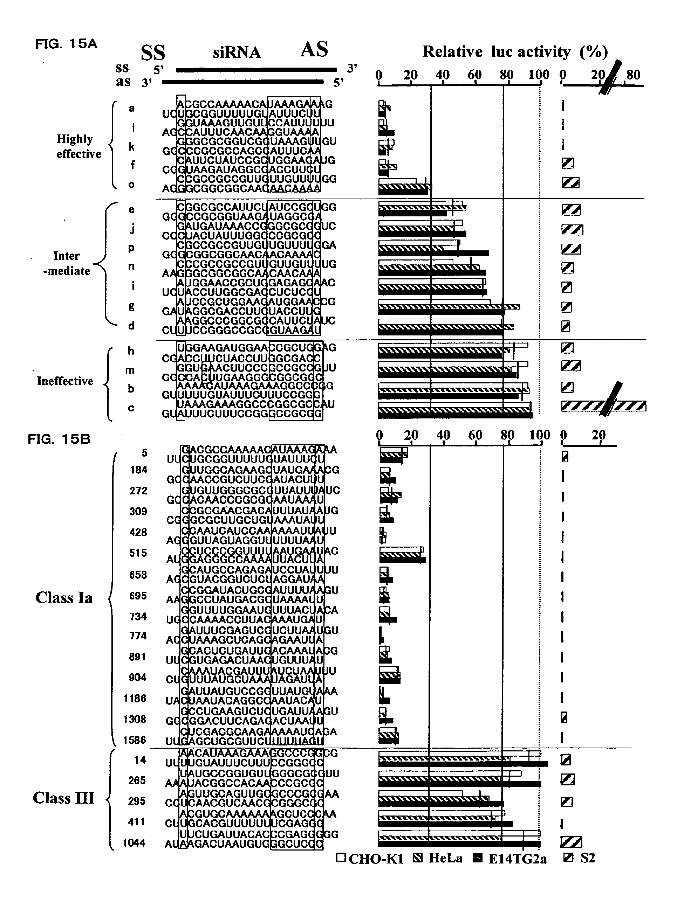

Figure15A shows the classification of 16 luc siRNAs.

SiRNA-dependent reduction in firefly luciferase activity in three mammalian (CHO-K1, HeLa and E14TG2a) and Drosophila (S2) cells was examined using 50 nM of 16 siRNAs, a-p, shown in Fig. 14. The siRNAs were classified into three groups. The details of the result will be shown below.

Fig. 15B shows RNAi activities caused by siRNAs designed using our sequence preference rules. Using the rules, 15 class Ia and 5 class III siRNAs were designed and their capabilities to bring about RNAi in CHO-K1, HeLa, E14TG2a and S2 cells were examined. The siRNA number indicates the nucleotide position within the luc coding region, corresponding to the 3' end of the siRNA antisense strand. The concentration of siRNA was 50 nM and RNAi effects were observed 24 hours after transfection.

### 4. Vimentin RNAi and immunostaining

One milliliter of HeLa cell suspension (1 x 10⁵ cells/ml) was inoculated into a 1.5-cm well 24 hours prior to the first transfection. Cells were treated with three cycles of transfection carried out in 24 hour intervals with vimentin siRNA at 50 nM. Lipofectamine 2000 (Invitrogen) was used for transfection. Estimated transfection efficiency was > 95%. Cells were fixed with 3.7% formaldehyde in phosphate buffered saline and permeabilized 24 hours after the last transfection. After washing with PBS, cells were doubly stained with anti-porcine Vimentin antibody (Oncogene Research Products), cy3-conjugated second antibody (Jackson Immuno Research), and anti-human Yes antibody (Upstate Biotechnology), with cy5-conjugated second antibody (Jackson Immuno Research).

### 5. Oct4 RNAi assay

Using Lipofectamine 2000 (Invitrogen), E14TG2a cells (2 x 10⁵ cells/ml) were cotransfected with 50 nM Oct 4 siRNA shown in Fig. 16B and pCAGIPuro-EGFP (0.5 µg/ml), encoding EGFP and puromycin resistant genes. Puromycin (2 µg/ml; Clontech) was added to the medium 24 hours after transfection, and morphological change was observed under a phase contrast microscope 3 days after transfection. RNA was also extracted 3 days after transfection using RNeasy (QIAGEN) and was applied to RT-PCR using the RNA LA-PCR kit (Takara). Almost all cells were found to express EGFP 3 days after transfection. The following primers were used for RT-PCR to measure the concentration of glyceraldehyde-3-phosphate dehydrogenase (Gapd) and Oct 4 mRNA. Gapd: 5'- GCCTCATCCGGTAGACAAAA (sequence number 1) and 5'-ACCGTGGTCATGAGTCCTTC (sequence number 2); Oct-4: 5'-AGCTGCTGAAGCAGAAGAGG (sequence number 3) and 5'-TGTCTACCTCCCTTGCCTTG (sequence number 4).

### 6. RNAi assay for EGFP, ECFP and DsRed

HeLa cells (1 x 10⁵ cells/ml) were transfected with pCAGGS-EGFP (0.25 µg/well), pCAGGS-DsRed (0.25 µg/well; ref.15) and siRNA (50 nM) for EGFP RNAi. For ECFP RNAi, HeLa cell transfection was carried out with pECFP -N1 (0.25 µg/well; Clontech), pCAGGS-DsRed (0.25 µg/well) and siRNA (50 nM). Transfection was carried out using Lipofectamine 2000 (Invitrogen). RNAi activity was estimated by counting EGFP- or ECFP-positive cells among DsRed-positive cells under a fluorescence microscope (Zeiss). pCAGGS-EGFP was constructed by inserting an EGFP fragment of pEGFP-N1 (Clontech) into the EcoRI site of pCAGGS.

### 7. In vitro electroporation

Fertile chick eggs obtained from a local farm were incubated at 37°C for 2 days. The eggs were windowed, and 0.1-0.5 µl of PBS containing pCAGGS-EGFP (0.1 µ 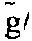 /µl) and pCAGGS-DsRed (0.1 µ 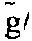 /µl) and siRNA (5 µ 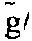 /µl) along with 0.01% of luxol fast blue was injected into the central canal of the spinal cord at the wing level using a glass capillary with a tip diameter of 50-100 µm. A pair of platinum electrodes 4 mm apart (Nepagene) was used for electroporation. Transfection occurred exclusively on the right hemilateral side of the neural tube. Five timed pulses of 50 milli-second duration at 20 mV were used. Embryos were incubated at 37°C for 2 days and killed. EGFP and DsRed expressions were observed under a fluorescence microscope 4 days after transfection.

### 8. Construction of siRNA expression plasmids for DNA-based RNAi

Single-stranded DNA oligonucleotides, about 80 nucleotides in length and encoding, in order: (1) a 21nt siRNA sense strand (among the 21 nucleotides, 2 nucleotides at the 3'-terminal are a part of the trimming sequence in this invention); (2) a human miRNA loop; and (3) the 19nt antisense strand of the identical siRNA, minus 3' overhangs, were annealed with corresponding complementary single-stranded DNA oligonucleotides. The resultant dsDNA was inserted into the BamHI/HindIII site ofpSilencer 3.0-H1 (Ambion) to generate FLx-m23L or FLx-m212L plasmids, where x indicates the position of corresponding target sequence in the firefly luc gene. In FL826-m212L, the order of sense and antisense strands were reversed. As human miRNA loops, m23L and m212L, derived from miR-23 and miR-212, respectively, were used. Escherichia coli XL1-Blue competent cells (Gibco BRL) were transformed with the resultant plasmids. Plasmid DNA was purified using a commercial DNA purification kit (QIAGEN). HeLa cells (1 x 10⁵ cells/ml) were transfected with 150 ng of the plasmid DNA along with pGL3-Control (1 µg) and pRL-SV40 (0.1 µg, Promega). pSilencer with no insert was used as a control. Luciferase activity was measured using the Dual-Luciferase Reporter Assay System (Promega) 3 days following transfection.

### 9. Free energy calculation

Standard Gibbs free energies, which reflect the stability of pentamer subsequences, were calculated from the siRNA duplex end containing 5' antisense strand end (position 1) according to the nearest neighbour method described by Freier, et al. The values from position 16-19 were not calculated because of the absence of available pentamer subsequences.

### Results

### 1. Strong siRNA-sequence preference in mammalian RNAi

RNAi in mammalian cells was previously noted to vary considerably depending on the siRNA sequence. To examine this point in greater detail, 16 siRNAs targeting the firefly luciferase gene (luc) were prepared (Fig. 14) and assessed for ability to produce RNAi in human (HeLa), chinese hamster (CHO-K1), mouse ES (E14TG2a) and Drosophila (S2) cells by dual luciferase assay.

Fig. 14 shows the 9 luc target sequences, corresponding to siRNA a-i, which are spaced 6 nucleotides apart, while 3 of the remaining target sequences (corresponding to siRNA n-p) are spaced only 1 nucleotide apart. Cells were simultaneously transfected with plasmid DNA encoding the firefly luc gene (target), plasmid DNA with Renilla luciferase gene (reference) and 50 nM cognate siRNA and luciferase activity was measured 24 hours thereafter (Fig. 15A). In Fig. 15A, siRNA sequences are listed in rank, in order of average RNAi activity in three mammalian cells, so as to obtain some clarification of the relationship between siRNA sequence and resultant reduction in firefly luc gene activity.

In mammalian cells, RNAi activity varied significantly depending on the siRNA employed. Use of five highly effective siRNAs (a, 1, k, f and o) resulted in a 70-95% reduction in relative firefly-luciferasc-activity while use of 4 highly ineffective siRNAs (h, m, b and c) resulted in less than 20% reduction. Even a 1nt variation in the target sequence had a considerable effect on RNAi activity in mammalian cells (compare RNAi effects of siRNA-n and -o).

In contrast, firefly luciferase activity was always abolished at more than 85% upon transfecting Drosophila cells with any siRNA other than siRNA-c. Thus, most, if not all, siRNAs should be capable of producing highly effective RNAi in Drosophila cells, at least under certain conditions. Three of the 4 siRNAs (a, 1 and k) giving rise to the highest levels of RNAi in mammalian cells were also noted to bring about the highest levels of RNAi in Drosophila cells.

### 2. siRNA sequence requirement for highly effective and ineffective RNAi in mammalian cells

The values in Fig. 15A for reduction in relative firefly-luciferase-activity in CHO-K1, HeLa and E14TG2a cells can be seen to be virtually the same, suggesting that siRNA-based RNAi in mammalian cells is in accordance with the same rules for siRNA sequence preference. As shown in the upper margin of the figure, siRNA ends with the 5' antisense-strand and 5' sense-strand ends which are designated in the following as AS and SS ends, respectively.

Three immediately apparent features of the siRNA sequence may possibly serve to discriminate highly effective siRNAs from those that are ineffective.

First, the 5' AS end (5' end of the antisense strand) of highly effective siRNAs may always be A or U, with the counterpart of ineffective siRNAs being G or C. A/U and G/C residues were respectively found present at the 5' AS ends of all five highly effective and all four ineffective siRNAs.

Secondly, the 5' SS ends of highly effective siRNAs are preferably G or C, with the counterpart of ineffective siRNAs being A or U.

Thirdly, in the case of highly effective siRNAs, at least 4 out of 7 nucleotides in the 5' terminal antisense strand are A or U while the corresponding region of ineffective siRNAs, GC-rich.

Most, if not all, siRNAs associated with mixed features appear to belong to an siRNA class with intermediate RNAi activity. A possible molecular basis for the effectiveness of siRNA-a is discussed below.

siRNAs may be grouped into three classes of I to III, based on combinations of terminal base sequences. Class I consists of siRNAs possessing A/U at the 5' AS end, G/C at the 5' SS end and at least 4 A/U nucleotides in a 7 nucleotide-long, 5' terminal end of the antisense strand, whereas those with opposite features are class III siRNAs. All other siRNAs are considered to belong to class II. Class I siRNAs may be sub-divided into two classes of Ia and Ib. Class I siRNAs with 5-7 A/U residues in a 7 nucleotide-long, 5' terminal end of the antisense strand are presumed to belong to class Ia siRNAs; the remaining belong to class Ib.

It is possible to generate 1631 different siRNAs based on the firefly luc coding sequence. The number of class I siRNAs was calculated as 275 (17 % of the total) and that of class Ia siRNAs, 154 (9 %). To test the validity of the above rules for siRNA-sequence preference, assessment was made of the ability of 15 different class Ia and 5 class III siRNAs to give rise to RNAi using three mammalian and Drosophila S2 cells (Fig. 15B).

All class Ia siRNAs brought about highly effective RNAi in all three mammalian cells as well as Drosophila cells while little or no effective RNAi resulted via transfection of class III siRNAs in the mammalian cells. We thus conclude that the rules stipulated here for siRNA sequence preference predict sequences for highly effective and ineffective siRNAs for mammalian RNAi at least in the case of the exogenous firefly luc gene.

### 3. Silencing of mammalian endogenous genes by siRNA transfection

Examination was made to determine whether the rules for siRNA-sequence preference would be applicable for designing highly effective and ineffective siRNAs for RNAi of mammalian endogenous genes.

The right margin of Figs. 16A and 16B shows class Ia and class III siRNAs, designed for highly effective and ineffective RNAi, respectively, of vimentin and Oct 4 in mammalian cells (HeLa and E14TG2a).

Candidate siRNAs designed by the present rules were further selected by Blast search so that the activity of any gene other than the target would not be affected by the siRNA introduced into cells. Class Ia siRNAs unique to vimentin and Oct 4, respectively, were found to represent 5 % (n=64) and 3 % (n=37) of all possible siRNAs based on vimentin and Oct 4 gene sequences.

The vimentin gene codes for an intermediate filament protein. It has been reported that reduction in vimentin gene activity by cognate siRNA transfection is difficult. Three cycles of siRNA transfection (one transfection/day) were thus carried out on HeLa cells prior to immunostaining for vimentin and Yes (control).

All 10 vimentin class Ia siRNAs were found to significantly reduce vimentin protein but not Yes signals (Fig. 16A). Little or no reduction in vimentin or Yes signals could be detected on using class III vimentin siRNAs for RNAi.

RT-PCR results (K.U.-T. and K.S., unpublished data) indicated that 70 to 95% of vimentin mRNA was degraded by class Ia vimentin siRNA, but that virtually no vimentin mRNA cleavage occurred by class III siRNA.

Oct 4 is a POU transcription factor encoded by Pou5f1 (Oct 4) gene and is considered to be a regulator of ES cell pluripotency. 50 to 100% increment in Oct 4 expression may cause the differentiation of pluripotent ES cells into primitive endoderm and mesoderm, while reduction in Oct 4 expression induces loss of pluripotency to differentiate ES cells into trophectoderm, which is characterized by flat morphology and induced expression of Hand 1 and Psx.

Three class Ia siRNAs (Oct-670, Oct-797 and Oct-821) and two class III siRNAs (Oct-161 and Oct-566) for Oct 4 RNAi were prepared and examined for change in cell morphology and gene expression three days following transfection of 50 nM cognate siRNA.

As partly shown in Fig. 16B, the pluripotent ES cells treated with cognate class Ia siRNAs, Oct-670, Oct-797 and Oct-821, flattened out over the culture surface, with enlarged nuclei acquired in many cases. Oct 4 expression was virtually eliminated (Fig. 16B) while the expression of trophectoderm markers, Hand 1 and Psx, were induced (K.U.-T., unpublished data). In contrast, no apparent change in morphology or gene expression could be found as a result of class III Oct 4 siRNAs, Oct-566 and Oct-161 (Fig. 16B). Our rules for siRNA sequence preference are thus shown to serve quite well for identifying highly effective and ineffective siRNAs for RNAi of endogenous genes in mammals.

Thirty-two class Ia siRNAs for firefly luc, vimentin and Oct 4 were examined and 31 (97%) of which were found to be capable of giving rise to highly efficient RNAi in human, chinese hamster and mouse cells. Virtually all of the investigated class Ia siRNAs were thus shown to be highly efficient RNAi reagents for mammalian cells. Thus, it is concluded that our rules for siRNA sequence preference may be highly useful for the design of effective siRNAs for RNAi of both exogenous and endogenous genes in mammalian cells.
Figure 16A: Silencing of vimentin, a human endogenous gene, by class Ia and class III siRNAs. Ten class Ia (VIM-270, VIM-368, VIM-596, VIM-812, VIM-857, VIM-1097, VIM-1128, VIM-1148, VIM-1235, and VIM-1298) and three class III (VIM-35, VIM-155, and VIM-491) siRNAs were designed and their RNAi activities were examined in HeLa cells subjected to three cycles of 50 nM siRNA transfection. On day 3, cells were stained for vimentin (target) and Yes (control).
Figure 16B: Effects of siRNA transfection on the expression of Oct 4, a mouse endogenous gene. E14TG2a (mouse ES) cells were transfected with class Ia (Oct-670, Oct-797, and Oct-821) or class III (Oct-161 and Oct-566). Gapd was used as a control.
Figure 16C: ECFP RNAi caused by an uncognate EGFP siRNA. EGFP-441 is a class Ia EGFP siRNA but not identical in sequence to ECFP-441, a class II ECFP siRNA possessing G at the 5' AS end.
Figure 16D: In ovo RNAi in chick embryo. EGFP and DsRed expression plasmids were co-electroporated into chick spinal cord with class Ia siRNAs (EGFP-416, EGFP-441, and DsRed-399) or class III siRNAs (EGCFP-666 and DsRed-383).

### 4. siRNAs with long stretches of G/C residues are incapable of bringing about high levels of RNAi in both mammalian and Drosophila cells

siRNA-n may be an exceptional member of class Ia siRNAs in that, unlike any others which we evaluated, it was incapable of giving rise to high levels of RNAi in mammalian cells when transfected at 50 nM (see Fig. 15A).

An investigation was thus made to clarify in greater detail relations among the siRNA sequence, siRNA concentration, and RNAi activity in CHO-K1 or S2 cells using the 16 siRNAs shown in Fig. 15A (Fig. 17A).

With siRNA at 0.005 to 5 nM, most graph points for siRNAs which gave rise to effective RNAi in CHO-K1 or S2 cells after transfection at 50nM overlapped or were situated near the shaded area bounded by two lines, intersecting, respectively, the horizontal axis at 0.5 and 5 and the 50% line of relative luciferase activity at 0.05 and 0.5.

The vertical bars in Fig. 17A show the relative luciferase or RNAi activity range for siRNAs which give rise to effective RNAi in CHO-K1 or S2 cells subsequent to transfection at 50 nM. siRNAs that bring about highly effective RNAi on transfection at 50 nM would thus appear comprised of heterogenous members with over 10 times the capacity to bring about RNAi.

A comparison of RNAi effects due to individual siRNA in CHO-K1 and S2 cells is presented in each of the 11 pictures in Fig. 17B. The pictures are arranged according to siRNA classification and order of RNAi activity.

Maximum levels of RNAi resulted from the transfection of siRNA-1, a class Ia siRNA, in both CHO-K1 and S2 cells. Note that suppression due to siRNA-1 in S2 cells was virtually the same as in CHO-K1 cells. We interpret this finding as suggesting that virtually all siRNA-1 molecules incorporated into cells become fully functional in both Drosophila and mammals.

Hardly any RNAi occurred with transfection of siRNA-c, a class III siRNA, to S2 and CHO-K1 cells. Mammalian and Drosophila cells would thus appear to possess virtually the same capacity for siRNA-mediated RNAi induction, the maximum and the minimum limits of which are determined by the transfection of siRNA-1 and -c, respectively. Although within each class, siRNA-dependent RNAi activity in S2 cells increases with increasing RNAi activity in CHO-K1 cells, our rules for siRNA sequence preference may not be applicable for predicting highly effective and ineffective siRNAs for RNAi in S2 cells.

RNAi-inducing capability in S2 cells was much the same for two class Ia siRNAs (o and n) and two class III siRNAs (b and h). Three class II siRNAs (a, I, and g) were found much more effective in S2 cells compared to two class Ia siRNAs (o and n).

We noted that siRNA-n, the most ineffective class Ia siRNA, possesses a long GC stretch extending from the 5' end of the sense strand and that class Ia-siRNA-dependent RNAi activity in S2 and CHO-K1 cells is negatively correlated with the length of the GC stretch extending from the 5' end of the sense strand. Similar negative effects of a long GC stretch on RNAi were also evident in class II-or class III-dependent RNAi in CHO-K1 and S2 cells.

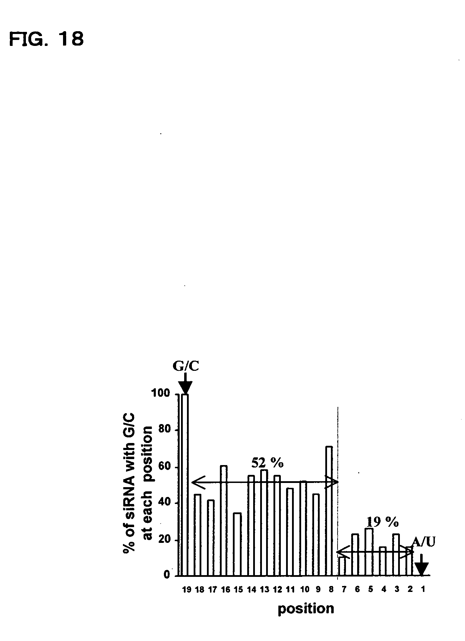

In contrast, the average GC content in the 11 bp-long region adjacent to the 5' SS end was approximately 50% in the case of the 31 highly effective class Ia siRNAs (Fig. 18). It may thus follow that a long GC stretch in the siRNA sequence serves as a suppressor of RNAi, the extent depending on length of the stretch.

### 5. Possible dual functions of the 5' end of the siRNA antisense strand

During RNAi of EGFP (enhanced green fluorescent protein) and ECFP (a derivative of EGFP), EGFP-441, an siRNA homologous in sequence to the EGFP but not completely so to the ECFP gene, was noted to be capable of effectively inactivating ECFP.

HeLa cells were transfected simultaneously with DsRed plasmid DNA (control), EGFP or ECFP plasmid DNA (target) and siRNA, and the relative number of target gene expressing cells was counted at various times. As shown in Fig. 16C, nearly all EGFP signals from EGFP expressing cells were abolished 24 hours after transfection, when EGFP-441, a cognate class Ia siRNA, was transfected, while EGCFP-666, a class III siRNA completely homologous in sequence to EGFP and ECFP genes, could reduce only a few EGFP signals 2 days following transfection. EGFP-441 is homologous in sequence to ECFP mRNA except for the position corresponding to the 5' AS end (see the right margin of Fig. 16C). Fig. 16C shows that EGFP-441 is capable of more effectively bringing about ECFP RNAi than ECFP-441, a class II siRNA completely identical in sequence to the target (ECFP mRNA).

EGFP-441 abolished nearly 70% of ECFP signals at 24 hours following transfection and the rest were almost entirely eliminated at 2 days after transfection. On challenging ECFP with the cognate siRNA, ECFP-441 (class II), most of ECFP signals could still be detected 2 days following transfection.

The presence of A/U at the 5' end of the siRNA antisense strand would thus appear essential for some RNAi process other than mRNA recognition. The fact that EGFP mRNA is a better target for EGFP-441 than ECFP indicates that the 5' end of the siRNA antisense strand is also involved in hydrogen bonding between the target mRNA and the siRNA antisense strand. Accordingly, the 5' end of the antisense strand would be likely to be involved in two separate RNAi processes, RISC formation, which includes siRNA unwinding, and mRNA recognition.

The time course of RNAi, as followed using several highly effective EGFP or ECFP siRNAs, showed target gene activity abolishment to remain at more than 70% for 7 days, at least starting from day 2. In contrast, little or no RNAi effects were evident on using ineffective class III siRNAs (data not shown).

### 6. siRNA sequence requirement for DNA-vector based RNAi

To determine whether target sequence preference in mammalian siRNA-based RNAi is intrinsic to the RNAi mechanism, a study was made to clarify whether similar rules for target sequence preference would hold for DNA-based mammalian RNAi, in which siRNA is produced via cleavage of hairpin-type RNA first transcribed and then transported from nuclei. pSilencer and firefly luc were used as vector and target genes, respectively. The profiles of RNAi activity change in DNA-induced RNAi can be seen from Fig. 19 and are basically the same as siRNA-based RNAi. That is, all the pSilencer with the DNA insert encoding hairpin-type class Ia siRNA (shRNA) induced highly efficient RNAi in mammalian cells 3 days following transfection. In contrast, little or no RNAi was induced by transfection of pSilencer with the DNA insert encoding the hairpin of class III siRNA (FL14-m23L). siRNA sequence preference in mammalian siRNA-based RNAi may thus be concluded to hold for DNA-based RNAi in mammalian cells and accordingly, should be a reflection of the intrinsic features of RNAi.

### 7. siRNA sequence requirement for RNAi in chick embryos

The siRNA sequence preference rules presented here may be applicable to RNAi in vertebrates other than mammals and may prove useful in the design of siRNAs for gene silencing in individuals. To confirm these possibilities, siRNAs designed by the present rules were introduced into the right half of the spinal cord of day 2 chick embryos by in ovo electroporation and the change in target gene activity on embryonic day 4 was examined (Fig. 16D).

EGFP and DsRed expressions served as criteria for assessing RNAi effects brought on by transfected siRNAs. EGFP-441, EGFP-416, DsRed-399 (Fig. 16D) and DsRed-231 (data not shown), all being class Ia siRNAs, were clearly shown capable of bringing about highly effective RNAi in the spinal cord of chick embryos. EGCFP-666, DsRed-140 (Fig. 16D) and DsRed-383 (data not shown), all belonging to class III, were found ineffective in this regard. Thus, our rules for siRNA sequence preference would certainly appear quite useful for the design of effective siRNAs in chick embryos.

### 8. Free energy calculation of siRNAs

The enhanced flexibility at the siRNA end containing the 5' antisense-strand end and low internal energy across the duplex (especially at the region 9-14) have recently been shown to be strongly correlated with siRNA function. Thus, internal stability reflecting the stability of pentamer subsequences was estimated in each of the 16 luc siRNAs shown in Fig. 15A, using the nearest-neighbour method.

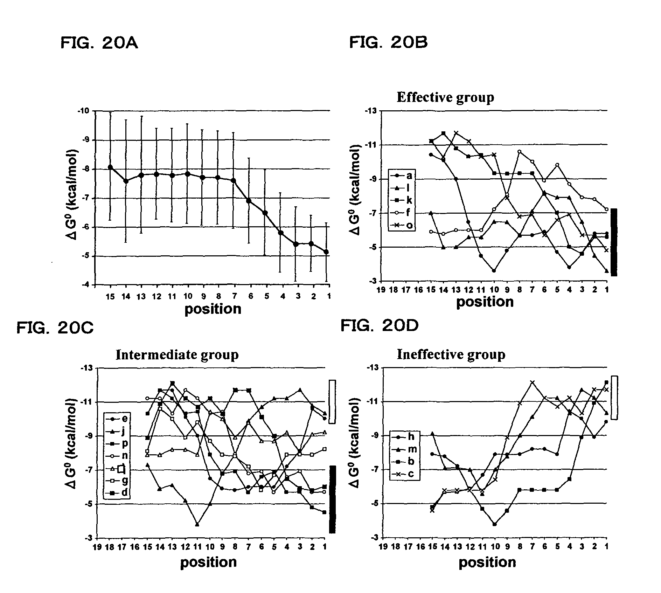

ΔG° at position 1 of 5 highly effective siRNAs varied from -3.6 to -7.2 kcal/mol (Fig. 20B), whereas for 7 siRNAs causing intermediate levels ofRNAi, from -4.5 to -10.3 kcal/mol (C) and for highly ineffective siRNAs, the values exceeded -9.8 kcal/mol (D). These values would support the notion that the duplex end containing the 5' antisense-strand end of highly effective siRNAs is considerably less thermostable.

However, our data disclosed no clear reduction in the absolute values of ΔG° in the region 9-14. To further examine this point, value distribution across the duplex was studied using 32 highly effective siRNAs shown in Figs. 15A, 15B, 16A, 16B, 16C,and 16D, but again, there was no apparent low internal energy across the duplex (Fig. 20A). Thus, the notion proposed by Khvorova et al. was partly supported by our study.

The experimental results in Figs. 20B and 20C indicate that ΔG° at position 1 of 3 siRNAs that gives rise to intermediate levels of RNAi in mammalian cells (p, n, and d) is within the range of those of 5 highly effective siRNAs (a, f, k, 1, and o). Thus, based on thermodynamic stability calculation, the selection of highly effective siRNAs from a random siRNA set may be quite likely possible but only at a probability of 60%.

### DISCUSSION

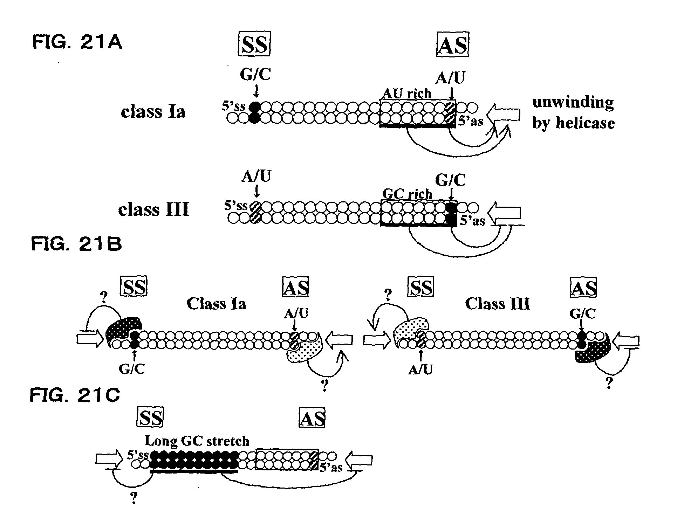

The relation between siRNA sequence and its ability to give rise to RNAi in mammalian cells was extensively examined here and, on the basis of the results, rules were established for siRNA sequence preference and are schematically presented in Fig. 21A.

siRNAs that satisfy the rules, namely, the conditions listed in this specification, will give rise to highly effective RNAi in mammalian cells and possibly also in chick embryos. Among the conditions, the four following sequence conditions, namely, A/U at the 5' AS end, G/C at the 5' SS end, at least 5 A/U residues in the 5' terminal one third of the antisense strand, and the absence of any GC stretch of more than 9 nucleotides in length, are highly important to achieve RNAi.

siRNAs opposite in features with respect to the first three conditions bring about little or no gene silencing.

A total of 57 highly effective and 16 ineffective siRNA candidates has been designed for 4 exogenous and 23 endogenous genes to date based on these rules (this work and our unpublished data) and all have been found to produce the anticipated RNAi activity in mammalian cells and chick embryos.

Recently, Holen et al. pointed out that siRNA-based RNAi in mammalian cells considerably varies depending on target sequences. Their experimental results shown in Fig. 1C of Nucleic Acid Research, 30, p.p. 1757-1766 (2002) are clearly explained based on our rules. They show that only four of 11 siRNAs examined could give rise to effective RNAi in HeLa, 293, Cos-1, and HaCaT cells. Our rules show that only these 4 effective siRNAs belong to class Ia or Ib, highly effective siRNA classes. Thus, the rules here may be concluded to be very useful for designing highly effective and ineffective siRNAs for silencing of mammalian and chick genes.

However, it should be pointed out that, while the four conditions above are almost entirely sufficient for highly effective gene-silencing, some may possibly be replaced by other functionally redundant conditions.

The secondary structure of target RNA has been shown to be important for target mRNA recognition by siRNAs. However, at variance with these considerations, our results would indicate that target sequences are much more essential for target recognition by siRNAs than the secondary structure. No special secondary structure of the target can be deduced from our rules. Possibly, the frequency of serious secondary structure occurrence may be quite low in protein-coding regions of mRNA used here as targets.

EGFP/ECFP RNAi experiments (see Fig. 16C) indicated that the presence of A/U at the 5' AS end is possibly required not only for target recognition but RISC formation as well, which includes siRNA unwinding. The step size of unwinding for UvrD DNA helicase is five base pairs and thus a one-step motor function of putative siRNA helicase may unwind several base pairs from one of the two siRNA ends at the earliest stage in RISC formation. The 7 bp-long AS terminal duplex regions of highly effective and ineffective siRNAs are AU-rich and GC-rich, respectively, and 5' AS ends of highly effective and ineffective siRNAs are A/U and G/C, respectively. It would thus follow that the putative siRNA helicase preferably initiates unwinding of the RNA duplex in an AU-rich terminal region with A/U at its 5' free end while RNA duplex unwinding from the GC-rich terminal region with G/C at its 5' free end is blocked. Our unpublished experiments (Y.N., K.U.-T. and K.S.) have indicated that while virtually no degradation of the sense target RNA (vimentin mRNA) is brought about by VIM-35, a class III vimentin siRNA, about 80% of antisense target RNA is cleaved by the same siRNA, which serves as a class Ib siRNA for antisense target silencing.

These considerations would appear consistent with the asymmetric RISC formation model recently proposed by Schwarz et al. for in vitro RNAi in Drosophila embryonic extracts. This model predicts that siRNA unwinding preferably occurs at an 'easier' duplex end, possessing A:U, G:U, or unpaired bases at its 5' end position and being thermodynamically less stable, and that the strand with the 5' end serves as a single-stranded guide RNA assembled into RISC.

The importance of thermodynamically unstable or flexible base pairs at or near the AS end for siRNA unwinding in HEK 297 cells has also been pointed out by Khvorova et al. A RISC formation mechanism similar to that proposed for the Drosophila in vitro system should thus also be applicable to mammalian and chick in vivo RNAi (see Fig. 21A).

According to the rules established here, 5' AS and SS ends of highly effective siRNAs should be A/U and G/C, respectively, with the counterparts of ineffective siRNAs being G/C and A/U (see Fig. 21B). This terminal base compositional asymmetry may be important for determining the direction of siRNA unwinding.

As shown in Figs. 21A, 21B, and 21C, siRNA unwinding might be effectively initiated from the AU-rich AS end in the case of class Ia siRNA, lacking a long GC stretch. On the other hand, siRNA duplex unwinding might be suppressed from the GC-rich class III AS end. G/C at the 5' SS end of class Ia and the 5' AS end of class III siRNAs might provide a site for binding of an unidentified protein possibly suppressing siRNA unwinding. Alternatively, A/U at the 5' SS end of class III and the 5' AS end of class Ia siRNAs might serve as a binding site for putative unwinding stimulation factors other than helicase. A long GC stretch such as that found in siRNA-n might prevent the elongation of siRNA-duplex denaturation from the AS end.

Recently, two Drosophila PIWI proteins have been shown to be capable of binding to a 5 bp single-stranded RNA or siRNA duplex. We found that the PAZ domain of eIF2C1, a human PIWI protein, binds to dsRNA with a 2-nucleotide-long 3' overhang but not to those with blunt or 5' overhang ends (N. Doi, K.U. -T., and K.S., unpublished data). In plant cells infected with tombusvirus, p19 may bind to siRNA ends and may inhibit post-transcriptional gene silencing. Thus, a protein or protein complex, possibly not relevant to helicase but capable of binding preferentially to G/C or A/U at siRNA ends, might be involved in early strand separation of siRNA so as to either suppress or stimulate siRNA duplex unwinding.

Helicase functions might be doubly suppressed by G/C at the 5' AS end position and an adjacent GC-rich sequence in highly ineffective siRNAs, while helicase functions appear blocked only by a single G/C pair at the 5' SS end position (Figs. 21A and 21B), suggesting that a single G/C pair at the 5' SS end position and a GC-rich sequence near the 5' SS end might be functionally redundant to each other and accordingly, the latter might serve as a substitute for the former. We consider that this might be the reason why siRNA-a (a class II siRNA) is capable of acting as a highly effective siRNA (see Figs. 15A and 17B).

The results in Fig. 15A indicate that siRNA-n, possessing a 10 bp-long G/C stretch extending from the SS end, is incapable of giving rise to highly effective RNAi in mammalian cells, although it belongs to class Ia. Complete strand separation of siRNA appears to be required for active RISC formation and consequently, a long G/C stretch extending from the SS end may prevent helicase from unwinding not only from the SS end but from the AS end as well in a G/C-stretch-length dependent manner (Figs. 21A and 21B).

In contrast to in vitro RNAi in Drosophila, in vivo Drosophila RNAi was far less sensitive to the siRNA sequence (see Figs. 15A and 15B); virtually all siRNAs gave rise to effective RNAi in S2 cells when used at 50 nM. Our siRNA sequence preference rules established based on mammalian RNAi data were found to not be directly applicable to in vivo Drosophila (Figs. 15A and 15B). Unlike mammalian cells, Drosophila cells might produce more protein components required for RISC formation and hence, be capable of accumulating a considerable amount of RISC with a less efficient siRNA strand. That is, asymmetric RISC formation may possibly not be a rate-limiting step in RNAi in Drosophila cells.

Figs. 17A and 17B also indicate highly effective class Ia siRNAs to be comprised of heterogeneous members with over 10 times the capacity to bring about RNAi and maximum gene silencing activity to be induced by siRNA-1 transfection of CHO-K1 and S2 cells.

Schwarz et al. indicated gene silencing activity of siRNAs in the Drosophila in vitro system to be improved by the introduction of a U:G pair or unpaired bases at the 5' AS end position. There may thus be the possibility of converting almost all class Ia siRNAs to siRNAs capable of inducing maximum levels of RNAi or RNAi levels brought about by siRNA-1 in mammalian cells via a change in terminal base pairing.

In a separate study, 19,986 human and 16,256 murine sequences registered in the NCBI Reference Sequence (RefSeq) database were examined using the siRNA sequence preference rules established here and 92% and 99% of human and mouse sequences, respectively, were noted to possess at least one unique potential target for class Ia siRNA without a long G/C stretch (Y.N., K.U.-T., and K.S., unpublished data). Our rules should thus find wide scope of application to the design of siRNAs which are highly effective for mammalian RNAi including systematic mammalian functional genomics.

### REFERENCES

1. Fire, A., Xu, S., Montgomery, M. K., Kostas, S. A., Driver, S. E., and Mello, C.C., Potent and specific genetic interference by double-stranded RNA in Caenorhabditis elegans, Nature, 391, 806-811 (1998)
2. McManus ,M. T. and Sharp, P. A., Gene silencing in mammals by small interfering RNAs, Nat. Rev. Genet., 3, 737-747 (2002)
3. Hammond, S. M., Caudy, A. A., and Hannon, G. J., Post-transcriptional gene silencing by double-stranded RNA, Nat. Rev. Genet., 2, 110-119 (2002)
4. Hannon, G. J., RNA interference, Nature, 418, 244-251 (2002)
5. Bernstein, E., Caudy, A. A., Hammond, S. M., and Hannon,G.J., Role for a bidentate ribonuclease in the initiation step of RNA interference, Nature, 409, 363-366 (2001)
6. Ketting, R. F., Fischer, S. E. J., Bernstein, E., Sijen, T., Hannon, G. J., and Plasterk, R. H. A., Dicer functions in RNA interference and in synthesis of small developmental timing in C. elegans, Genes Dev., 15, 2654-2659 (2001)
7. Hammond, S. M., Bernstein, E., Beach, D., and Hannon, G. J., An RNA-directed nuclease mediates post-transcriptional gene silencing in Drosophila cells, Nature, 404, 293-296 (2000)
8. Nykanen, A., Haley, B., and Zamore, P. D., ATP requirements and small interfering RNA structure in the RNA interference pathway, Cell, 107, 309-321 (2001)
9. Elbashir, S. M., Lendeckel, W., and Tuschl, T., RNA interference is mediated by 21 and 22 ntRNAs, Genes Dev., 15, 188-200 (2001)
10. Hammond, S. M., Boettcher, S., Caudy, A. A., Kobayashi, R., and Hannon, G. J., Argonaute2, a link between genetic and biochemical analyses of RNAi, Science, 293, 1146-1150 (2001)
11. Caudy, A. A., Myers, M., Hannon, G. J., and Hammond, S. M., Fragile X-related protein and VIG associated with the RNA interference machinery, Genes Dev., 16, 2491-2496 (2002)
12. Zamore, P. D., Tuschl, T., Sharp, P. A., and Bartel, D. P., RNAi: double-stranded RNA directs the ATP-dependent cleavage of mRNA at 21 to 23 nucleotide intervals, Cell, 101, 25-33 (2000)
13. Kataoka, Y., Takeichi, M., and Uemura, T., Developmental roles and molecular characterization of a Drosophila homologue of Arabidopsis Argonaute1, the founder of a novel gene superfamily, Genes Cells, 6, 313-325 (2001)
14. Williams, R. W. and Rubin, G. M., ARGONAUTE1 is required for efficient RNA interference in Drosophila embryos, Proc. Natl. Acad. Sci. USA, 14, 6889-6894 (2002)
15. Doi, N., Zenno, S., Ueda, R., Ohki-Hamazaki, H., Ui-Tei, K., and Saigo, K., Requirement of Dicer and eIF2C translation initiation factors for short-interfering-RNA-mediated gene silencing in mammalian cells, Curr. Biol., 13, 41-46 (2003)
16. Martinez, J., Patkaniowska, A., Urlaub, H., Lührmann, R., and Tuschl, T., Single-stranded antisense siRNAs guide target RNA cleavage in RNAi, Cell, 110, 563-574 (2002)
17. Kennerdell, J. R., Yamaguchi, S., and Carthew, R. W., RNAi is activated during Drosophila oocyte maturation in a manner dependent on aubergine and spindle-E, Genes Dev., 16, 1884-1889 (2002)
18. Tijsterman, M., Ketting, R. F., Okihara, K. L., Sijen, T., and Plasterk, R. H. A., RNA helicase MUT-14-dependent gene silencing triggered in C. elegans by short antisense RNAs, Science, 295, 694-697 (2002)
19. Elbashir, S. M., Harborth, J., Lendeckel, W., Yalcin, A., Weber, K., and Tuschl, T., Duplexes of 21-nucleotide RNAs mediate RNA interference in cultured mammalian cells, Nature, 411, 494-498 (2001)
20. Elbashir, S. M., Martinez, J., Patkaniowska, A., Lendeckel, W., and Tuschl, T., Functional anatomy of siRNAs for mediating efficient RNAi in Drosophila melanogaster embryo lysate, EMBO J., 20, 6877-6888 (2001)
21. Stark, G. R., Kerr, I. M., Williams, B. R., Silverman, R. H., and Schreiber, R. D., How cells respond to interferons. Annu. Rev. Biochem., 67, 277-264 (1998)
22. Ui-Tei, K., Zenno, S., Miyata, Y., and Saigo, K., Sensitive assay of RNA interference in Drosophila and chinese hamster cultured cells using firefly luciferase gene as target, FEBS Lett., 479, 79-82 (2000)
23. Billy, E., Brondani, V., Zhang, H., Müller, U., and Filipowicz, W., Specific interference with gene expression induced by long, double-stranded RNA in mouse embryonal teratocarcinoma cell lines, Proc. Natl. Acad. Sci. USA, 98, 14428-14433 (2001)
24. Paddison, P. J., Caudy, A. A., and Hannon, G. J., Stable suppression of gene expression by RNAi in mammalian cells, Proc. Natl. Acad. Sci. USA, 99, 1443-1448 (2002)
25. Yang, S., Tutton, S., Pierce, E., and Yoon, K., Specific double-stranded RNA interference in undifferentiated mouse embryonic stem cells, Mol. Cell. Biol., 21, 7807-7816 (2001)
26. Wianny, F. and Zemicka-Goetz, M., Specific interference with gene function by double-stranded RNA in early mouse development, Nat. Cell Biol., 2, 70-75 (1999)
27. Holen, T., Amrzguioui, M., Wiiger, M. T., Babaie, E., and Prydz, H., Positional effects of short interfering RNAs targeting the human coagulation trigger tissue factor, Nucleic Acids Res., 30, 1757-1766 (2002)
28. Harborth, J., Elbashir, S. M., Vandenburgh, K., Manninga, H., Scaringe, S. A., Weber, K., and Tuschl, T., Sequence, chemical and structural variation of small interfering RNAs and short hairpin RNAs and the effect on mammalian gene silencing, Antisense Nucleic Acid Drug Dev., 13, 83-105 (2003)
29. Schwarz, D. S., Hutvágner, G., Du, T., Xu, Z., Aronin, N., and Zamore, P. D., Asymmetry in the assembly of the RNAi enzyme complex, Cell, 115, 199-208 (2003)
30. Khvorova, A., Reynolds, A., and Jayasena, S. D., Functional siRNAs and miRNAs exhibit strand bias, Cell, 115, 209-216 (2003)
31. Niwa, H., Yamamura, K., and Miyazaki, J., Efficient selection for high-expression transfectants with a novel eukaryotic vector, Gene, 108, 193-200 (1991)
32. Lagos-Quintana, M., Rauhut, R., Lendeckel, W., and Tuschl, T., Identification of novel genes coding for small expressed RNA, Science, 294, 852-858 (2001)
33. Mourelatos, Z., Dostie, J., Paushkin, S., Sharma, A., Charroux, B., Abel, L., Rappsilber, J., Mann, M., and Dreyfuss, G., miRNPs: a novel class of ribonucleoproteins containing numerous microRNAs, Genes Dev., 16, 720-728 (2002)
34. Lim, L. P., Glasner, M. E., Yekta, S., Gurge, C. B. and Bartel, D. P., Vertebrate microRNA genes, Science, 299, 1540 (2003)
35. Freier, S. M., Kierzek, R., Jaeger, J. A., Sugimoto, N., Caruthers, N. M., Neilson, T., and Turner, D. H., Improved free-energy parameters for predictions of RNA duplex stability, Proc. Natl. Acad. Sci. USA, 83, 9373-9377 (1986)
36. Niwa, H., Miyazaki, J., and Smith, A. G., Quantitative expression of Oct-3/4 defines differentiation, dedifferentiation or self-renewal of ES cells, Nature, 24, 372-376 (2000)
37. Chun, J.-Y., Han, Y.-J., and Ahn, K.-Y., Psx homeobox gene is X-linked and specifically expressed in trophoblast cells of mouse placenta, Dev. Dyn., 216, 257-266 (1999)
38. Shi, Y., Mammalian RNAi for the masses, Trend. Genet., 19, 9-12 (2003) Brummelkamp, T. R., Bernards, R., and Agami, R., A system for stable expression of short interfering RNAs in mammalian cells, Science, 296, 550-552 (2002)
39. Kawasaki, H. and Taira, K., Short hairpin type of dsRNAs that are controlled by tRNA^{VAL} promoter significantly induce RNAi-mediated gene silencing in the cytoplasm of human cells, Nucleic Acids Res., 31, 700-707 (2003)
40. Vickers, T. A., Koo, S., Bennett, C. F., Crooke, S. T., Dean, N. M., and Baker, B. F., Efficient reduction of target RNAs by small interfering RNA and Rnase H-dependent antisense agents, J. Biol. Chem., 278, 7108-7118 (2003)
41. Kretschmer-Kazemi Far, R. and Schzakiel, G., The activity of siRNA in mammalian cells is related to structural target accessibility: a comparison with antisense oligonucleotides, Nucleic Acids Res., 31, 4417-4424 (2003)
42. Ali, J. A. and Lohman, T. M., Kinetic measurement of the step size of DNA unwinding by Escherichia coli UvrD helicase, Science, 275, 377-380 (1997)
43. Lingel, A., Simon, B., Izaurralde, E., and Sattler, M., Structure and nucleic-acid binding of the Drosophila Argonaute 2 PAZ domain, Nature, 426, 465-469 (2003)
44. Yan, K. S., Yan, S., Farooq, A., Han, A., Zeng, L., and Zhou, M.-M., Structure and conserved RNA binding of the PAZ domain, Nature, 426, 469-474 (2003)
45. Song, J.-J., Liu, J., Tolia, N. H., Schneiderman, J., Smith, S. K., Martienssen, R. A., Hannon, G. J., and Joshua-Tor, L., The crystal structure of the Argonaute2 PAZ domain reveals an RNA binding motif in RNAi effector complexes, Nat. Struct. Biol., 10, 1026-1032 (2003)
46. Silhavy, D., Molnár, A., Lucioli, A., Szittya, G., Homyik, C., Tavazza, M., and Burgyán, A viral protein suppresses RNA silencing and binds silencing-generates, 21- to 25-nucleotide double-stranded RNAs, EMBO J., 21, 3070-3080 (2002)

## Claims

1. A method for designing an oligo- or polynucleotide sequence for achieving RNA interference in mammalian cells which comprises a sense sequence, a trimming sequence and an antisense sequence, comprising:
selecting as the sense sequence a sequence consisting of (i) a sequence (B or K) which is homologous to a part of a sequence (b) of a target gene to be subjected to said RNA interference and (ii) at least one sequence selected from the group consisting of (ii-1) a sequence (C or L) which is added to the 3' end o f the sequence (B or K) and comprises 0 to 5 nucleotides and (ii-2) a sequence (D or M) which is added to the 5' end of the sequence (B) and comprises 0 to 5 nucleotides, wherein
when said sense sequence is 5' of said trimming sequence, the base on the terminal nucleotide at the 5' end of the sense sequence is guanine and the base on the terminal nucleotide at the 3' end of the sense sequence is adenine, thymine or uracil, and
when said sense sequence is 3' of said trimming sequence, the base on the terminal nucleotide at the 5' end of the sense sequence is guanine or cytosine and the base on the terminal nucleotide at the 3' end of the sense sequence is cytosine, thymine or uracil and the 7-bp-long region of the 3' terminal of the sense sequence is rich in at least one base selected from the group consisting of adenine, thymine and uracil, and
the number of nucleotides in the sense sequence is such that RNA interference can occur without cytotoxicity;
selecting as the antisense sequence a sequence complementary to the sense sequence, wherein the nucleotides of the 5' and 3' ends of the antisense sequence are entirely complementary to the corresponding nucleotides of the sense sequence, respectively,
and when the antisense sequence is 3' of said trimming sequence, the 7-bp-long region of the 5' terminal of the antisense sequence is rich in at least one base selected from the group consisting of adenine, thymine and uracil, and the number of nucleotides in the antisense sequence is such that RNA interference can occur without cytotoxicity; and
selecting as the trimming sequence a sequence which comprises 5 to 52 nucleotides and is represented by the formula: (G or C)-X-Y-Z-(C or G), wherein the nucleotides at either end are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
wherein the sense sequence, the trimming sequence, and the antisense sequence are consecutive in the order sense sequence, trimming sequence and antisense sequence or antisense sequence, trimming sequence and sense sequence, and this consecutive sequence does not comprise four or more consecutive nucleotides with thymine and/or uracil bases.

2. A method as claimed in claim 1, wherein said sequence comprises said sense sequence, trimming sequence and anti-sense sequence in this order and said method comprises:
selecting as the sense sequence a sequence (A) consisting of (i) a sequence (B) which is homologous to a part of a sequence (b) of a target gene to be subjected to said RNA interference and (ii) at least one sequence selected from the group consisting of (ii-1) a sequence (C) which is added to the 3' end of the sequence (B) and comprises 0 to 5 nucleotides and (ii-2) a sequence (D) which is added to the 5' end of the sequence (B) and comprises 0 to 5 nucleotides, wherein the base on the terminal nucleotide at the 5' end of the sense sequence is guanine, the base on the terminal nucleotide at the 3' end of the sense sequence is adenine, uracil or thymine, and the number of nucleotides in the sense sequence is such that RNA interference can occur without cytotoxicity;
selecting as the antisense sequence a sequence (E) which is complementary to the sense sequence, wherein the nucleotides of the 5' and 3' ends of the antisense sequence are entirely complementary to the corresponding nucleotides of the sense sequence, respectively, the 7-bp-long region of the 5' terminal of the antisense sequence is rich in at least one base selected from the group consisting of adenine, uracil and thymine, and the number of nucleotides in the antisense sequence is such that RNA interference can occur without cytotoxicity; and
selecting as the trimming sequence a sequence (F) which comprises 5 to 52 nucleotides and is represented by the formula: (G or C)-X-Y-Z-(C or G), wherein the nucleotides at either end are entirely complementary to each other, the X region and the Z region each comprise 0 to 10 nucleotides with bases selected from the group consisting of adenine, uracil, thymine, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
wherein the sense sequence, the trimming sequence, and the antisense sequence are consecutive, and this consecutive sequence does not comprise four or more consecutive nucleotides with uracil or thymine bases.

3. The method according to claim 2, wherein the sequence comprises an overhang portion which consists of 1 to 3 nucleotides and is added to the 3' end of the antisense sequence.

4. The method according to claim 2 or 3, wherein the sense sequence is identical to a part of the sequence (b) of the target gene except for the terminal nucleotide of the sense sequence at its 3' end, and the antisense sequence is entirely complementary to the sense sequence.

5. The method according to any one of claims 2 to 4, wherein the lengths of the sense sequence and the anti-sense sequence are decided so that the cleavage sites by Dicer are between the second nucleotide and the third nucleotide from the 5' end of the trimming sequence, and between the trimming sequence and the antisense sequence.

6. A method as claimed in claim 1, wherein said sequence comprises said antisense sequence, trimming sequence, and sense sequence in this order, and said method comprises:
selecting as the sense sequence a sequence (J) consisting of (i) a sequence (K) which is homologous to a part of a sequence (b) of a target gene which is to be subjected to said RNA interference and (ii) at least one sequence selected from the group consisting of (ii-1) a sequence (L) which is added to the 3' end o f the sequence (K) and comprises 0 to 5 nucleotides and (ii-2) a sequence (M) which is added to the 5' end of the sequence (K) and comprises 0 to 5 nucleotides, wherein the base on the terminal nucleotide at the 5' end of the sense sequence is guanine or cytosine, the base on the terminal nucleotide at the 3' end of the sense sequence is cytosine, uracil or thymine, the 7-bp-long region of the 3' terminal of the sense sequence is rich in at least one base selected from the group consisting of adenine, uracil and thymine, and the number of nucleotides in the sense sequence is such that RNA interference can occur without giving cytotoxicity;
selecting as the antisense sequence a sequence (N) which is complementary to the sense sequence, wherein the nucleotides of the 5' and 3' ends of the antisense sequence are entirely complementary to the corresponding nucleotides of the sense sequence, respectively, and the number of nucleotides in the antisense sequence is such that RNA interference can occur without giving cytotoxicity; and
selecting as the trimming sequence a sequence (F) which comprises 5 to 52 nucleotides and is represented by the formula: (G or C)-X-Y-Z-(C or G), wherein the nucleotides at either end are entirely complementary to each other, the X region and the Z region each comprise 0 to 10 nucleotides with bases selected from the group consisting of adenine, uracil, thymine, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
wherein the antisense sequence, the trimming sequence, and the sense sequence are consecutive, and this consecutive sequence does not comprise four or more consecutive nucleotides with uracil or thymine bases.

7. The method according to claim 6, wherein the sequence comprises an overhang portion which consists of 1 to 3 nucleotides and is added to the 3' end of the sense sequence.

8. The method according to claim 6 or 7, wherein the sense sequence is identical to a part of the sequence (b) of the target gene except for the terminal nucleotide of the sense sequence at its 5' end, and the antisense sequence is entirely complementary to the sense sequence.

9. The method according to any one of claims 6 to 8, wherein the lengths of the sense sequence and the anti-sense sequence are decided so that the cleavage sites by Dicer are between the second nucleotide and the third nucleotide from the 5' end of the trimming sequence, and between the trimming sequence and the sense sequence.

10. The method according to any one of claims 1 to 9, wherein the sense sequence and the antisense sequence comprise no long stretches of nucleotides on which the bases are guanine and/or cytosine.

11. The method according to any one of claims 1 to 10, wherein the Y region comprises 6 to 20 nucleotides which are not complementary to each other and becomes a loop portion when the sense strand and the antisense strand are bound to one another in a double-stranded portion.

12. The method according to any one of claims 1 to 11, wherein the sense sequence and the antisense sequence each comprises 13 to 28 nucleotides.

13. The method according to any one of claims 1 to 3, 5 to 7 and 9 to 12, wherein the sense sequence is identical to a part of sequence (b) of the target gene, and the antisense sequence is entirely complementary to the sense sequence.

14. The method according to any one of claims 1 to 13, wherein the sequence comprises 30 to 90 nucleotides.

15. The method of any one of claims 1 to 14, wherein the oligo- or polynucleotide is DNA.

16. The method according to any one of claims 1 to 14, wherein the oligo- or polynucleotide is RNA.

17. The method according to any one of claims 1 to 14, wherein the oligo- or polynucleotide is shRNA which can cause RNA interference in mammalian cells, when expressed by an RNA polymerase III-type promoter.

18. An oligo- or polynucleotide molecule obtainable by the method as defined in any one of claims 1 to 17.

19. An oligo- or polynucleotide for achieving RNA interference comprising a sense sequence, a trimming sequence, and an antisense sequence in this order, wherein:
the sense sequence consists of (i) a sequence (B) which is homologous to a part of a sequence (b) of a target gene to be subjected to said RNA interference and (ii) at least one sequence selected from the group consisting of (ii-1) a sequence (C) which is added to the 3' end of the sequence (B) and comprises 0 to 5 nucleotides and (ii-2) a sequence (D) which is added to the 5' end of the sequence (B) and comprises 0 to 5 nucleotides, wherein the base on the terminal nucleotide at the 5' end of the sense sequence is guanine, the base on the terminal nucleotide at the 3' end of the sense sequence is adenine, thymine or uracil, and the number of nucleotides in the sense sequence is such that RNA interference can occur without cytotoxicity;
the antisense sequence is complementary to the sense sequence, wherein the nucleotides of the 5' and 3' ends of the antisense sequence are entirely complementary to the corresponding nucleotides of the sense sequence, respectively, the 7-bp-long region of the 5' terminal of the antisense sequence is rich in at least one base selected from the group consisting of adenine, thymine and uracil, and the number of nucleotides in the antisense sequence is such that RNA interference can occur without cytotoxicity; and
the trimming sequence comprises 5 to 52 nucleotides and is represented by the formula: (G or C)-X-Y-Z-(C or G), wherein the nucleotides at either end are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
wherein the sense sequence, the trimming sequence, and the antisense sequence are consecutive, and this consecutive sequence does not comprise four or more consecutive nucleotides with thymine and/or uracil bases.

20. An oligo- or polynucleotide according to claim 19 wherein said oligo- or polynucleotide has the features as defined in any one of claims 3 to 5.

21. An oligo- or polynucleotide for achieving RNA interference comprising an antisense sequence, a trimming sequence, and a sense sequence in this order, wherein:
the sense sequence consists of (i) a sequence (K) which is homologous to a part of a sequence (b) of a target gene to be subjected to said RNA interference and (ii) at least one sequence selected from the group consisting of (ii-1) a sequence (L) which is added to the 3' end of the sequence (K) and comprises 0 to 5 nucleotides and (ii-2) a sequence (M) which is added to the 5' end of the sequence (K) and comprises 0 to 5 nucleotides, wherein the base at the terminal nucleotide of the 5' end of the sense sequence is guanine or cytosine, the base at the terminal nucleotide of the 3' end of the sense sequence is cytosine, thymine or uracil, the 7-bp-long region of the 3' terminal of the sense sequence is rich in at least one base selected from the group consisting of adenine, thymine and uracil, and the number of nucleotides in the sense sequence is such that RNA interference can occur without cytotoxicity;
the anti-sense sequence is complementary to the sense sequence, wherein the nucleotides of the 5' and 3' ends of the antisense sequence are entirely complementary to the corresponding nucleotides of the sense sequence, respectively, and the number of nucleotides in the antisense sequence is such that RNA interference can occur without cytotoxicity; and
the trimming sequence comprises 5 to 52 nucleotides and is represented by the formula: (G or C)-X-Y-Z-(C or G), wherein the nucleotides at either end are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
wherein the antisense sequence, the trimming sequence, and the sense sequence are consecutive, and this consecutive sequence does not comprise four or more consecutive nucleotides with thymine and/or uracil bases.

22. An oligo- or polynucleotide according to claim 22 wherein said oligo- or polynucleotide has the features as defined in any one of claims 7 to 9.

23. An oligo- or polynucleotide according to any one of claims 19 to 22 wherein said oligo- or polynucleotide has the features as defined in any one of claims 10 to 17.

24. A double-stranded DNA comprising the oligo- or polynucleotide molecule as claimed in any one of claims 18 to 23, wherein said molecule is DNA, and another DNA which is complementary to said DNA molecule of any one of claims 18 to 23.

25. A recombinant DNA comprising an RNA polymerase III-type transcriptional promoter and the double-stranded DNA of claim 24 which has been inserted downstream of the promoter.

26. The recombinant DNA according to claim 25, for use in gene therapy.

27. The recombinant DNA according to claim 25, for use in the prevention of a disease.

28. A double-stranded RNA comprising the oligo- or polynucleotide molecule as claimed in any one of claims 18 to 23, wherein said molecule is RNA, and another RNA which is complementary to said RNA of any one of claims claim 18 to 23.

29. A cell transfected with the recombinant DNA of claim 25.

30. A method for causing RNA interference in a mammalian cell comprising transfecting the recombinant DNA of claim 25 to the mammalian cell.

31. A method for designing an oligo- or polynucleotide sequence for achieving RNA interference in mammalian cells, comprising:
(1) a step comprising acquiring information of a nucleotide sequence of a target gene for RNA interference and creating information of partial sequences each having a predetermined number of consecutive nucleotides from the acquired information;
(2) a step of judging the nucleotide at the 5' end wherein a sequence(s) with a guanine base on the terminal nucleotide at the 5' end is selected from the partial sequences in the created information of partial sequences;
(3) a step of judging the nucleotide at the 3' end wherein a sequence(s) with an adenine, thymine, or uracil base on the terminal nucleotide at the 3' end is selected from the partial sequences in the created information of partial sequences;
(4) a step of judging whether specific nucleotides are present wherein a sequence(s) which is rich in at least one base selected from the group consisting of adenine, thymine and uracil in 7 nucleotides at the 3' terminal is selected from the partial sequences in the created information of partial sequences;
(5) a step of designing a sense sequence/antisense sequence wherein for each of the partial sequence(s) that were selected in all of the above steps (2), (3), and (4), a sense sequence that is identical to the selected sequence and an antisense sequence that is entirely complementary to the sense sequence are determined;
(6) a step of designing a trimming sequence wherein a trimming sequence comprising 5 to 52 nucleotides is determined which is represented by the formula: (G or C)-X-Y-Z-(C or G), wherein the nucleotides of both ends are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
(7) a step of designing an oligo- or polynucleotide sequence wherein the sequences that were decided in the above steps (5) and (6) are consecutively arranged in the order of the sense sequence, the trimming sequence, and the antisense sequence; and
(8) a step of selecting at least one sequence from the sequences designed in the above step (7) which does not contain four or more consecutive nucleotides with the bases thymine and/or uracil.

32. A method for designing an oligo- or polynucleotide sequence for achieving RNA interference in mammalian cells, comprising:
(1) a step of acquiring information of a nucleotide sequence of a target gene for RNA interference and creating information of partial sequences each having a predetermined number of consecutive nucleotides from the acquired information;
(2) a step of judging the nucleotide at the 5' end wherein a sequence(s) with a guanine or cytosine base on the terminal nucleotide at the 5' end is selected from the partial sequences in the created information of partial sequences;
(3) a step of judging the nucleotide at the 3' end wherein a sequence(s) with a cytosine, thymine, or uracil base on the terminal nucleotide at the 3' end is selected from the partial sequences in the created information of partial sequences;
(4) a step of judging whether specific nucleotides are present wherein a sequence(s) which is rich in at least one base selected from the group consisting of adenine, thymine and uracil in 7 nucleotides at the 3' terminal is selected from the partial sequences in the created information of partial sequences;
(5) a step of designing a sense sequence/antisense sequence wherein for each of the partial sequence(s) that was selected in all of the above steps (2), (3), and (4), a sense sequence that is identical to the selected sequence and an antisense sequence that is entirely complementary to the sense sequence are determined;
(6) a step of designing a trimming sequence wherein a trimming sequence comprising 5 to 52 nucleotides is determined which is represented by the formula: (G or C)-X-Y-Z-(C or G), wherein the nucleotides of both ends are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
(7) a step of designing an oligo- or polynucleotide sequence wherein the sequences that were decided in the above steps (5) and (6) are consecutively arranged in the order of the sense sequence, the trimming sequence, and the antisense sequence; and
(8) a step of selecting at least one sequence from the sequences designed in the above step (7) which does not contain four or more consecutive nucleotides with the bases thymine and/or uracil.

33. A method as claimed in claim 31 or 32 for designing an oligo- or polynucleotide as defined in any one of claims 18 to 23.

34. A method for designing the sequence of an shRNA as defined in any one of claims 1 to 17 or 31 to 33 wherein said design is achieved by a data processing apparatus.

35. A data processing apparatus configured for processing information for designing an oligo- or polynucleotide sequence for achieving RNA interference in mammalian cells, comprising:
(1) a portion for acquiring information of a nucleotide sequence of a target gene for RNA interference and creating information of partial sequences each having a predetermined number of consecutive nucleotides from the acquired information;
(2) a portion for judging the nucleotide at the 5' end wherein a sequence(s) with a guanine base on the terminal nucleotide at the 5' end is selected from the partial sequences in the created information of partial sequences;
(3) a portion for judging the nucleotide at the 3' end wherein a sequence(s) with an adenine, thymine, or uracil base on the terminal nucleotide at the 3' end is selected from the partial sequences in the created information of partial sequences;
(4) a portion for judging whether specific nucleotides are present, wherein a sequence(s) which is rich in at least one base selected from the group consisting of adenine, thymine and uracil in 7 nucleotides at the 3' terminal is selected from the partial sequences in the created information of partial sequences;
(5) a portion for designing a sense sequence/antisense sequence wherein for each of the partial sequence(s) that was selected in all of the above portions (2), (3), and (4), a sense sequence that is identical to the selected sequence and an antisense sequence that is entirely complementary to the sense sequence are determined;
(6) a portion for designing a trimming sequence wherein a trimming sequence comprising 5 to 52 nucleotides is determined which is represented by the formula: (G or C)-X-Y-Z-(C or G), wherein the nucleotides of both ends are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
(7) a portion for designing an oligo- or polynucleotide sequence wherein the sequences that were decided in the above portions (5) and (6) are consecutively arranged in the order of the sense sequence, the trimming sequence, and the antisense sequence; and
(8) a portion for selecting at least one sequence from the sequences designed in the above portion (7) which does not contain four or more consecutive nucleotides with the bases thymine and/or uracil.

36. A data processing apparatus configured for processing information for designing an oligo- or polynucleotide sequence for achieving RNA interference in mammalian cells, comprising:
(1) a portion for acquiring information of a nucleotide sequence of a target gene for RNA interference and creating information of partial sequences each having a predetermined number of consecutive nucleotides from the acquired information;
(2) a portion for judging the nucleotide at the 5' end wherein a sequence(s) with a guanine or cytosine base on the terminal nucleotide at the 5' end is selected from the partial sequences in the created information of partial sequences;
(3) a portion for judging the nucleotide at the 3' end wherein a sequence(s) with a cytosine, thymine or uracil base on the terminal nucleotide at the 3' end is selected from the partial sequences in the created information of partial sequences;
(4) a portion for judging whether specific nucleotides are present wherein a sequence(s) which is rich in at least one base selected from the group consisting of adenine, thymine and uracil in 7 nucleotides at the 3' terminal is selected from the partial sequences in the created information of partial sequences;
(5) a portion for designing a sense sequence/antisense sequence wherein for each of the partial sequence(s) that was selected in all of the above portions (2), (3), and (4), a sense sequence that is identical to the selected sequence and an antisense sequence that is entirely complementary to the sense sequence are determined;
(6) a portion for designing a trimming sequence wherein a trimming sequence comprising 5 to 52 nucleotides is determined which is represented by the formula: (G or C)-X-Y-Z-(C or G), wherein the nucleotides of both ends are entirely complementary to each other, the X region and the Z region each comprise 0 to 10 nucleotides with bases selected from the group consisting of adenine, thymine, uracil, guanine and cytosine, and are complementary to each other when the trimming sequence is folded in the Y region, the Y region comprises 3 to 50 nucleotides, and in the Y region at least one nucleotide adjacent to the X region and at least one nucleotide adjacent to the Z region are not complementary to each other;
(7) a portion for designing an oligo- or polynucleotide sequence wherein the sequences that were decided in the above portions (5) and (6) are consecutively arranged in the order of the sense sequence, the trimming sequence, and the antisense sequence; and
(8) a portion for selecting at least one sequence from the sequences designed in the above portion (7) which does not contain four or more consecutive nucleotides with the bases thymine and/or uracil.

37. The data processing apparatus of claim 35 or 36 for processing information for designing an oligo- or polynucleotide as defined in any one of claims 18 to 23.

38. A computer program for practicing a method for processing information for designing an oligo- or polynucleotide sequence according to the method of any one of claims 31 to 34.

39. A data processing apparatus onto which a program according to claim 38 is installed.

40. A computer-readable recording medium on which the program as defined in claim 38 is recorded.

41. A method of preparing an oligo- or polynucleotide molecule suitable for achieving RNA interference in mammalian cells, comprising designing the sequence according to a method of any one of claims 1 to 17 or 31 to 34 and producing an oligo- or polynucleotide molecule with said sequence.


SEQUENCE LISTING

<110> SAIGO, Kaoru
TEI, Kumiko
<120> OLIGO- or POLYNUCLEOTIDES
<130> US60/539332
<140> FP005
<141> 2004-01-28
<160> 141
<170> PatentIn version 3.1

<210> 1
<211> 20
<212> DNA
<213> Artificial
<220>
<223> PCR primer
<400> 1
gcctcatccg gtagacaaaa 20

<210> 2
<211> 20
<212> DNA
<213> Artificial
<220>
<223> PCR primer
<400> 2
accgtggtca tgagtccttc 20

<210> 3
<211> 20
<212> DNA

<213> Artificial

<220>

<223> PCR primer

<400> 3

agctgctgaa gcagaagagg 20

<210> 4

<211> 19

<212> DNA

<213> Artificial

<220>

<223> PCR primer

<400> 4

tgtcacctcc cttgccttg 19

<210> 5

<211> 21

<212> RNA

<213> Artificial

<220>

<223> sensestrand siRNA as evaluation subject

<400> 5

acgccaaaaa cauaaagaaa g 21

<210> 6

<211> 21

<212> RNA

<213> Artificial

<220>

<223> antisense strand siRNA as evaluation subject

<400> 6

uucuuuaugu uuuuggcguc u 21

<210> 7
<211> 21
<212> RNA
<213> Artificial
<220>
<223> sense strand siRNA as evaluation subject
<400> 7
gguaaaguug uuccauuuuu u 21

<210> 8
<211> 21
<212> RNA
<213> Artificial
<220>
<223> antisense strand siRNA as evaluation subject
<400> 8
aaaauggaac aacuuuaccg a 21

<210> 9
<211> 21
<212> RNA
<213> Artificial
<220>
<223> sense strand siRNA as evaluation subject
<400> 9
gggcgcgguc gguaaaguug u 21

<210> 10
<211> 21
<212> RNA
<213> Artificial

<220>
<223> antisense strand siRNA as evaluation subject
<400> 10
aacuuuaccg accgcgcccg g 21

<210> 11
<211> 21
<212> RNA
<213> Artificial
<220>
<223> sense strand siRNA as evaluation subject
<400> 11
cauucuaucc gcuggaagau g 21

<210> 12
<211> 21
<212> RNA
<213> Artificial
<220>
<223> antisense strand siRNA as evaluation subject
<400> 12
ucuuccagcg gauagaaugg c 21

<210> 13
<211> 21
<212> RNA
<213> Artificial
<220>
<223> sense strand siRNA as evaluation subject
<400> 13
ccgccgccgu uguuguuuug g 21

<210> 14
<211> 21
<212> RNA
<213> Artificial
<220>
<223> antisense strand siRNA as evaluation subject
<400> 14
aaaacaacaa cggcggcggg a 21

<210> 15
<211> 21
<212> RNA
<213> Artificial
<220>
<223> sense strand siRNA as evaluation subject
<400> 15
cggcgccauu cuauccgcug g 21

<210> 16
<211> 21
<212> RNA
<213> Artificial
<220>
<223> antisense strand siRNA as evaluation subject
<400> 16
agcggauaga auggcgccgg g 21

<210> 17
<211> 21
<212> RNA
<213> Artificial
<220>

<223> sense strand siRNA as evaluation subject
<400> 17
gaugauaaac cgggcgcggu c 21

<210> 18
<211> 21
<212> RNA
<213> Artificial
<220>
<223> antisense strand siRNA as evaluation subject
<400> 18
ccgcgcccgg uuuaucaucc c 21

<210> 19
<211> 21
<212> RNA
<213> Artificial
<220>
<223> sense strand siRNA as evaluation subject
<400> 19
cgccgccguu guuguuuugg a 21

<210> 20
<211> 21
<212> RNA
<213> Artificial
<220>
<223> antisense strand siRNA as evaluation subject
<400> 20
caaaacaaca acggcggcgg g 21

<210> 21

<211> 21
<212> RNA
<213> Artificial
<220>
<223> sense strand siRNA as evaluation subject
<400> 21
cccgccgccg uuguuguuuu g 21

<210> 22
<211> 21
<212> RNA
<213> Artificial
<220>
<223> antisense strand siRNA as evaluation subject
<400> 22
aaacaacaac ggcggcggga a 21

<210> 23
<211> 21
<212> RNA
<213> Artificial
<220>
<223> sense strand siRNA as evaluation subject
<400> 23
auggaaccgc uggagagcaa c 21

<210> 24
<211> 21
<212> RNA
<213> Artificial
<220>
<223> antisense strand siRNA as evaluation subject

<400> 24
ugcucuccag cgguuccauc u                                21

<210> 25
<211> 21
<212> RNA
<213> Artificial
<220>
<223> senses trand siRNA as evaluation subject
<400> 25
auccgcugga agauggaacc g                                21

<210> 26
<211> 21
<212> RNA
<213> Artificial
<220>
<223> antisense strand siRNA as evaluation subject

<400> 26
guuccaucuu ccagcggaua g                                21
<210> 27
<211> 21
<212> RNA
<213> Artificial
<220>
<223> sense strand siRNA as evaluation subject
<400> 27
aaggcccggc gccauucuau c                                21

<210> 28
<211> 21

<212> RNA
<213> Artificial
<220>
<223> antisense strand siRNA as evaluation subject
<400> 28
uagaauggcg ccgggccuuu c                                21

<210> 29
<211> 21
<212> RNA
<213> Artificial
<220>
<223> sense strand siRNA as evaluation subject
<400> 29
uggaagaugg aaccgcugga g                                21

<210> 30
<211> 21
<212> RNA
<213> Artificial
<220>
<223> antisense strand siRNA as evaluation subject
<400> 30
ccagcgguuc caucuuccag c                                21

<210> 31
<211> 21
<212> RNA
<213> Artificial
<220>
<223> sense strand siRNA as evaluation subject
<400> 31

ggugaacuuc ccgccgccgu u                                21

<210> 32
<211> 21
<212> RNA
<213> Artificial
<220>
<223> antisense strand siRNA as evaluation subject
<400> 32
cggcggcggg aaguucaccg g                                21

<210> 33
<211> 21
<212> RNA
<213> Artificial
<220>
<223> sense strand siRNA as evaluation subject
<400> 33
aaaacauaaa gaaaggcccg g                                21

<210> 34
<211> 21
<212> RNA
<213> Artificial
<220>
<223> antisense strand siRNA as evaluation subject
<400> 34
gggccuuucu uuauguuuuu g                                21

<210> 35
<211> 21
<212> RNA

<213> Artificial
<220>
<223> sense strand siRNA as evaluation subject
<400> 35
uaaagaaagg cccggcgcca u 21

<210> 36
<211> 21
<212> RNA
<213> Artificial
<220>
<223> antisense strand siRNA as evaluation subject
<400> 36
ggcgccgggc cuuucuuuau g 21

<210> 37
<211> 21
<212> RNA
<213> Artificial
<220>
<223> sense strand siRNA as evaluation subject
<400> 37
gacgccaaaa acauaaagaa a 21

<210> 38
<211> 21
<212> RNA
<213> Artificial
<220>
<223> antisense strand siRNA as evaluation subject
<400> 38
ucuuuauguu uuuggcgucu u 21

<210> 39
<211> 21
<212> RNA
<213> Artificial
<220>
<223> sense strand siRNA as evaluation subject
<400> 39
guuggcagaa gcuaugaaac g 21

<210> 40
<211> 21
<212> RNA
<213> Artificial
<220>
<223> antisense strand siRNA as evaluation subject
<400> 40
uuucauagcu ucugccaacc g 21

<210> 41
<211> 21
<212> RNA
<213> Artificial
<220>
<223> sense strand siRNA as evaluation subject
<400> 41
guguugggcg cguuauuuau c 21

<210> 42
<211> 21
<212> RNA
<213> Artificial

<220>
<223> antisense strand siRNA as evaluation subject
<400> 42
uaaauaacgc gcccaacacc g 21

<210> 43
<211> 21
<212> RNA
<213> Artificial
<220>
<223> sense strand siRNA as evaluation subject
<400> 43
ccgcgaacga cauuuauaau g 21

<210> 44
<211> 21
<212> RNA
<213> Artificial
<220>
<223> antisense strand siRNA as evaluation subject
<400> 44
uuauaaaugu cguucgcggg c 21

<210> 45
<211> 21
<212> RNA
<213> Artificial
<220>
<223> sensestrand siRNA as evaluation subject
<400> 45
ccaaucaucc aaaaaauuau u 21

<210> 46
<211> 21
<212> RNA
<213> Artificial
<220>
<223> antisense strand siRNA as evaluation subject
<400> 46
   uaauuuuuug gaugauuggg a 21

<210> 47
<211> 21
<212> RNA
<213> Artificial
<220>
<223> sense strand siRNA as evaluation subject
<400> 47
ccucccgguu uuaaugaaua c 21

<210> 48
<211> 21
<212> RNA
<213> Artificial
<220>
<223> antisense strand siRNA as evaluation subject
<400> 48
auucauuaaa accgggaggu a 21

<210> 49
<211> 21
<212> RNA
<213> Artificial
<220>

<223> sense strand siRNA as evaluation subject
<400> 49
gcaugccaga gauccuauuu u 21

<210> 50
<211> 21
<212> RNA
<213> Artificial
<220>
<223> antisense strand siRNA as evaluation subject
<400> 50
aauaggaucu cuggcaugcg a 21

<210> 51
<211> 21
<212> RNA
<213> Artificial
<220>
<223> sense strand siRNA as evaluation subject
<400> 51
ccggauacug cgauuuuaag u 21

<210> 52
<211> 21
<212> RNA
<213> Artificial
<220>
<223> antisense strand siRNA as evaluation subject
<400> 52
uuaaaaucgc aguauccgga a 21

<210> 53

<211> 21
<212> RNA
<213> Artificial
<220>
<223> sense strand siRNA as evaluation subject
<400> 53
gguuuuggaa uguuuacuac a                                21

<210> 54
<211> 21
<212> RNA
<213> Artificial
<220>
<223> antisense strand siRNA as evaluation subject
<400> 54
uaguaaacau uccaaaaccg u                                21

<210> 55
<211> 21
<212> RNA
<213> Artificial
<220>
<223> sense strand siRNA as evaluation subject
<400> 55
gauuucgagu cgucuuaaug u                                21

<210> 56
<211> 21
<212> RNA
<213> Artificial
<220>
<223> antisense strand siRNA as evaluation subject

<400> 56
auuaagacga cucgaaaucc a                                21

<210> 57
<211> 21
<212> RNA
<213> Artificial
<220>
<223> sense strand siRNA as evaluation subject
<400> 57
gcacucugau ugacaaauac g                                21

<210> 58
<211> 21
<212> RNA
<213> Artificial
<220>
<223> antisense strand siRNA as evaluation subject
<400> 58
uauuugucaa ucagagugcu u                                21

<210> 59
<211> 21
<212> RNA
<213> Artificial
<220>
<223> sense strand siRNA as evaluation subject
<400> 59
caaauacgau uuaucuaauu u                                21

<210> 60
<211> 21

<212> RNA
<213> Artificial
<220>
<223> antisense strand siRNA as evaluation subject
<400> 60
auuagauaaa ucguauuugu c 21

<210> 61
<211> 21
<212> RNA
<213> Artificial
<220>
<223> sense strand siRNA as evaluation subject
<400> 61
gauuaugucc gguuauguaa a 21

<210> 62
<211> 21
<212> RNA
<213> Artificial
<220>
<223> antisense strand siRNA as evaluation subject
<400> 62
uacauaaccg gacauaauca u 21

<210> 63
<211> 21
<212> RNA
<213> Artificial
<220>
<223> sense strand siRNA as evaluation subject
<400> 63

gccugaaguc ucugauuaag u                                21

<210> 64
<211> 21
<212> RNA
<213> Artificial
<220>
<223> antisense strand siRNA as evaluation subject
<400> 64
uuaaucagag acuucaggcg g                                21

<210> 65
<211> 21
<212> RNA
<213> Artificial
<220>
<223> sense strand siRNA as evaluation subject
<400> 65
cucgacgcaa gaaaaaucag a                                21

<210> 66
<211> 21
<212> RNA
<213> Artificial
<220>
<223> antisense strand siRNA as evaluation subject
<400> 66
ugauuuuucu ugcgucgagu u                                21

<210> 67
<211> 21
<212> RNA

<213> Artificial

<220>

<223> sense strand siRNA as evaluation subject

<400> 67

aacauaaaga aaggcccggc g 21

<210> 68

<211> 21

<212> RNA

<213> Artificial

<220>

<223> antisense strand siRNA as evaluation subject

<400> 68

ccgggccuuu cuuuauguuu u 21

<210> 69

<211> 21

<212> RNA

<213> Artificial

<220>

<223> sense strand siRNA as evaluation subject

<400> 69

uaugccggug uugggcgcgu u 21

<210> 70

<211> 21

<212> RNA

<213> Artificial

<220>

<223> antisense strand siRNA as evaluation subject

<400> 70

cgcgcccaac accggcauaa a 21

<210> 71
<211> 21
<212> RNA
<213> Artificial
<220>
<223> sense strand siRNA as evaluation subject
<400> 71
aguugcaguu gcgcccgcga a 21

<210> 72
<211> 21
<212> RNA
<213> Artificial
<220>
<223> antisense strand siRNA as evaluation subject
<400> 72
cgcgggcgca acugcaacuc c 21

<210> 73
<211> 21
<212> RNA
<213> Artificial
<220>
<223> sense strand siRNA as evaluation subject
<400> 73
acgugcaaaa aaagcuccca a 21

<210> 74
<211> 21
<212> RNA
<213> Artificial

<220>
<223> antisense strand siRNA as evaluation subject
<400> 74
gggagcuuuu uuugcacguu c 21

<210> 75
<211> 21
<212> RNA
<213> Artificial
<220>
<223> antisense strand siRNA as evaluation subject
<400> 75
uucugauuac acccgagggg g 21

<210> 76
<211> 21
<212> RNA
<213> Artificial
<220>
<223> antisense strand siRNA as evaluation subject
<400> 76
cccucgggug uaaucagaau a 21

<210> 77
<211> 21
<212> RNA
<213> Artificial
<220>
<223> sense strand siRNA as evaluation subject; VIM270
<400> 77
ccaucaacac cgaguucaag a 21

<210> 78
<211> 21
<212> RNA
<213> Artificial
<220>
<223> antisense strand siRNA as evaluation subject;VIM270
<400> 78
uugaacucgg uguugauggc g 21

<210> 79
<211> 21
<212> RNA
<213> Artificial
<220>
<223> sense strand siRNA as evaluation subject;VIM368
<400> 79
cuggagcagc agaauaagau c 21

<210> 80
<211> 21
<212> RNA
<213> Artificial
<220>
<223> antisense strand siRNA as evaluation subject;VIM368
<400> 80
ucuuauucug cugcuccagg a 21

<210> 81
<211> 21
<212> RNA
<213> Artificial
<220>

<223> sense strand siRNA as evaluation subject;VIM596

<400> 81

gaaaacaccc ugcaaucuuu c                                21

<210> 82

<211> 21

<212> RNA

<213> Artificial

<220>

<223> antisense strand siRNA as evaluation subject;VIM596

<400> 82

aagauugcag gguguuuucg g                                21

<210> 83

<211> 21

<212> RNA

<213> Artificial

<220>

<223> sense strand siRNA as evaluation subject;VIM812

<400> 83

guacgucagc aauaugaaag u                                21

<210> 84

<211> 21

<212> RNA

<213> Artificial

<220>

<223> antisense strand siRNA as evaluation subject;VIM812

<400> 84

uuucauauug cugacguacg u                                21

<210> 85

<211> 21
<212> RNA
<213> Artificial
<220>
<223> sense strand siRNA as evaluation subject;VIM857
<400> 85
gcagaagaau gguacaaauc c 21

<210> 86
<211> 21
<212> RNA
<213> Artificial
<220>
<223> antisense strand siRNA as evaluation subject;VIM857
<400> 86
auuuguacca uucuucugcc u 21

<210> 87
<211> 21
<212> RNA
<213> Artificial
<220>
<223> sense strand siRNA as evaluation subject;VIM1097
<400> 87
gaugagauuc agaauaugaa g 21

<210> 88
<211> 21
<212> RNA
<213> Artificial
<220>
<223> antisense strand siRNA as evaluation subject;VIM1097

<400> 88
ucauauucug aaucucaucc u 21

<210> 89
<211> 21
<212> RNA
<213> Artificial
<220>
<223> sense strand siRNA as evaluation subject;VIM1128
<400> 89
cucgucaccu ucgugaauac c 21

<210> 90
<211> 21
<212> RNA
<213> Artificial
<220>
<223> antisense strand siRNA as evaluation subject;VIM1128
<400> 90
uauucacgaa ggugacgagc c 21

<210> 91
<211> 21
<212> RNA
<213> Artificial
<220>
<223> sense strand siRNA as evaluation subject;VIM1148
<400> 91
caagaccugc ucaauguuaa g 21

<210> 92
<211> 21

<212> RNA
<213> Artificial
<220>
<223> antisense strand siRNA as evaluation subject;VIM1148

<400> 92
uaacauugag caggucuugg u 21

<210> 93
<211> 21
<212> RNA
<213> Artificial
<220>
<223> sense strand siRNA as evaluation subject;VIM1235
<400> 93
cugccucuuc caaacuuuuc c 21

<210> 94
<211> 21
<212> RNA
<213> Artificial
<220>
<223> antisense strand siRNA as evaluation subject;VIM1235
<400> 94
aaaaguuugg aagaggcaga g 21

<210> 95
<211> 21
<212> RNA
<213> Artificial
<220>
<223> sense strand siRNA as evaluation subject;VIM1298

<400> 95
guugauaccc acucaaaaag g 21

<210> 96
<211> 21
<212> RNA
<213> Artificial
<220>
<223> antisense strand siRNA as evaluation subject;VIM1298
<400> 96
uuuuugagug gguaucaacc a 21

<210> 97
<211> 21
<212> RNA
<213> Artificial
<220>
<223> sense strand siRNA as evaluation subject;VIM35
<400> 97
aggauguucg gcggcccggg c 21

<210> 98
<211> 21
<212> RNA
<213> Artificial
<220>
<223> antisense strand siRNA as evaluation subject;VIM35
<400> 98
ccgggccgcc gaacauccug c 21

<210> 99
<211> 21

<212> RNA
<213> Artificial
<220>
<223> sense strand siRNA as evaluation subject;VIM155
<400> 99
uacgccucgu ccccgggcgg c 21

<210> 100
<211> 21
<212> RNA
<213> Artificial
<220>
<223> antisense strand siRNA as evaluation subject;VIM155
<400> 100
cgcccgggga cgaggcguag a 21

<210> 101
<211> 21
<212> RNA
<213> Artificial
<220>
<223> sense strand siRNA as evaluation subject;VIM491
<400> 101
accaacgaca aagcccgcgu c 21

<210> 102
<211> 21
<212> RNA
<213> Artificial
<220>
<223> antisensestrand siRNA as evaluation subject;VIM491
<400> 102

cgcgggcuuu gucguugguu a                    21

<210> 103
<211> 21
<212> RNA
<213> Artificial
<220>
<223> sense strand siRNA as evaluation subject;Oct670
<400> 103
gagaaagcga acuagcauug a                    21

<210> 104
<211> 21
<212> RNA
<213> Artificial
<220>
<223> antisense strand siRNA as evaluation subject;Oct670
<400> 104
aaugcuaguu cgcuuucucu u                    21

<210> 105
<211> 21
<212> RNA
<213> Artificial
<220>
<223> sense strand siRNA as evaluation subject;Oct797
<400> 105
guucgaguau gguucuguaa c                    21

<210> 106
<211> 21
<212> RNA

<213> Artificial

<220>

<223> antisense strand siRNA as evaluation subject;Oct797

<400> 106

uacagaacca uacucgaacc a 21

<210> 107

<211> 21

<212> RNA

<213> Artificial

<220>

<223> sense strand siRNA as evaluation subject;Oct821

<400> 107

cgccagaagg gcaaaagauc a 21

<210> 108

<211> 21

<212> RNA

<213> Artificial

<220>

<223> antisense strand siRNA as evaluation subject;Oct821

<400> 108

aucuuuugcc cuucuggcgc c 21

<210> 109

<211> 21

<212> RNA

<213> Artificial

<220>

<223> sense strand siRNA as evaluation subject;Oct161

<400> 109

aucuccccau guccgcccgc a 21

<210> 110
<211> 21
<212> RNA
<213> Artificial
<220>
<223> antisense strand siRNA as evaluation subject;Oct161
<400> 110
cgggcggaca uggggagauc c 21

<210> 111
<211> 21
<212> RNA
<213> Artificial
<220>
<223> sense strand siRNA as evaluation subject;Oct566
<400> 111
auguguaagc ugcggccccu g 21

<210> 112
<211> 21
<212> RNA
<213> Artificial
<220>
<223> antisense strand siRNA as evaluation subject;Oct566
<400> 112
ggggccgcag cuuacacaug u 21

<210> 113
<211> 21
<212> RNA
<213> Artificial

<220>
<223> sense strand siRNA as evaluation subject;EGFP441

<400> 113
gccacaacgu cuauaucaug g 21

<210> 114
<211> 21
<212> RNA
<213> Artificial
<220>
<223> antisense strand siRNA as evaluation subject;EGFP441
<400> 114
augauauaga cguuguggcu g 21

<210> 115
<211> 23
<212> RNA
<213> Artificial
<220>
<223> egfp mRNA
<400> 115
cagccacaac gucuauauca ugg 23

<210> 116
<211> 23
<212> RNA
<213> Artificial
<220>
<223> ecfp mRNA
<400> 116
cagccacaac gucuauauca ccg 23

<210> 117
<211> 21
<212> RNA
<213> Artificial
<220>
<223> sense strand siRNA as evaluation subject;ECFP441
<400> 117
gccacaacgu cuauaucacc g 21

<210> 118
<211> 21
<212> RNA
<213> Artificial
<220>
<223> antisense strand siRNA as evaluation subject;ECFP441
<400> 118
gugauauaga cguuguggcu g 21

<210> 119
<211> 21
<212> RNA
<213> Artificial
<220>
<223> sense strand siRNA as evaluation subject;EGFP416
<400> 119
cacaagcugg aguacaacua c 21

<210> 120
<211> 21
<212> RNA
<213> Artificial

<220>
<223> antisense strand siRNA as evaluation subject;EGFP416
<400> 120
aguuguacuc cagcuugugc c 21

<210> 121
<211> 21
<212> RNA
<213> Artificial
<220>
<223> sense strand siRNA as evaluation subject;DsRed399
<400> 121
gccccguaau gcagaagaag a 21

<210> 122
<211> 21
<212> RNA
<213> Artificial
<220>
<223> antisense strand siRNA as evaluation subject;DsRed399
<400> 122
uucuucugca uuacggggcc g 21

<210> 123
<211> 21
<212> RNA
<213> Artificial
<220>
<223> sense strand siRNA as evaluation subject;DsRed231
<400> 123
ccgacauccc cgacuacaag a 21

<210> 124
<211> 21
<212> RNA
<213> Artificial
<220>
<223> antisense strand siRNA as evaluation subject;DsRed231
<400> 124
uuguagucgg ggaugucggc g 21

<210> 125
<211> 21
<212> RNA
<213> Artificial
<220>
<223> sense strand siRNA as evaluation subject;EGCFP666
<400> 125
aguucgugac cgccgccggg a 21

<210> 126
<211> 21
<212> RNA
<213> Artificial
<220>
<223> antisense strand siRNA as evaluation subject;EGCFP666
<400> 126
ccggcggcgg ucacgaacuc c 21

<210> 127
<211> 21
<212> RNA
<213> Artificial
<220>

<223> sense strand siRNA as evaluation subject;DsRed140
<400> 127
aaggugacca agggcggccc c 21

<210> 128
<211> 21
<212> RNA
<213> Artificial
<220>
<223> antisense strand siRNA as evaluation subject;DsRed140
<400> 128
ggccgcccuu ggucaccuuc a 21

<210> 129
<211> 21
<212> RNA
<213> Artificial
<220>
<223> sense strand siRNA as evaluation subject;DsRed383
<400> 129
aacuuccccu ccgacggccc c 21

<210> 130
<211> 21
<212> RNA
<213> Artificial
<220>
<223> antisense strand siRNA as evaluation subject;DsRed383
<400> 130
ggccgucgga ggggaaguuc a 21

<210> 131

<211> 56
<212> RNA
<213> Artificial
<220>
<223> shRNA as evaluation subject;FL184-m23L
<400> 131
guuggcagaa gcuaugaaac ggcuuccugu caccguuuca uagcuucugc caacuu 56

<210> 132
<211> 56
<212> RNA
<213> Artificial
<220>
<223> shRNA as evaluation subject;FL774-m23L
<400> 132
gauuucgagu cgucuuaaug ugcuuccugu cacacauuaa gacgacucga aaucuu 56

<210> 133
<211> 56
<212> RNA
<213> Artificial
<220>
<223> shRNA as evaluation subject;FL891-m23L
<400> 133
gcacucugau ugacaaauac ggcuuccugu caccguauuu gucaaucaga gugcuu 56

<210> 134
<211> 56
<212> RNA
<213> Artificial
<220>
<223> shRNA as evaluation subject;FL902-m23L

<400> 134

gacaaauacg auuuaucuaa ugcuuccugu cacauuagau aaaucguauu ugucuu 56

<210> 135

<211> 56

<212> RNA

<213> Artificial

<220>

<223> shRNA as evaluation subject;FL1186-m23L

<400> 135

gauuaucucc gguuauguaa agcuuccugu cacuuuacau aaccggacau aaucuu 56

<210> 136

<211> 56

<212> RNA

<213> Artificial

<220>

<223> shRNA as evaluation subject;FL1240-m23L

<400> 136

ggauggaugg cuacauucug ggcuuccugu cacccagaau guagccaucc auccuu 56

<210> 137

<211> 56

<212> RNA

<213> Artificial

<220>

<223> shRNA as evaluation subject;FL1308-m23L

<400> 137

gccugaaguc ucugauuaag ugcuuccugu cacacuuaau cagagacuuc aggcuu 56

<210> 138

<211> 56

<212> RNA
<213> Artificial
<220>
<223> shRNA as evaluation subject;FL14-m23L
<400> 138
aacauaaaga aaggcccggc ggcuuccugu caccgccggg ccuuucuuua uguuuu 56

<210> 139
<211> 59
<212> RNA
<213> Artificial
<220>
<223> shRNA as evaluation subject;FL620-m212L
<400> 139
gucgcucugc cucauagaac ugcccgggcc gcccucaguu cuaugaggca gagcgacuu 59

<210> 140
<211> 59
<212> RNA
<213> Artificial
<220>
<223> shRNA as evaluation subject;FL774-m212L
<400> 140
gauuucgagu cgucuuaaug ugcccgggcc gcccucacau uaagacgacu cgaaaucuu 59

<210> 141
<211> 59
<212> RNA
<213> Artificial
<220>
<223> shRNA as evaluation subject;FL826-m212L
<400> 141

aaucuuguaa uccugaaggc ugcccgggcc gcccucagcc uucaggauua caagauuuu 59